# EUROPEAN PATENT APPLICATION

(11) **EP 2 881 403 A1**
(43) Date of publication of application: **10.06.2015**
(21) Application number: 13826178.9
(22) Date of filing: 29.07.2013
(51) Int. Cl.: C07K 17/00, C07K 14/315, C07K 19/00, C07K 1/14, C07K 1/22, C12N 15/09

(54) **SCAVENGER CONTAINING PROTEIN FORMED FROM TANDEM-TYPE MULTIMER OF MUTANT EXTRACELLULAR DOMAIN OF PROTEIN G**

(30) Priority: 03.08.2012 JP 2012172452
(71) Applicant: National Institute of Advanced Industrial Science and Technology, Tokyo 100-8921 (JP); Daicel Corporation, Osaka-shi, Osaka 530-0001 (JP)
(72) Inventor: HONDA, Shinya, Tsukuba-shi Ibaraki 305-8561 (JP); WATANABE, Hideki, Tsukuba-shi Ibaraki 305-8561 (JP); YOSHIDA, Chuya, Tsukuba-shi Ibaraki 305-8561 (JP); UEDA, Momoko, Himeji-shi Hyogo 671-1283 (JP); NAKAI, Yasuto, Himeji-shi Hyogo 671-1283 (JP); ISOBE, Yutaka, Himeji-shi Hyogo 671-1283 (JP)
(74) Representative: Power, David
(86) International application number: PCT/JP2013/070423
(87) International publication number: WO 2014/021240

(57) **Abstract**

The purpose of the present invention is to provide a superior protein with more decreased binding property to the Fc region of immunoglobulin and/or more decreased binding property to the Fab region thereof in the weakly acidic region in comparison with a protein containing an extracellular domain of a wild-type protein G without spoiling the high antibody binding property in the neutral region, and to allow the antibody to be easily captured and recovered without denaturing it by means of a capturing agent and column comprising the above protein.

The present invention relates to a capturing agent for an antibody, immunoglobulin G or a protein comprising an Fc region of the immunoglobulin G, wherein a protein consisting of a tandem-type multimer of extracellular domain mutants that have binding property to the protein comprising the Fc region of the immunoglobulin G is immobilized to a water-insoluble solid support via mainly an amino group, and a column for a chromatography for separating and purifying the protein, which is filled with the capturing agent.

### SEQUENCE LISTING

<110> National Institute of Advanced Industrial science and Technology; Daicel Corporation
<120> Capturing agent comprising novel modified protein of tandem-type polymer comprising extracellular-domain variants of protein G
<130> PCT-AB13018
<150> JP 2012-172452
   <151> 2012-08-03
<160> 37
<170> PatentIn version 3.1
<210> 1
   <211> 56
   <212> PRT
   <213> Streptcoccus sp.(Protein G B1domain; wild)
<400> 1
   Asp Thr Tyr Lys Leu Ile Leu Asn Gly Lys Thr Leu Lys Gly Glu Thr
   1               5                   10                  15
   Thr Thr Glu Ala Val Asp Ala Ala Thr Ala Glu Lys Val Phe Lys Gln
               20                  25                  30
   Tyr Ala Asn Asp Asn Gly Val Asp Gly Glu Trp Thr Tyr Asp Asp Ala
           35                  40                  45
   Thr Lys Thr Phe Thr Val Thr Glu
       50                  55
<210> 2
   <211> 56
   <212> PRT
   <213> Streptcoccus sp.(Protein G B2domain; wild)
<400> 2
   Thr Thr Tyr Lys Leu Val Ile Asn Gly Lys Thr Leu Lys Gly Glu Thr
   1               5                   10                  15
   Thr Thr Glu Ala Val Asp Ala Ala Thr Ala Glu Lys Val Phe Lys Gln
               20                  25                  30
   Tyr Ala Asn Asp Asn Gly Val Asp Gly Glu Trp Thr Tyr Asp Asp Ala
           35                  40                  45
   Thr Lys Thr Phe Thr Val Thr Glu
       50                  55
<210> 3
   <211> 56
   <212> PRT
   <213> Streptcoccus sp.(Protein G B3domain; wild)
<400> 3
   Thr Thr Tyr Lys Leu Val Ile Asn Gly Lys Thr Leu Lys Gly Glu Thr
   1               5                   10                  15
   Thr Thr Lys Ala Val Asp Ala Glu Thr Ala Glu Lys Ala Phe Lys Gln
               20                  25                  30
   Tyr Ala Asn Asp Asn Gly Val Asp Gly Val Trp Thr Tyr Asp Asp Ala
           35                  40                  45
   Thr Lys Thr Phe Thr Val Thr Glu
       50                  55
<210> 4
   <211> 56
   <212> PRT
   <213> Artificial
<220>
   <223> ProteinG B1domain mutant
<220>
   <221> MISC_FEATURE
   <222> (35)..(35)
   <223> Xaa is Asn or Lys
<220>
   <221> MISC_FEATURE
   <222> (36)..(36)
   <223> Xaa is Asp or Glu
<220>
   <221> MISC_FEATURE
   <222> (37)..(37)
   <223> Xaa is Asn His or Leu
<220>
   <221> MISC_FEATURE
   <222> (47)..(47)
   <223> Xaa is Asp or Pro
<220>
   <221> MISC_FEATURE
   <222> (48)..(48)
   <223> Xaa is Ala, Lys or Glu
<220>
   <221> MISC_FEATURE
   <222> (22)..(22)
   <223> Xaa is Asp or His
<220>
   <221> MISC_FEATURE
   <222> (25)..(25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (42)..(42)
   <223> Xaa is Glu or His
<220>
   <221> MISC_FEATURE
   <222> (11)..(11)
   <223> Xaa is Thr or Arg
<220>
   <221> MISC_FEATURE
   <222> (17)..(17)
   <223> Xaa is Thr or Ile
<400> 4
   Asp Thr Tyr Lys Leu Ile Leu Asn Gly Lys Xaa Leu Lys Gly Glu Thr
   1               5                   10                  15
   Xaa Thr Glu Ala Val Xaa Ala Ala Xaa Ala Glu Lys Val Phe Lys Xaa
               20                  25                  30
   Tyr Ala Xaa Xaa Xaa Gly Val Xaa Gly Xaa Trp Thr Tyr Asp Xaa Xaa
           35                  40                  45
   Thr Lys Thr Phe Thr Val Thr Glu
       50                  55
<210> 5
   <211> 56
   <212> PRT
   <213> Artificial
<220>
   <223> ProteinG B2domain mutant
<220>
   <221> MISC_FEATURE
   <222> (35)..(35)
   <223> Xaa is Asn or Lys
<220>
   <221> MISC_FEATURE
   <222> (36)..(36)
   <223> Xaa is Asp or Glu
<220>
   <221> MISC_FEATURE
   <222> (37)..(37)
   <223> Xaa is Asn,His or Leu
<220>
   <221> MISC_FEATURE
   <222> (47)..(47)
   <223> Xaa is Asp or Pro
<220>
   <221> MISC_FEATURE
   <222> (48)..(48)
   <223> Xaa is Ala,Lys or Glu
<220>
   <221> MISC_FEATURE
   <222> (22)..(22)
   <223> Xaa is Asp or His
<220>
   <221> MISC_FEATURE
   <222> (25)..(25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (42)..(42)
   <223> Xaa is Glu or His
<220>
   <221> MISC_FEATURE
   <222> (11)..(11)
   <223> Xaa is Thr or Arg
<220>
   <221> MISC_FEATURE
   <222> (17)..(17) <223> Xaa is Thr or Ile
<400> 5
   Thr Thr Tyr Lys Leu Val Ile Asn Gly Lys Xaa Leu Lys Gly Glu Thr
   1               5                   10                  15
   Xaa Thr Glu Ala Val Xaa Ala Ala Xaa Ala Glu Lys Val Phe Lys Xaa
               20                  25                  30
   Tyr Ala Xaa Xaa Xaa Gly Val Xaa Gly Xaa Trp Thr Tyr Asp Xaa Xaa
           35                  40                  45
   Thr Lys Thr Phe Thr Val Thr Glu
       50                  55
<210> 6
   <211> 56
   <212> PRT
   <213> Artificial
<220>
   <223> ProteinG B3domain mutant
<220>
   <221> MISC_FEATURE
   <222> (35)..(35)
   <223> Xaa is Asn or Lys
<220>
   <221> MISC_FEATURE
   <222> (36)..(36)
   <223> Xaa is Asp or Glu
<220>
   <221> MISC_FEATURE
   <222> (37)..(37)
   <223> Xaa is Asn,His or Leu
<220>
   <221> MISC_FEATURE
   <222> (47)..(47)
   <223> Xaa is Asp or Pro
<220>
   <221> MISC_FEATURE
   <222> (48)..(48)
   <223> Xaa is Ala,Lys or Glu
<220>
   <221> MISC_FEATURE
   <222> (22) .. (22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (11)..(11)
   <223> Xaa is Thr or Arg
<220>
   <221> MISC_FEATURE
   <222> (17)..(17)
   <223> Xaa is Thr or Ile
<400> 6
   Thr Thr Tyr Lys Leu Val Ile Asn Gly Lys Xaa Leu Lys Gly Glu Thr
   1               5                   10                  15
   Xaa Thr Lys Ala Val Xaa Ala Glu Xaa Ala Glu Lys Ala Phe Lys Xaa
               20                  25                  30
   Tyr Ala Xaa Xaa Xaa Gly Val Xaa Gly Val Trp Thr Tyr Asp Xaa Xaa
           35                  40                  45
   Thr Lys Thr Phe Thr Val Thr Glu
       50                  55
<210> 7
   <211> 56
   <212> PRT
   <213> Artificial
<220>
   <223> ProteinG B1domain mutant
<220>
   <221> MISC_FEATURE
   <222> (22)..(22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (42)..(42)
   <223> Xaa is Glu or His
<220>
   <221> MISC_FEATURE
   <222> (11)..(11)
   <223> Xaa is Thr or Arg
<220>
   <221> MISC_FEATURE
   <222> (17)..(17)
   <223> Xaa is Thr or Ile
<400> 7
   Asp Thr Tyr Lys Leu Ile Leu Asn Gly Lys Xaa Leu Lys Gly Glu Thr
   1               5                   10                  15
   Xaa Thr Glu Ala Val Xaa Ala Ala Xaa Ala Glu Lys Val Phe Lys Xaa
               20                  25                  30
   Tyr Ala Asn Asp Asn Gly Val Xaa Gly Xaa Trp Thr Tyr Asp Asp Ala
           35                  40                  45
   Thr Lys Thr Phe Thr Val Thr Glu
       50                  55
<210> 8
   <211> 56
   <212> PRT
   <213> Artificial
<220>
   <223> proteinG B2domain mutant
<220>
   <221> MISC_FEATURE
   <222> (22)..(22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (42)..(42)
   <223> Xaa is Glu or His
<220>
   <221> MISC_FEATURE
   <222> (11)..(11)
   <223> Xaa is Thr or Arg
<220>
   <221> MISC_FEATURE
   <222> (17)..(17)
   <223> Xaa is Thr or Ile
<400> 8
   Thr Thr Tyr Lys Leu Val Ile Asn Gly Lys Xaa Leu Lys Gly Glu Thr
   1               5                   10                  15
   Xaa Thr Glu Ala Val Xaa Ala Ala Xaa Ala Glu Lys Val Phe Lys Xaa
               20                  25                  30
   Tyr Ala Asn Asp Asn Gly Val Xaa Gly Xaa Trp Thr Tyr Asp Asp Ala
           35                  40                  45
   Thr Lys Thr Phe Thr Val Thr Glu
       50                  55
<210> 9
   <211> 56
   <212> PRT
   <213> Artificial
<220>
   <223> ProteinG B3domain mutant
<220>
   <221> MISC_FEATURE
   <222> (22)..(22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (11)..(11)
   <223> Xaa is Thr or Arg
<220>
   <221> MISC_FEATURE
   <222> (17)..(17)
   <223> Xaa is Thr or Ile
<400> 9
   Thr Thr Tyr Lys Leu Val Ile Asn Gly Lys Xaa Leu Lys Gly Glu Thr
   1 5 10 15
   Xaa Thr Lys Ala Val Xaa Ala Glu Xaa Ala Glu Lys Ala Phe Lys Xaa
   20 25 30
   Tyr Ala Asn Asp Asn Gly Val Xaa Gly Val Trp Thr Tyr Asp Asp Ala
   35 40 45
   Thr Lys Thr Phe Thr Val Thr Glu
   50 55
<210> 10
   <211> 56
   <212> PRT
   <213> Artificial
<220>
   <223> ProteinG B1domain mutant
<220>
   <221> MISC_FEATURE
   <222> (22)..(22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (42)..(42)
   <223> Xaa is Glu or His
<400> 10
   Asp Thr Tyr Lys Leu Ile Leu Asn Gly Lys Thr Leu Lys Gly Glu Thr
   1               5                   10                  15
   Thr Thr Glu Ala Val Xaa Ala Ala Xaa Ala Glu Lys Val Phe Lys Xaa
               20                  25                  30
   Tyr Ala Asn Asp Asn Gly Val Xaa Gly Xaa Trp Thr Tyr Asp Asp Ala
           35                  40                  45
   Thr Lys Thr Phe Thr Val Thr Glu
       50                  55
<210> 11
   <211> 56
   <212> PRT
   <213> Artificial
<220>
   <223> ProteinG B2domain mutant
<220>
   <221> MISC_FEATURE
   <222> (22)..(22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<220>
   <221> MISC_FEATURE
   <222> (42)..(42)
   <223> Xaa is Glu or His
<400> 11
   Thr Thr Tyr Lys Leu Val Ile Asn Gly Lys Thr Leu Lys Gly Glu Thr
   1               5                   10                  15
   Thr Thr Glu Ala Val Xaa Ala Ala Xaa Ala Glu Lys Val Phe Lys Xaa
               20                  25                  30
   Tyr Ala Asn Asp Asn Gly Val Xaa Gly Xaa Trp Thr Tyr Asp Asp Ala
           35                  40                  45
   Thr Lys Thr Phe Thr Val Thr Glu
       50                  55
<210> 12
   <211> 56
   <212> PRT
   <213> Artificial
<220>
   <223> ProteinG B3domain mutant
<220>
   <221> MISC_FEATURE
   <222> (22)..(22)
   <223> Xaa is Asp or His
<220>
   <221> MISC_FEATURE
   <222> (25) .. (25)
   <223> Xaa is Thr or His
<220>
   <221> MISC_FEATURE
   <222> (32)..(32)
   <223> Xaa is Gln or His
<220>
   <221> MISC_FEATURE
   <222> (40)..(40)
   <223> Xaa is Asp or His
<400> 12
   Thr Thr Tyr Lys Leu Val Ile Asn Gly Lys Thr Leu Lys Gly Glu Thr
   1               5                   10                  15
   Thr Thr Lys Ala Val Xaa Ala Glu Xaa Ala Glu Lys Ala Phe Lys Xaa
               20                  25                  30
   Tyr Ala Asn Asp Asn Gly Val Xaa Gly Val Trp Thr Tyr Asp Asp Ala
           35                  40                  45
   Thr Lys Thr Phe Thr Val Thr Glu
       50                  55
<210> 13
   <211> 56
   <212> PRT
   <213> Artificial
<220>
   <223> ProteinG B1domain mutant
<400> 13
   Asp Thr Tyr Lys Leu Ile Leu Asn Gly Lys Thr Leu Lys Gly Glu Thr
   1               5                   10                  15
   Thr Thr Glu Ala Val Asp Ala Ala Thr Ala Glu Lys Val Phe Lys Gln
               20                  25                  30
   Tyr Ala Asn Glu His Gly Val Asp Gly Glu Trp Thr Tyr Asp Pro Glu
           35                  40                  45
   Thr Lys Thr Phe Thr Val Thr Glu
       50                  55
<210> 14
   <211> 56
   <212> PRT
   <213> Artificial
<220>
   <223> ProteinG B1domain mutant
<400> 14
   Asp Thr Tyr Lys Leu Ile Leu Asn Gly Lys Thr Leu Lys Gly Glu Thr
   1               5                   10                  15
   Thr Thr Glu Ala Val Asp Ala Ala Thr Ala Glu Lys Val Phe Lys Gln
               20                  25                  30
   Tyr Ala Asn Glu His Gly Val His Gly Glu Trp Thr Tyr Asp Pro Glu
           35                  40                  45
   Thr Lys Thr Phe Thr Val Thr Glu
       50                  55
<210> 15
   <211> 56
   <212> PRT
   <213> Artificial
<220>
   <223> ProteinG B1domain mutant
<400> 15
   Asp Thr Tyr Lys Leu Ile Leu Asn Gly Lys Thr Leu Lys Gly Glu Thr
   1               5                   10                  15
   Thr Thr Glu Ala Val Asp Ala Ala Thr Ala Glu Lys Val Phe Lys Gln
               20                  25                  30
   Tyr Ala Asn Glu His Gly Val Asp Gly His Trp Thr Tyr Asp Pro Glu
           35                  40                  45
   Thr Lys Thr Phe Thr Val Thr Glu
       50                  55
<210> 16
   <211> 56
   <212> PRT
   <213> Artificial
<220>
   <223> ProteinG B1domain mutant
<400> 16
   Asp Thr Tyr Lys Leu Ile Leu Asn Gly Lys Arg Leu Lys Gly Glu Thr
   1               5                   10                  15
   Thr Thr Glu Ala Val Asp Ala Ala Thr Ala Glu Lys Val Phe Lys Gln
               20                  25                  30
   Tyr Ala Asn Glu His Gly Val Asp Gly Glu Trp Thr Tyr Asp Pro Glu
           35                  40                  45
   Thr Lys Thr Phe Thr Val Thr Glu
       50                  55
<210> 17
   <211> 56
   <212> PRT
   <213> Artificial
<220>
   <223> ProteinG B1domain mutant
<400> 17
   Asp Thr Tyr Lys Leu Ile Leu Asn Gly Lys Thr Leu Lys Gly Glu Thr
   1               5                   10                  15
   Ile Thr Glu Ala Val Asp Ala Ala Thr Ala Glu Lys Val Phe Lys Gln
               20                  25                  30
   Tyr Ala Asn Glu His Gly Val Asp Gly Glu Trp Thr Tyr Asp Pro Glu
           35                  40                  45
   Thr Lys Thr Phe Thr Val Thr Glu
       50                  55
<210> 18
   <211> 56
   <212> PRT
   <213> Artificial
<220>
   <223> ProteinG B1domain mutant
<400> 18
   Asp Thr Tyr Lys Leu Ile Leu Asn Gly Lys Arg Leu Lys Gly Glu Thr
   1               5                   10                  15
   Ile Thr Glu Ala Val Asp Ala Ala Thr Ala Glu Lys Val Phe Lys Gln
               20                  25                  30
   Tyr Ala Asn Glu His Gly Val Asp Gly Glu Trp Thr Tyr Asp Pro Glu
   35 40 45
   Thr Lys Thr Phe Thr Val Thr Glu
   50 55
<210> 19
   <211> 56
   <212> PRT
   <213> Artificial
<220>
   <223> ProteinG B1domain mutant
<400> 19
   Asp Thr Tyr Lys Leu Ile Leu Asn Gly Lys Thr Leu Lys Gly Glu Thr
   1 5 10 15
   Thr Thr Glu Ala Val Asp Ala Ala Thr Ala Glu Lys Val Phe Lys His
   20 25 30
   Tyr Ala Asn Glu His Gly Val His Gly His Trp Thr Tyr Asp Pro Glu
   35 40 45
   Thr Lys Thr Phe Thr Val Thr Glu
   50 55
<210> 20
   <211> 56
   <212> PRT
<213> Artificial
<220>
   <223> ProteinG B1domain mutant
<400> 20
   Asp Thr Tyr Lys Leu Ile Leu Asn Gly Lys Thr Leu Lys Gly Glu Thr
   1 5 10 15
   Thr Thr Glu Ala Val His Ala Ala Thr Ala Glu Lys Val Phe Lys His
   20 25 30
   Tyr Ala Asn Glu His Gly Val His Gly His Trp Thr Tyr Asp Pro Glu
   35 40 45
   Thr Lys Thr Phe Thr Val Thr Glu
   50 55
<210> 21
   <211> 168
   <212> DNA
   <213> Polynucleotide encoding Streptcoccus sp. Protein G B1domain
<400> 21
   gacacttaca aattaatcct taatggtaaa acattgaaag gcgaaacaac tactgaagct     60
   gttgatgctg ctactgcaga aaaagtcttc aaacaatacg ctaacgacaa cggtgttgac    120
   ggtgaatgga cttacgacga tgcgactaag acctttacag ttactgaa                 168
<210> 22
   <211> 168
   <212> DNA
   <213> Artificial
<220>
   <223> Polynucleotide encoding protein G B1domain mutant
<400> 22
   gacacttaca aattaatcct taatggtaaa acattgaaag gcgaaacaac tactgaagct     60
   gttgatgctg ctactgcaga aaaagtcttc aaacaatacg ctaacgaaca tggtgttgac    120
   ggtgaatgga cttacgaccc ggaaactaag acctttacag ttactgaa                 168
<210> 23
   <211> 168
   <212> DNA
   <213> Artificial
<220>
   <223> Polynucleotide encoding protein G B1domain mutant
<400> 23
   gatacctaca aactgatcct gaatggtaaa accctgaaag gcgaaaccac cactgaggcg     60
   gtagacgctg cgaccgcgga gaaagttttc aaacagtacg ctaacgaaca cggtgttcac    120
   ggcgagtgga cctacgaccc ggaaaccaag accttcaccg ttaccgaa                 168
<210> 24
   <211> 168
   <212> DNA
   <213> Artificial
<220>
   <223> Polynucleotide encoding protein G B1domain mutant
<400> 24
   gatacctaca aactgatcct gaatggtaaa accctgaaag gcgaaaccac cactgaggcg 60
   gtagacgctg cgaccgcgga gaaagttttc aaacagtacg ctaacgaaca cggtgttgac 120
   ggccactgga cctacgaccc ggaaaccaag accttcaccg ttaccgaa 168
<210> 25
   <211> 168
   <212> DNA
   <213> Artificial
<220>
   <223> Polynucleotide encoding protein G B1domain mutant
<400> 25
   gatacctaca aactgatcct gaatggtaaa cgtctgaaag gcgaaaccac cactgaggcg 60
   gtagacgctg cgaccgcgga gaaagttttc aaacagtacg ctaacgaaca cggtgttgac 120
   ggcgagtgga cctacgaccc ggaaaccaag accttcaccg ttaccgaa 168
<210> 26
   <211> 168
   <212> DNA
   <213> Artificial
<220>
   <223> Polynucleotide encoding protein G B1domain mutant
<400> 26
   gatacctaca aactgatcct gaatggtaaa accctgaaag gcgaaaccat caccgaggcg 60
   gtagacgctg cgaccgcgga gaaagttttc aaacagtacg ctaacgaaca cggtgttgac 120
   ggcgagtgga cctacgaccc ggaaaccaag accttcaccg ttaccgaa 168
<210> 27
   <211> 168
   <212> DNA
   <213> Artificial
<220>
   <223> Polynucleotide encoding protein G B1domain mutant
<400> 27
   gatacctaca aactgatcct gaatggtaaa cgcctgaaag gcgaaaccat caccgaggcg 60
   gtagacgctg cgaccgcgga gaaagttttc aaacagtacg ctaacgaaca cggtgttgac 120
   ggcgagtgga cctacgaccc ggaaaccaag accttcaccg ttaccgaa 168
<210> 28
   <211> 168
   <212> DNA
   <213> Artificial
<220>
   <223> Polynucleotide encoding protein G B1domain mutant
<400> 28
   gatacctaca aactgatcct gaatggtaaa cgcctgaaag gcgaaaccat caccgaggcg     60
   gtagacgctg cgaccgcgga gaaagttttc aaacagtacg ctaacgaaca cggtgttgac    120
   ggcgagtgga cctacgaccc ggaaaccaag accttcaccg ttaccgaa                 168
<210> 29
   <211> 168
   <212> DNA
   <213> Artificial
<220>
   <223> Polynucleotide encoding protein G B1domain mutant
<400> 29 <210> 30
   <211> 2456
   <212> DNA
   <213> Streptcoccus sp.G148(polynucleotide encoding Protein G; wild)
<400> 30
<210> 31
   <211> 103
   <212> PRT
   <213> Klebsiella pneeumoniae (C-terminal sequense oxaloacetate decarboxylase alpha-subunit c-terminal domain)
<400> 31
   Met Gly Ala Gly Thr Pro Val Thr Ala Pro Leu Ala Gly Thr Ile Trp
   1               5                   10                  15
   Lys Val Leu Ala Ser Glu Gly Gln Thr Val Ala Ala Gly Glu Val Leu
               20                  25                  30
   Leu Ile Leu Glu Ala Met Lys Met Glu Thr Glu Ile Arg Ala Ala Gln
           35                  40                  45
   Ala Gly Thr Val Arg Gly Ile Ala Val Lys Ala Gly Asp Ala Val Ala
       50                  55                  60
   Val Gly Asp Thr Leu Met Thr Leu Ala Gly Ser Gly Ser Asp Leu Tyr
   65 70 75 80
   Asp Asp Asp Asp Lys Gly Ile Ile Thr Ser Leu Tyr Lys Lys Ala Gly
   85 90 95
   Ser Ala Ala Ala Pro Phe Thr
   100
<210> 32
   <211> 30
   <212> DNA
   <213> Artificial
<220>
   <223> Primer
<400> 32
   atagctccat ggacacttac aaattaatcc 30
<210> 33
   <211> 30
   <212> DNA
   <213> Artificial
<220>
   <223> Primer
<400> 33
   attggatcct tattcagtaa ctgtaaaggt 30
<210> 34
   <211> 30
   <212> DNA
   <213> Artificial
<220>
   <223> Primer
<400> 34
   atagctccat ggatacctac aaactgatcc 30
<210> 35
   <211> 30
   <212> DNA
   <213> Artificial
<220>
   <223> Primer
<400> 35
   attggatcct tattcggtaa cggtgaaggt 30
<210> 36
   <211> 711
   <212> DNA
   <213> Artificial Sequence
<220>
   <223> CGB01H-3D
<220>
   <221> CDS
   <222> (1)..(711)
   <223>
<400> 36
   atg gcg tat tac gat cca gaa acg ggc act tgg tat tct ggc ggt cgt        48
   Met Ala Tyr Tyr Asp Pro Glu Thr Gly Thr Trp Tyr Ser Gly Gly Arg
   1               5                   10                  15
   gat acc tat aaa ctg att ctg aat ggt aaa acc ctg aaa ggt gaa acc        96
   Asp Thr Tyr Lys Leu Ile Leu Asn Gly Lys Thr Leu Lys Gly Glu Thr
               20                  25                  30
   acc acc gaa gca gtt gat gca gca acc gca gaa aaa gtt ttt aaa caa       144
   Thr Thr Glu Ala Val Asp Ala Ala Thr Ala Glu Lys Val Phe Lys Gln
           35                  40                  45
   tat gcc aat gac aac ggt gtt gac ggt gaa tgg acc tat gat gat gcg       192
   Tyr Ala Asn Asp Asn Gly Val Asp Gly Glu Trp Thr Tyr Asp Asp Ala
       50                  55                  60
   acc aaa acc ttt acc gtt acc gaa cgt ccg gaa gtt att gat gca agc       240
   Thr Lys Thr Phe Thr Val Thr Glu Arg Pro Glu Val Ile Asp Ala Ser
   65                  70                  75                  80
   gaa ctg acc cca gca gtg gat aca tat aag ctg atc ctg aac ggc aag       288
   Glu Leu Thr Pro Ala Val Asp Thr Tyr Lys Leu Ile Leu Asn Gly Lys
                   85                  90                  95
   aca ctg aag ggc gaa acc aca act gaa gcc gtg gat gcc gcc acc gcc       336
   Thr Leu Lys Gly Glu Thr Thr Thr Glu Ala Val Asp Ala Ala Thr Ala
               100                 105                 110
   gaa aag gtg ttt aag cag tac gca aac gat aat ggc gtg gat ggc gag       384
   Glu Lys Val Phe Lys Gln Tyr Ala Asn Asp Asn Gly Val Asp Gly Glu
           115                 120                 125
   tgg aca tat gat gac gcc aca aag aca ttt acc gtg aca gaa cgc cct       432
   Trp Thr Tyr Asp Asp Ala Thr Lys Thr Phe Thr Val Thr Glu Arg Pro
       130                 135                 140
   gaa gtg atc gat gca tct gaa ctg aca ccg gcc gtt gat act tat aaa       480
   Glu Val Ile Asp Ala Ser Glu Leu Thr Pro Ala Val Asp Thr Tyr Lys
   145                 150                 155                 160
   ctg atc ctg aat gga aag act ctg aaa gga gag aca acc acg gaa gcg       528
   Leu Ile Leu Asn Gly Lys Thr Leu Lys Gly Glu Thr Thr Thr Glu Ala
                   165                 170                 175
   gtg gat gcg gcg aca gcg gaa aaa gta ttc aag cag tat gcg aac gat       576
   Val Asp Ala Ala Thr Ala Glu Lys Val Phe Lys Gln Tyr Ala Asn Asp
               180                 185                 190
   aac ggc gta gat ggc gaa tgg act tat gat gac gcg act aaa acg ttc       624
   Asn Gly Val Asp Gly Glu Trp Thr Tyr Asp Asp Ala Thr Lys Thr Phe
           195                 200                 205
   aca gtg acc gag cgc cct gag gtt att gat gcc tca gag ctg acc ccg       672
   Thr Val Thr Glu Arg Pro Glu Val Ile Asp Ala Ser Glu Leu Thr Pro
       210                 215                 220
   gct gtt ggt ggt ggt ggt tgt cat cat cat cac cat cat                   711
   Ala Val Gly Gly Gly Gly Cys His His His His His His
   225                 230                 235
<210> 37
   <211> 711
   <212> DNA
   <213> Artificial Sequence
<220>
   <223> CGB19H-3D
<220>
   <221> CDS
   <222> (1)..(711)
   <223>
<400> 37
   atg gcg tat tac gat cca gaa acg ggc act tgg tat tct ggc ggt cgt       48
   Met Ala Tyr Tyr Asp Pro Glu Thr Gly Thr Trp Tyr Ser Gly Gly Arg
   1               5                   10                  15
   gat acc tat aaa ctg att ctg aat ggt aaa acc ctg aaa ggt gaa acc       96
   Asp Thr Tyr Lys Leu Ile Leu Asn Gly Lys Thr Leu Lys Gly Glu Thr
               20                  25                  30
   acc acc gaa gca gtt gat gca gca acc gca gaa aaa gtt ttt aaa cat      144
   Thr Thr Glu Ala Val Asp Ala Ala Thr Ala Glu Lys Val Phe Lys His
           35                  40                  45
   tat gcc aat gaa cat ggt gtt cat ggt cat tgg acc tat gat ccg gaa      192
   Tyr Ala Asn Glu His Gly Val His Gly His Trp Thr Tyr Asp Pro Glu
       50                  55                  60
   acc aaa acc ttt acc gtt acc gaa cgt ccg gaa gtt att gat gca agc      240
   Thr Lys Thr Phe Thr Val Thr Glu Arg Pro Glu Val Ile Asp Ala Ser
   65                  70                  75                  80
   gaa ctg acc cca gca gtg gat aca tat aag ctg atc ctg aac ggc aag      288
   Glu Leu Thr Pro Ala Val Asp Thr Tyr Lys Leu Ile Leu Asn Gly Lys
                   85                  90                  95
   aca ctg aag ggc gaa acc aca act gaa gcc gtg gat gcc gcc acc gcc      336
   Thr Leu Lys Gly Glu Thr Thr Thr Glu Ala Val Asp Ala Ala Thr Ala
               100                 105                 110
   gaa aag gtg ttt aag cac tac gca aac gaa cat ggc gtg cac ggc cac      384
   Glu Lys Val Phe Lys His Tyr Ala Asn Glu His Gly Val His Gly His
           115                 120                 125
   tgg aca tat gat cct gag aca aag aca ttt acc gtg aca gaa cgc cct      432
   Trp Thr Tyr Asp Pro Glu Thr Lys Thr Phe Thr Val Thr Glu Arg Pro
       130                 135                 140
   gaa gtg atc gat gca tct gaa ctg aca ccg gcc gtt gat act tat aaa      480
   Glu Val Ile Asp Ala Ser Glu Leu Thr Pro Ala Val Asp Thr Tyr Lys
   145                 150                 155                 160
   ctg atc ctg aat gga aag act ctg aaa gga gag aca acc acg gaa gcg      528
   Leu Ile Leu Asn Gly Lys Thr Leu Lys Gly Glu Thr Thr Thr Glu Ala
                   165                 170                 175
   gtg gat gcg gcg aca gcg gaa aaa gta ttc aag cat tat gcg aac gag      576
   Val Asp Ala Ala Thr Ala Glu Lys Val Phe Lys His Tyr Ala Asn Glu
               180                 185                 190
   cac ggc gta cat ggc cat tgg act tat gat cca gag act aaa acg ttc      624
   His Gly Val His Gly His Trp Thr Tyr Asp Pro Glu Thr Lys Thr Phe
           195                 200                 205
   aca gtg acc gag cgc cct gag gtt att gat gcc tca gag ctg acc ccg      672
   Thr Val Thr Glu Arg Pro Glu Val Ile Asp Ala Ser Glu Leu Thr Pro
       210                 215                 220
   gct gtt ggt ggt ggt ggt tgt cat cat cat cac cat cat                  711
   Ala Val Gly Gly Gly Gly Cys His His His His His His
   225                 230                 235

## Description

### Technical Field

The present invention relates to a capturing agent for an antibody, immunoglobulin G or a protein comprising an Fc region of the immunoglobulin G, wherein a protein consisting of or comprising a tandem-type multimer of extracellular domain mutants that have binding property to the protein comprising the Fc region of the immunoglobulin G is immobilized to a water-insoluble solid support, and to a column for a chromatography for separating and purifying the protein, which is filled with the capturing agent, and the like.

### Background Art

The protein G, which is a protein derived from streptococus, is a membrane protein present in a cell membrane of streptococcus of genus Streptococcus, and it is known that the protein G has specific binding property to the Fc region of immunoglobulin G which is a kind of the antibody (Non-patent Document 1, Patent Document 1). The protein G is a multi-domain type membrane protein consisting of a plurality of domains, some extracellular domains of which exhibit the binding property to the protein having the Fc region of immunoglobulin G (hereinafter, referred to as "antibody binding property") (Non-patent Document 2). For example, in a protein G derived from a G148 strain shown in FIG. 1, three domains of B1, B2 and B3 exhibit the antibody binding property (also written as "C1, C2 and C3 domains" depending on a document). Also, a protein G from a GX7805 strain has three antibody binding domains and a protein G from a GX7809 has two antibody binding domains. These proteins are all miniature proteins with less than 60 amino acids, and it is known that they have high identity among each amino acid sequence). It is also known that even if the protein G is cut to isolate each domain alone, the antibody binding property is maintained (Non-patent Document 3).

Many extracellular domain of protein G-containing products utilizing the selective antibody binding property of the extracellular domain of the protein G are currently marketed (for example, a carrier for affinity chromatography for purifying the antibody (Patent Documents 3 and 4), an inspection reagent and a research reagent for detecting the antibody, and the like). It is known that a binding power between the extracellular domain of the protein G and the antibody is high in a neutral to weakly acidic region and is low in a strongly acidic region (Non-patent Document 4). Therefore, when isolating, recovering and purifying the antibody, first, a sample solution containing the antibody, such as a serum, is made in a neutral state and then is brought into contact with a water-insoluble solid support, such as a bead, to which the extracellular domain of the protein G is immobilized, so that the antibody is selectively absorbed. Next, the water-insoluble solid support is cleaned with a neutral to weak acid solution (pH 5 to pH 8) to remove components other than the antibody. Last, a strongly acidic solution having pH 2.4 to pH 3.5 is generally added to desorb the antibody from the immobilized protein G and to elute the antibody with the strongly acidic solution (Patent Document 3). By this process, the antibody can be isolated, recovered and purified with high purity.

However, the antibody may be degraded by the strongly acidic solution having pH 2.4 to pH 3.5 due to denatured aggregation or the like, and, depending on the type of the antibody, an original function may be lost (Non-patent Document 4). Although the process is attempted in a weakly acidic region above pH 2.4 to pH 3.5 in order to solve such a problem, the antibody is not eluted from the protein G in the weakly acidic region because the binding power between the extracellular domain of the protein G and the antibody is strong, so that a sufficient recovery amount is not attained. Moreover, it is known that the extracellular domain of the protein G also binds to an Fab region (Non-patent Document 2), and one antibody molecule can bind to the extracellular domain of the protein G in two regions, the Fc region and the Fab region. In such a binding state, the antibody and the extracellular domain of the protein G cannot be easily dissociated, so that it becomes difficult to recover the antibody.

In the past, the inventors have developed an improved protein consisting of extracellular domain mutants of the protein G with thermal stability, chemical resistance to a denaturing agent, resistance to a proteolytic enzyme, and the like (these properties are also generally referred to as "protein stability" in brief) (Patent Document 5 and Patent Document 6), and have further developed an improved protein with decreased binding property to the Fc region of immunoglobulin and/or decreased binding property to the Fab region thereof in the weakly acidic region (Patent Document 7). However, each of these improved proteins contains only one domain exhibiting the antibody binding property.

### [Prior Arts]

### [Patent Documents]

[Patent Document 1] JP 03-501801 W
[Patent Document 2] Japanese Patent No.2764021
[Patent Document 3] JP 03-128400 A
[Patent Document 4] JP 2003-088381 A
[Patent Document 5] JP 2009-95322 A
[Patent Document 6] JP 2009-118749 A
[Patent Document 7] JP 2009-297018 A

### [Non-patent Documents]

[Non-patent Document 1] Bjorck L, Kronvall G. (1984) Purification and some properties of streptococcal protein G, a novel IgG-binding reagent. J Immunol. 133, 969-974.
[Non-patent Document 2] Boyle M. D.P., Ed. (1990) Bacterial Immunoglobulin Binding Proteins. Academic Press, Inc., San Diego, CA, USA.
[Non-patent Document 3] Gallagher T, Alexander P, Bryan P, Gilliland GL. (1994) Two crystal structures of the B1 immunoglobulin-binding domain of streptococcal protein G and comparison with NMR. Biochemistry 19, 4721-4729.
[Non-patent Document 4] Gagnon P. (1996) Purification Tools for Monoclonal Antibodies, Validated Biosystems Inc., Tucson, AZ, USA.

### Summary of Invention

### Problems to be Solved by the Invention

Therefore, the technical problem solved by the present invention is to solve the points at issue in the above-mentioned prior art, and further to provide a capturing agent for the antibody, the immunoglobulin G or the protein having the Fc region of immunoglobulin G (such as the antibody), which is characterized in that the former protein, which is useful as a filler for an affinity chromatography for purifying the antibody, is immobilized.

More specifically, another problem to be solved by the invention is to provide a capturing agent for the antibody and the like by means of a superior protein with more decreased binding property to the Fc region of immunoglobulin and/or more decreased binding property to the Fab region thereof in the weakly acidic region in comparison with a protein containing an extracellular domain of a wild-type protein G without spoiling the high antibody binding property in the neutral region, thereby allowing the antibody to be easily captured and recovered without denaturing it.

Furthermore, the technical problem is to provide a column for the chromatography for separating and purifying the protein filled with the capturing agent, especially a column for the affinity chromatography for purifying the antibody.

### Means for Solving the Problems

The inventors have considered that, in order to prevent the antibody from being degraded by a strong acid when the antibody is eluted with an acid from the solid support to which the extracellular domain of the protein G is immobilized, an amino acid sequence of the extracellular domain of the protein G should be modified so that the antibody can be eluted from the solid support with a weakly acidic solution. And through extensive research, they have developed a protein comprising a tandem-type multimer constituted by tandemly connecting extracellular domain mutants of the protein G which is an antibody-binding protein, and have verified that the protein has the same binding property to the Fc region of immunoglobulin in the neutral region as that of the wild-type protein G, that the binding property to the Fc region of immunoglobulin in the weakly acidic region is largely decreased in comparison with a tandem-type multimer of the extracellular domains of the wild-type protein G, further that the protein comprising the tandem-type multimer has similar effects for the Fc region of human immunoglobulins included in different subclasses, such as IgG1 and IgG3, and moreover that the tandem-type multimer has superior antibody-binding property in the neutral region in comparison with a monomer consisting of the same domain mutant.

The inventors then found that a capturing agent for an antibody, immunoglobulin G or a protein comprising an Fc region of the immunoglobulin G could be prepared by immobilizing the protein consisting of a tandem-type multimer of extracellular domain mutants to the water-insoluble solid support via mainly an amino group, and that the capturing agent would show a significantly excellent binding property such as a high static binding capacity (SBC) defined below for a capturing agent for the proteins such as the antibody when it was used as a column-filler, leading to the completion of the present invention.

Namely, each aspect of the present invention is as follows.

### Aspect 1

A capturing agent for an antibody, immunoglobulin G or a protein comprising an Fc region of the immunoglobulin G, wherein a protein consisting of a tandem-type multimer of extracellular domain mutants that have binding property to the protein comprising the Fc region of the immunoglobulin G is immobilized to a water-insoluble solid support via mainly an amino group.

### Aspect 2

The capturing agent according to the aspect 1, which is a capturing agent for an antibody, immunoglobulin G or a protein comprising an Fc region of the immunoglobulin G, wherein a protein consisting of a tandem-type multimer of extracellular domain mutants that have binding property to the protein comprising the Fc region of the immunoglobulin G is immobilized to a water-insoluble solid support, and a static binding capacity (SBC) is 40 (mg-human IgG/ml-gel) or more.

### Aspect 3

The capturing agent according to the aspect 2, wherein the static binding capacity (SBC) is 44 (mg-human IgG/ml-gel) or more

### Aspect 4

The capturing agent according to any one of the aspects 1 to 3, wherein the tandem-type multimer is a tandem-type trimer.

### Aspect 5

The capturing agent according to any one of the aspects 1 to 4, wherein the extracellular domain mutants constituting the multimer are the same as one another.

### Aspect 6

The capturing agent according to any one of the aspects 1 to 5, wherein each of the extracellular domain mutants is connected by a linker sequence.

### Aspect 7

The capturing agent according to any one of the aspects 1 to 6, wherein the extracellular domain having the binding property to the protein comprising the Fc region of immunoglobulin G is any one of B1, B2 and B3 of a protein G from streptococcus of genus Streptococcus.

### Aspect 8

The capturing agent according to any one of the aspects 1 to 7, wherein the protein has the binding property to the Fc region of immunoglobulin G, and
at least binding property of the protein to an Fab region of immunoglobulin G and/or binding property of the protein to the Fc region in a weakly acidic region is decreased in comparison with a protein consisting of a tandem-type multimer of B domain of a wild-type protein G.

### Aspect 9

The capturing agent according to any one of the aspects 1 to 8, wherein
at least one of the extracellular domain mutants constituting the multimer is a mutant protein of B1 domain protein of the wild-type protein G,
the mutant protein consists of an amino acid sequence represented by (a) or of the amino acid sequence obtained by deleting, substituting, inserting or adding one or several amino acid residues in the amino acid sequence represented by (a),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with a B1 domain protein of the wild-type protein G.
(a)AspThrTyrLysLeuIleLeuAsnGlyLysX11LeuLysGlyGluThrX17ThrGluAlaValX22 AlaAla X25AlaGluLysValPheLysX32TyrAlaX35X36X37GlyValX40GlyX42TrpThrTyrAspX47X48ThrLys ThrPheThrValThrGlu
(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or Leu; X47 is Asp or Pro; X48 is Ala, Lys or Glu; X22 is Asp; X25 is Thr; X32 is Gln; X40 is Asp; X42 is Glu; and X11 is Thr, and X17 is Thr simultaneously.)

### Aspect 10

The capturing agent according to any one of the aspects 1 to 8, wherein
the at least one extracellular domain mutant constituting the multimer is a mutant protein of B2 domain protein of the wild-type protein G,
the mutant protein consists of an amino acid sequence represented by (b) or of the amino acid sequence obtained by deleting, substituting, inserting or adding one or several amino acid residues in the amino acid sequence represented by (b),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B2 domain protein of the wild-type protein G.
(b)ThrThrTyrLysLeuValIleAsnGlyLysX11LeuLysGlyGluThrX17ThrGluAlaValX22AlaAlaX 25AlaGluLysValPheLysX32TyrAlaX35X36X37GlyValX40GlyX42TrpThrTyrAspX47X48ThrLysT hrPheThrValThrGlu
(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; X42 is Glu; and X11 is Thr and X17 is Thr simultaneously.)

### Aspect 11

The capturing agent according to any one of the aspects 1 to 8, wherein
the at least one extracellular domain mutant constituting the multimer is a mutant protein of B3 domain protein of the wild-type protein G,
the mutant protein consists of an amino acid sequence represented by (c) or of the amino acid sequence obtained by deleting, substituting, inserting or adding one or several amino acid residues in the amino acid sequence represented by (c),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B3 domain protein of the wild-type protein G.
(c)ThrThrTyrLysLeuValIleAsnGlyLysX11LeuLysGlyGluThrX17ThrLysAlaValX22AlaGluX 25AlaGluLysAlaPheLysX32TyrAlaX35X36X37GlyValX40GlyValTrpThrTyrAspX47X48ThrLysTh rPheThrValThrGlu
(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; and X11 is Thr and X17 is Thr simultaneously.)

### Aspect 12

The capturing agent according to any one of the aspects 1 to 8, wherein
the at least one extracellular domain mutant constituting the multimer is a mutant protein of B1 domain protein of the wild-type protein G,
the mutant protein consists of an amino acid sequence represented by (d) or of the amino acid sequence obtained by deleting, substituting, inserting or adding one or several amino acid residues in the amino acid sequence represented by (d),
the mutant protein has the binding property to the Fc region of immunoglobulin G, and
the mutant protein has the binding property to the Fab region of immunoglobulin G and/or the binding property to the Fc region in the weakly acidic region is decreased in comparison with B1 domain protein of the wild-type protein G.
(d)AspThrTyrLysLeuIleLeuAsnGlyLysX11LeuLysGlyGluThrX17ThrGluAlaValX22AlaAla X25AlaGluLysValPheLysX32TyrAlaAsnAspAsnGlyValX40GlyX42TrpThrTyrAspAspAlaThrLysT hrPheThrValThrGlu
(In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

### Aspect 13

The capturing agent according to any one of the aspects 1 to 8, wherein
the at least one extracellular domain mutant constituting the multimer is a mutant protein of B2 domain protein of the wild-type protein G,
the mutant protein consists of an amino acid sequence represented by (e) or of the amino acid sequence obtained by deleting, substituting, inserting or adding one or several amino acid residues in the amino acid sequence represented by (e),
the mutant protein has the binding property to the Fc region of immunoglobulin G, and
the mutant protein has the binding property to the Fab region of immunoglobulin G and/or the binding property to the Fc region in the weakly acidic region is decreased in comparison with B2 domain protein of the wild-type protein G.
(e)ThrThrTyrLysLeuValIleAsnGlyLysX11LeuLysGlyGluThrX17ThrGluAlaValX22AlaAlaX 25AlaGluLysValPheLysX32TyrAlaAsnAspAsnGlyValX40GlyX42TrpThrTyrAspAspAlaThrLysThr PheThrValThrGlu
(In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

### Aspect 14

The capturing agent according to any one of the aspects 1 to 8, wherein
the at least one extracellular domain mutant constituting the multimer is a mutant protein of B3 domain protein of the wild-type protein G,
the mutant protein consists of an amino acid sequence represented by (f) or of the amino acid sequence obtained by deleting, substituting, inserting or adding one or several amino acid residues in the amino acid sequence represented by (f),
the mutant protein has the binding property to the Fc region of immunoglobulin G, and
the mutant protein has the binding property to the Fab region of immunoglobulin G and/or the binding property to the Fc region in the weakly acidic region is decreased in comparison with B3 domain protein of the wild-type protein G.
(f)ThrThrTyrLysLeuValIleAsnGlyLysX11LeuLysGlyGluThrX17ThrLysAlaValX22AlaGluX 25AlaGluLysAlaPheLysX32TyrAlaAsnAspAsnGlyValX40GlyValTrpThrTyrAspAspAlaThrLysThr PheThrValThrGlu
(In the amino acid sequence, X22 represents Asp or His; X25 represents Thr or His; X32 represents Gln or His; X40 represents Asp or His; X11 represents Thr or Arg; and X17 represents Thr or Ile, respectively, with the proviso that a case is excluded where X22 is Asp; X25 is Thr; X32 is Gln; X40 is Asp; and X11 is Thr and X17 is Thr simultaneously.)

### Aspect 15

The capturing agent according to any one of the aspects 1 to 8, wherein
the at least one extracellular domain mutant constituting the multimer is a mutant protein of B1 domain protein of the wild-type protein G,
the mutant protein consists of an amino acid sequence represented by (g) or of the amino acid sequence obtained by deleting, substituting, inserting or adding one or several amino acid residues in the amino acid sequence represented by (g),
the mutant protein has the binding property to the Fc region of immunoglobulin G, and
the mutant protein has the binding property to the Fc region in the weakly acidic region is decreased in comparison with B1 domain protein of the wild-type protein G.
(g)AspThrTyrLysLeuIleLeuAsnGlyLysThrLeuLysGlyGluThrThrThrGluAlaValX22AlaAlaX 25AlaGluLysValPheLysX32TyrAlaAsnAspAsnGlyValX40GlyX42TrpThrTyrAspAspAlaThrLysThr PheThrValThrGlu
(In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

### Aspect 16

The capturing agent according to any one of the aspects 1 to 8, wherein
the at least one extracellular domain mutant constituting the multimer is each of mutant proteins of B2 domain protein of the wild-type protein G,
the mutant protein consists of an amino acid sequence represented by (h) or of the amino acid sequence obtained by deleting, substituting, inserting or adding one or several amino acid residues in the amino acid sequence represented by (h),
the mutant protein has the binding property to the Fc region of immunoglobulin G, and
the mutant protein has the binding property to the Fc region in the weakly acidic region is decreased in comparison with B2 domain protein of the wild-type protein G.
(h)ThrThrTyrLysLeuValIleAsnGlyLysThrLeuLysGlyGluThrThrThrGluAlaValX22AlaAlaX2 SAlaGluLysValPheLysX32TyrAlaAsnAspAsnGlyValX40GlyX42TrpThrTyrAspAspAlaThrLysThrP heThrValThrGlu
(In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

### Aspect 17

The capturing agent according to any one of the aspects 1 to 8, wherein
the at least one extracellular domain mutant constituting the multimer is each of mutant proteins of B3 domain protein of the wild-type protein G,
the mutant protein consists of an amino acid sequence represented by (i) or of the amino acid sequence obtained by deleting, substituting, inserting or adding one or several amino acid residues in the amino acid sequence represented by (i),
the mutant protein has the binding property to the Fc region of immunoglobulin G, and
the mutant protein has the binding property to the Fc region in the weakly acidic region is decreased in comparison with B3 domain protein of the wild-type protein G.
(i)ThrThrTyrLysLeuValIleAsnGlyLysThrLeuLysGlyGluThrThrThrLysAlaValX22AlaGluX2 5AlaGluLysAlaPheLysX32TyrAlaAsnAspAsnGlyValX40GlyValTrpThrTyrAspAspAlaThrLysThrP heThrValThrGlu
(In the amino acid sequence, X22 represents Asp or His; X25 represents Thr or His; X32 represents Gln or His; and X40 represents Asp or His, respectively, with the proviso that a case is excluded where X22 is Asp; and X25 is Thr; X32 is Gln and X40 is Asp simultaneously.)

### Aspect 18

The capturing agent according to any one of the aspects 1 to 8, wherein at least one of the extracellular domain mutants constituting the multimer consists of an amino acid sequence represented by any one of SEQ ID NO. 13 to 20 or an amino acid sequence obtained by deleting, substituting, inserting or adding one or several amino acid residues in the amino acid sequences represented by any one of SEQ ID NO. 13 to 20.

### Aspect 19

The capturing agent according to any one of the aspects 1 to 8, wherein the three extracellular domain mutants constituting the trimer consist of the amino acid sequence represented by SEQ ID NO. 19 or the amino acid sequence obtained by deleting, substituting, inserting or adding one or several amino acid residues in the amino acid sequence represented by SEQ ID NO. 19.

### Aspect 20

A column for a chromatography for separating and purifying the protein, which is filled with the capturing agent according to any one of the aspects 1 to 19.

### Advantages of Invention

The present invention can provide the capturing agent which is obtained by immobilizing a protein consisting of the tandem-type multimer to a water-insoluble solid support via mainly an amino group, in which, although, while the original antibody binding property of the protein in the neutral region is being maintained, the binding property of the protein to the Fc region of human immunoglobulins G included in the different subclasses such as IgGland IgG3 in the weakly acidic region is largely decreased, for example, in comparison with a tandem-type multimer of a B1 domain of a wild-type protein G consisting of an amino acid sequence represented by [SEQ ID NO. 1].

Furthermore, the capturing agent has the significantly excellent binding property such as the high static binding capacity (SBC) defined below for the capturing agent for the proteins such as the antibody when it is filled in the column.

Therefore, in the column for the chromatography for separating and purifying the protein in which the capturing agent of the present invention containing the protein is filled, the captured antibody can be more easily eluted without denaturation in the weakly acidic region.

It is noted that, in Patent Document 7 cited herein, although a general concept corresponding to the tandem-type multimer of the present invention is disclosed, a synthesis example was not actually described and the remarkable effects as described above are not also described or suggested at all.

### Brief Description of Drawings

FIG. 1 shows gene structure of the protein G derived from Streptococcus sp. G148.
FIG. 2 shows amino acid sequences of the antibody binding domains of the proteins G (underlined parts are different parts from the B1 domain).
FIG. 3 shows a base sequence of the gene of the protein G derived from Streptococcus sp. G148 (SEQ ID NO. 30) (an underlined part corresponds to a structural gene, and bold letters correspond to the antibody binding domains).
FIG. 4 shows an n-terminal sequence of an oxaloacetate decarboxylase alpha-subunit c-terminal domain (OXADac) - protein G mutant fusion protein (SEQ ID NO. 31) (an underlined part is an amino acid sequence corresponding to the OXADac).
FIG. 5 shows stereoscopic structure of a complex of the B2 domain of the protein G and an Fc region of human immunoglobulin G₁.
FIG. 6 shows stereoscopic structure of a complex of the B3 domain of the protein G and an Fab region of mouse immunoglobulin G₁.
FIG. 7 is graphs showing results of antibody dissociation evaluation of the mutant proteins in the weakly acidic region with immobilized columns (1).
FIG. 8 is graphs showing results of antibody dissociation evaluation of the mutant proteins in the weakly acidic region with immobilized columns (2).
FIG. 9 shows results obtained by evaluating antibody binding dissociation activity of the mutant proteins by the surface plasmon resonance (SPR) method. It shows sensorgrams of M-PG01, M-PG07 and M-PG19, respectively, in that order from the top. Concentration of the mutant proteins is 100 nM, 200 nM, 300 nM, 400 nM and 500 nM.
FIG. 10 is a graph showing pH dependence of antibody avidity (1/K_{D}) of the mutant proteins.
FIG. 11 is a graph showing relative changes of the antibody avidity of the mutant proteins. Dissociation constants (K_{D}) at each pH value are standardized by K_{D} at pH 7.4.
FIG. 12 shows stereoscopic structure of the mutant protein M-PG 19 (left). For comparison, stereoscopic structure of the B1 domain of the wild-type protein G is also shown (right).
FIG. 13 shows domain structure and mutant amino acids of a trimer wild-type PG (CGB01H-3D, FIG. 13 upper) and the mutant-type PG, which is the protein of the present invention, (CGB19H-3D, FIG. 13 under), in both of which a cysteine residue and His tag are fused to the 3' terminal side.
FIG. 14 is graphs showing results of a pH gradient affinity chromatography in an Epoxy-activated CGB01H-3D immobilized columns.
FIG. 15 is graphs showing results of the pH gradient affinity chromatography in an Epoxy-activated CGB19H-3D immobilized columns.
FIG. 16 is graphs showing results of the pH gradient affinity chromatography in a CGB19H-3D immobilized SulfoLink columns.
FIG. 17 is graphs showing results of a pH stepwise change affinity chromatography in the Epoxy-activated CGB01H-3D immobilized columns.
FIG. 18 is graphs showing results of the pH stepwise change affinity chromatography in the Epoxy-activated CGB19H-3D immobilized columns.
FIG. 19 is graphs showing results of the pH stepwise change affinity chromatography in Epoxy-activated immobilized columns.
FIG. 20 is graphs showing results of the pH stepwise change affinity chromatography in the CGB19H-3D immobilized SulfoLink columns.
FIG. 21 is graphs showing comparisons between the tandem-type multimer of the extracellular domain mutants of the protein G and the monomer of the extracellular domain mutant thereof.
FIG. 22 is a graph showing results of the pH gradient affinity chromatography in anDSC-activated CGB19H-3D immobilized columns.

### Embodiments of the Invention

The capturing agent according to the present invention is characterized in that the protein consisting of the tandem-type multimer of extracellular domain mutants that have binding property to the protein comprising the Fc region of the immunoglobulin G is immobilized to the water-insoluble solid support via mainly an amino group. Since said capturing agent always has the excellent antibody-binding property, it is useful as a filler for the column for the chromatography for separating and purifying the protein. Thus, said column filled with the capturing agent can be used for purification and removal; and diagnosis, treatment and detection using the antibody.

### Immobilization of the protein

The capturing agent of the present invention can be prepared by immobilizing the mutant proteins according to the present invention to the water-insoluble solid support (carrier). A water-insoluble carrier used herein includes an inorganic carrier such as a glass bead and silica gel; an organic carrier consisting of a synthesis polymer such as cross-linked polyvinyl alcohol, cross-linked polyacrylate, cross-linked polyacrylamide and cross-linked polystyrene, or polysaccharide such as crystalline cellulose, cross-linked cellulose, cross-linked agarose and cross-linked dextran; a composite carrier such as organic-organic and organic-inorganic obtained by combinations thereof; or the like; among which a hydrophilic carrier is preferable since the nonspecific absorption is relatively little and the selectivity to the antibody, the immunoglobulin G or the protein having the Fc region of immunoglobulin G is excellent. The hydrophilic carrier as used herein refers to a carrier in which a contact angle with water is 60° or less in the case that the compound constituting the carrier is formed into a flat plate. A typical example of such a carrier includes a carrier consisting of polysaccharide such as cellulose, chitosan and dextran, polyvinyl alcohol, saponified ethylene - vinyl acetate copolymer, polyacrylamide, polyacrylic acid, polymethacrylic acid, polymethyl methacrylate, polyacrylic acid-grafted polyethylene, polyacrylamide-grafted polyethylene, glass, or the like.

Commercial products include GCL2000 and GC700 which are porous cellulose gel, Sephacryl S-1000 in which allyl dextran and methylenebisacrylamide are cross-linked by covalent bonds, Toyopearl which is an acrylate-based carrier, SepharoseCL4B which is an agarose-based cross-linked carrier, Eupergit C250L which is polymethacrylamide activated with epoxy groups, and the like. However, the carrier in the present invention is not limited to only these carriers and activated carriers. The above-mentioned carriers may be each independently used, or may be used as a mixture of any two or more thereof. Moreover, based on the purposes and methods for using the antibody capturing agent, the water-insoluble carrier used herein has desirably a wide surface area, and has preferably pores of a suitable size, i.e. porous carrier.

The carrier may be in any form, such as bead-shaped, fiber-shaped and membrane-shaped (including hollow fiber), which can be arbitrarily selected. Due to ease of preparation of a carrier with specific exclusion limit molecular weight, the bead-shaped carrier is particularly preferably used. A bead-shaped carrier with an average particle diameter of 10 µm to 2500 µm is easy to use, and, in particular, from a viewpoint of ease of ligand immobilization reaction, a range from 25µm to 800µm is preferable.

Moreover, it is convenient for the immobilization of ligand that functional groups which can be used for the immobilization reaction of ligand exist on the carrier surface. A typical example of the functional groups includes a hydroxyl group, an amino group, an aldehyde group, a carboxyl group, a thiol group, a silanol group, an amide group, the epoxy group, a succinyl imide group, an acid anhydride groups, an iodoacetyl group, and the like.

In the immobilization of the mutant protein to the above-mentioned carrier, it is more preferable that capture efficiency is improved by decreasing steric hindrance of the mutant protein and further that the mutant protein is immobilized via a hydrophilic spacer to suppress non-specific binding. As the hydrophilic spacer, a derivative of polyalkylene oxide in which, for example, the carboxyl group, the amino group, the aldehyde group, the epoxy group or the like was substituted at the both terminals is preferably used.

The capturing agent according to the present invention is characterized in that the protein consisting of the tandem-type multimer of extracellular domain mutants is immobilized to the water-insoluble solid support via mainly an amino group. The phrase "the protein is immobilized to the water-insoluble solid support via mainly an amino group" used herein means that amino groups comprised in the protein (ligand) are mainly involved in the binding with reacting (active) groups on the solid support. Thus, it includes the case where substantially only -NH₂ group in free amino acid residues comprised in a polypeptide constituting the protein are bound with reacting groups on the solid support. It also includes the case where a plurality kinds of amino acid residues such as free -NH₂ and -SH groups comprised in the polypeptide constituting the protein are bound with the reacting groups on the solid support, the amount of the bound -NH₂ group being the highest in all the bound amino acid residues (or, the -NH₂ group is mainly involved in the binding with the reacting groups on the solid support). In other words, the above conditions do not include the case where the binding is made by means of the epoxy group method (around pH 7 - 8), so that the plurality kinds of amino acid residues such as free -NH₂ and -SH groups comprised in the polypeptide constituting the protein are bound with the reacting groups on the solid support, and the amount of the bound -SH group is more than that of-NH₂ group.

Although a method and a condition for immobilizing the mutant protein which is introduced to the above-mentioned solid support (carrier), and organic compounds used as the spacer are not particularly limited, any method known in the art such as that adopted in the case where the protein and peptide are generally immobilized to the carrier can be used.

For example, a method comprising the steps of: reacting the carrier with cyanogen bromide (CNBr), N,N'-disuccinimidyl carbonate (DSC), epoxide and activated carbonic acid (NHS ester) or the like to activate the carrier; (substituting a functional group with which a compound to be immobilized as the ligand is easier to react in comparison with a functional group which the carrier originally has), and reacting the carrier with the compound to be immobilized as the ligand to immobilize; or an immobilization method comprising the step of: adding a condensing reagent, such as carbodiimide, or a reagent which has a plurality of functional groups in a molecule, such as glutaraldehyde, to a system where the carrier and the compound to be immobilized as the ligand exist to condense and crosslink may be included. However, it is more preferable that an immobilization method in which proteins are not detached easily from the carrier during sterilization or use of the capturing agent is applied.

The capturing agent thus prepared has a higher (larger) static binding capacity (SBC) than a capturing agent that is immobilized to a water-insoluble solid support via mainly the -SH group.

Thus, the capturing agent according to the present invention is characterized in that it has the static binding capacity (SBC) preferably of 40 (mg-human IgG/ml-gel) or more, more preferably of 44 (mg-human IgG/ml-gel) or more, so that it shows the significantly advantageous binding property to the antibody, immunoglobulin G or a protein comprising an Fc region of the immunoglobulin G.

The "static binding capacity" means a maximum antibody-binding capacity of a capturing agent, i.e., an immobilized protein (ligand) itself, which is not affected by a flow rate and the like. The static binding capacity may be determined by the following conditions:
A solution (400 µl) of 25% of the capturing agent (the ligand immobilized to the water-insoluble solid support) in PBS suspension is collected in a conical plastic container. PBS solution (14 ml) of y-globulin derived from human serum (Wako Pharmaceuticals) at 1mg/ml is added to the container, followed by stirring at 25 °C for three hours with a rotary mixer. The concentration of γ-globulin in a supernatant is determined based on absorbance at 280 nm, SBC is then obtained on a basis of material balance.

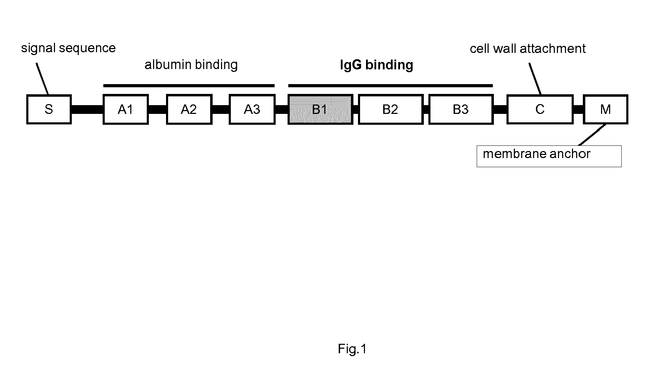

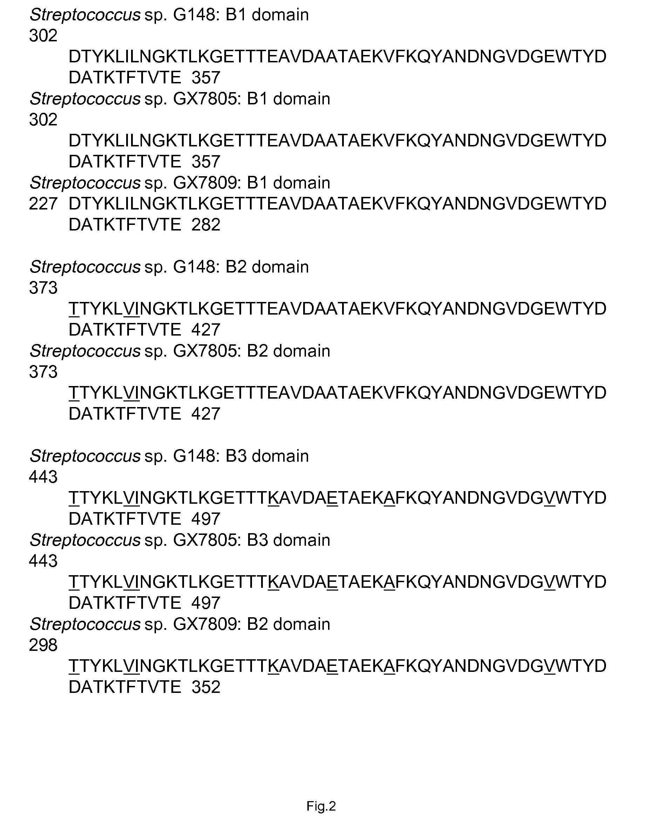

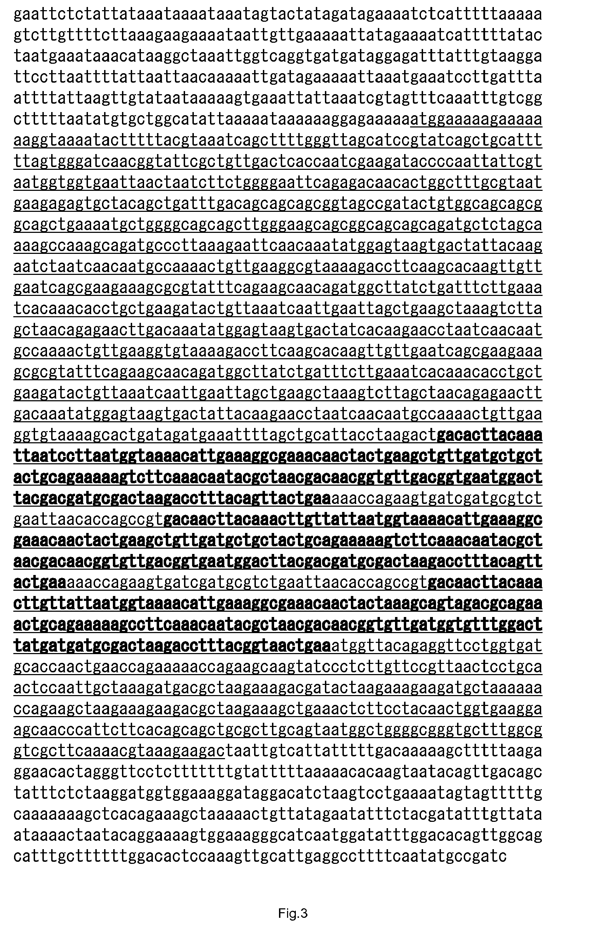

It is known that the protein G, which is the protein derived from streptococus, has the specific binding property to the Fc region of immunoglobulin G which is a kind of the antibody (Reference Document 1), so that the protein G is a protein useful for purification and removal of the antibody utilizing the antibody-binding property of the protein and useful for diagnosis, treatment, inspection and the like using the antibody. The protein G is a multi-domain type membrane protein consisting of a plurality of domains, some extracellular domains of which exhibit the binding property to the protein having the Fc region of immunoglobulin G (hereinafter, referred to as "antibody binding property") (Non-patent Document 2). For example, in a protein G derived from the G148 strain shown in FIG. 1 and FIG. 3 and represented by [SEQ ID NO. 30], three domains of B1, B2 and B3 exhibit the antibody binding property (also written as "C1, C2 and C3 domains" depending on a document). Also, the protein G from the GX7805 strain has the three antibody binding domains, and the protein G from the GX7809 has the two antibody binding domains. These proteins are all the miniature proteins with less than 60 amino acids, and there is high identity among each amino acid sequence (FIG. 2). It is also known that even if the protein G is cut to isolate each domain alone, the antibody binding property is maintained (Reference Document 3).

In the capturing agent according to the present invention, the protein consisting of or comprising the tandem-type multimer of the extracellular domain mutants, which have the binding property to the protein having the Fc region of immunoglobulin G, is immobilized to the water-insoluble solid support. The multimer corresponds to the above-mentioned wild-type protein G and, for example, may be appropriately a dimer or a trimer. Moreover, each extracellular domain mutant constituting the multimer which is contained in the protein of the present invention is different from one another or the same as one another.

Moreover, each extracellular domain mutant may be connected by a linker sequences. Such a linker sequences may be appropriately designed and prepared by those skilled in the art, taking into consideration an amino acid sequence of each mutant and the like.

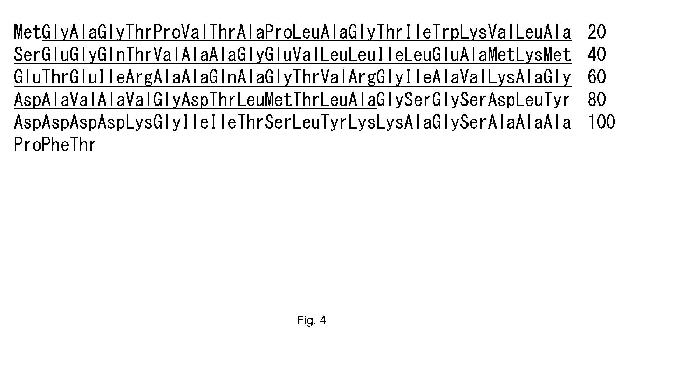

Also, the protein of the present invention may be a fusion protein consisting of a fusion-type amino acid sequence, in which an amino acid sequence of any other proteins is connected to an n-terminal side or a c-terminal side. For example, the protein may be [an amino acid sequence (a)] - a linker sequences E - a protein A, or a protein B - a linker sequences F - [an amino acid sequence (a)] - a linker sequences G - a protein C - a linker sequences H - [an amino acid sequence (c)]. The other amino acid sequences used in such a fusion protein include, for example, an amino acid sequence of an oxaloacetate decarboxylase alpha-subunit c-terminal domain (OXADac) shown in FIG. 4 or represented by [SEQ ID NO. 31]. As shown in the following examples, an OXADac - protein G mutant fusion protein in this case can have a plurality of functions of avidin binding property resulting from the OXADac region and the antibody binding property resulting from the protein G mutant region in a single molecule.

For example, in the case of synthesis of the protein of the present invention in a form of a fusion protein with the His tag or other proteins, even if the fusion protein is decomposed between the tag and a mutant protein or between the other proteins and the protein of the present invention with a sequence-specific proteolytic enzyme after the synthesis, one or several amino acid residues may remain in the n-terminal side or the c-terminal side of the protein of the present invention, and also, in the case of production of the protein of the present invention with an Escherichia coli or the like, a methionine or the like corresponding to an initiation codon may be added to the n-terminal side, but the addition of these amino acid residues does not change the following activity of the protein of the present invention. Moreover, the addition of these amino acid residues does not neutralize effects of designed mutation. Therefore, the protein of the present invention contains the mutation naturally. In order to prepare the protein of the present invention without the addition of such amino acid residues, the protein is produced, for example, with the Escherichia coli or the like, and furthermore the amino acid residue of the N-terminal is cut selectively with an enzyme such as methionyl aminopeptidase or the like (Reference Document 7) to separate and purify the protein from a reaction mixture by the chromatography or the like.

As a suitable example of the extracellular domain, which is an origin of the mutant constituting the tandem-type multimer contained in the protein of the present invention and which has the binding property to the protein having the Fc region of immunoglobulin G, any one of B1 B2 and B3 of the protein G from the streptococcus of genus Streptococcus may be cited.

The protein of the present invention has the binding property to the Fc region of immunoglobulin G, and has superior properties that at least binding property of the protein to the Fab region of immunoglobulin G (IgG1 and IgG3) and/or binding property of the protein to the Fc region in the weakly acidic region is significantly decreased in comparison with the protein comprising the tandem-type multimer of B domain of the wild-type protein G.

As preferable aspects of at least one of the extracellular domain mutants constituting the tandem-type multimer contained in the protein of the present invention, mutant proteins are listed below.

A. The first aspect of the mutant proteins of the present invention is described in the following (1) (2). (1) A mutant protein prepared by substituting another amino acid residue for any one or more of amino acid residues: Asp22, Ala24, Thr25, Lys28, Val29, Lys31, Gln32, Asn35, Asp36, Gly38, Asp40, Glu42, Thr44, as a target part for the mutation in a B1 or B2 domain protein of a wild-type protein G consisting of an amino acid sequence represented by SEQ ID NO. 1 or 2, characterized in that an amino acid residue described in any one of the following (i) to (iii) is substituted for each amino acid residue as the target part for the mutation, wherein the mutant protein has the binding property to the Fc region of immunoglobulin G, and wherein the binding property of the mutant protein to the Fc region of immunoglobulin G in the weakly acidic region is decreased in comparison with the B1 or B2 domain protein of the wild-type protein G. (i) Substitution to a charged amino acid residue when the amino acid residue as the target part for the mutation is an uncharged amino acid residue.
(ii) Substitution to a charged amino acid residue with opposite electric charge when the amino acid residue as the target part for the mutation is a charged amino acid residue.
(iii) Substitution to a histidine residue for the amino acid residue as the target part for the mutation.

(2) A mutant protein prepared by substituting another amino acid residue for any one or more of amino acid residues: Asp22, Thr25, Lys28, Lys31, Gln32, Asn35, Asp36, Gly38, Asp40, Thr44, as a target part for the mutation in a B3 domain protein of a wild-type protein G consisting of an amino acid sequence represented by SEQ ID NO. 3, characterized in that an amino acid residue described in any one of the following (i) to (iii) is substituted for each amino acid residue as the target part for the mutation, wherein the mutant protein has the binding property to the Fc region of immunoglobulin G, and wherein the binding property of the mutant protein to the Fc region of immunoglobulin G in the weakly acidic region is decreased in comparison with the B3 domain protein of the wild-type protein G.
   (i) Substitution to a charged amino acid residue when the amino acid residue as the target part for the mutation is an uncharged amino acid residue.
   (ii) Substitution to a charged amino acid residue with opposite electric charge when the amino acid residue as the target part for the mutation is a charged amino acid residue.
   (iii) Substitution to a histidine residue for the amino acid residue as the target part for the mutation.

The above-mentioned mutant proteins described in (1) and (2) are designed based on a target part for the mutation selected as follows and the amino acid residue which is substituted for the target part, and are obtained by a genetic engineering technique.

### [Selection of the target part for the mutation and specification of the amino acid residue which is substituted, based on an analysis of a surface bound to the Fc]

The part where the mutation is transduced for designing the amino acid sequence of the mutant protein of the present invention is selected by using a three-dimensional atomic coordinate data (Reference Document 4) of a complex in which the B2 domain of the protein G and the Fc region of immunoglobulin G are bound together. To decrease the antibody-binding property of the extracellular domain of the protein G in the weakly acidic region, substitution for an amino acid residue in a binding surface of the extracellular domain of the protein G directly related to the binding to the Fc region and substitution for surrounding amino acid residues thereof should be executed from the wild-type to a non wild-type. Therefore, first, in the complex in which the B2 domain of the protein G and the Fc region of immunoglobulin G had been bound together, amino acid residues of the B2 domain of the protein G within a fixed distance range from the Fc region were specified, and were selected as candidates of the target part for the mutation. Next, to minimize structural destabilization of the extracellular domain of the protein G associated with the amino acid substitution, among the above-mentioned candidates, only amino acid residues of the B2 domain of the protein G which had been exposed on a molecular surface were determined as the target parts for the mutation.

Thus, specifically, as shown in the following examples, by setting the above-mentioned distance range to 6.5 angstroms or less and exposed surface area ratio to 40 % and over, thirteen amino acid residues of Asp22, Ala24, Thr25, Lys28, Val29, Lys31, Gln32, Asn35, Asp36, Gly38, Asp40, Glu42 and Thr44 were selected as the target part for the mutation in the wild-type amino acid sequence of the B2 domain of the protein G (SEQ ID NO. 2).

Moreover, as described above, since there exists extremely high sequence identity among each extracellular domain of the protein G and little difference among each stereoscopic structure of the B1. B2 and B3 domains, the finding on the stereoscopic structure of the B2 domain - Fc complex is applicable to a B1 domain - Fc complex and a B3 domain - Fc complex. Therefore, not only in the B2 domain but also in the B1 domain and the B3 domain, the thirteen amino acid residues as the target part for the mutation resulting from the stereoscopic structure of the B2 domain - Fc complex can be selected as the target part for the mutation, as long as the same kind of amino acid is in a corresponding position. Namely, thirteen amino acid residues of Asp22, Ala24, Thr25, Lys28, Val29, Lys31, Gln32, Asn35, Asp36, Gly38, Asp40, Glu42 and Thr44, in the wild-type amino acid sequence of the B1 domain of the protein G (SEQ ID NO. 1), and ten amino acid residues of Asp22, Thr25, Lys28, Lys31, Gln32, Asn35, Asp36, Gly38, Asp40 and Thr44, in the wild-type amino acid sequence of the B3 domain of the protein G (SEQ ID NO. 3), were selected as the target part for the mutation.

On the other hand, as the amino acid residue which was substituted for the original amino acid residue as the target part for the mutation, any one of the following (i) to (iii) was specified.
(i) When the wild-type amino acid residue as the target part for the mutation is an amino acid with an uncharged side-chain (Gly, Ala, Val, Leu, Ile, Ser, Thr, Asn, Gln, Phe, Tyr, Trp, Met, Cys, Pro) , an amino acid with a charged side-chain (Asp, Glu, Lys, Arg, His) is substituted. Since a chemical state of a charged amino acid is greatly changed depending on pH, the charged amino acid can cause to change the antibody-binding property of the B2 domain of the protein G in the neutral region and the weakly acidic region.
(ii) When the wild-type amino acid residue as the target part for the mutation is a charged amino acid, a charged amino acid with opposite electric charge is substituted. Similarly to above, since the chemical state of the charged amino acid is greatly changed depending on pH, the charged amino acid can cause to change the antibody-binding property of the B2 domain of the protein G in the neutral region and the weakly acidic region.
(iii) When the wild-type amino acid residue as the target part for the mutation is other than a histidine, the histidine is substituted. Since a chemical state of the histidine is greatly changed in the neutral region and the weakly acidic region, the histidine can cause to change the antibody-binding property of the B2 domain of the protein G in the neutral region and the weakly acidic region.

Specifically, as shown in the following examples, Lys, Arg or His for Asp22; Asp, Glu, Lys, Arg or His for Ala24 (only in the B1, B2 domains); Asp, Glu, Lys, Arg or His for Thr25; Asp, Glu or His for Lys28; Asp, Glu, Lys, Arg or His for Val29 (only in the B1, B2 domains); Asp, Glu or His for Lys31; Asp, Glu, Lys, Arg or His for Gln32; Asp, Glu, Lys, Arg or His for Asn35; Lys, Arg or His for Asp36; Asp, Glu, Lys, Arg or His for Gly38; Lys, Arg or His for Asp40; Lys, Arg or His for Glu42 (only in the B1, B2 domains); and Asp, Glu, Lys, Arg or His for Thr44 were specified as the amino acid residue in positions where the substitution is executed. However, a case is excluded where an amino acid sequence according to the specification of these amino acid residues is that in which Lys is substituted for Asn35 and/or Glu is substituted for Asp36 and in which an amino acid sequence except the positions where the substitution is executed is the same as an amino acid sequence of each cell membrane domain of the wild-type protein G. Consequently, the first aspect of the mutant proteins is distinguished from the following mutant protein with the improved stability of the cell membrane domain of the protein G claimed by the inventors.

B. The second aspect of the mutant proteins of the present invention is described in the following (3).
(3) A mutant protein characterized by being prepared by substituting other kinds of amino acid residue other than a cysteine for any one or more of amino acid residues: Lys10, Thr11, Lys13, Gly4, Glug5, Thr16, Thr17, Asn35, Asp36, Gly38, as a target part for the mutation in the B1, B2 or B3 domain protein of the wild-type protein G consisting of the amino acid sequence represented by any one of SEQ ID NO. 1 to 3, wherein the mutant protein has the binding property to the Fc region of immunoglobulin G, and wherein the binding property of the mutant protein to the Fab region of immunoglobulin G is decreased in comparison with each corresponding B1, B2 or B3 domain protein of the wild-type protein G. The above-mentioned mutant protein described in (3) is designed based on a target part for the mutation selected as follows and an amino acid residue which is substituted for the target part, and is obtained by a genetic engineering technique.

### [Selection of the target part for the mutation and specification of the amino acid residue which is substituted, based on an analysis of a surface bound to the Fab]

The part where the mutation is transduced for designing the amino acid sequence of the mutant protein of the present invention is selected by using a three-dimensional atomic coordinate data (Reference Document 5) of a complex in which the B3 domain of the protein G and the Fab region of immunoglobulin G are bound together. It is known that the extracellular domain of the protein G binds to both the Fc region and the Fab region of immunoglobulin G (Reference Document 2). Therefore, one antibody molecule can simultaneously bind to the extracellular domain of a plurality of proteins G, and, in such a state, since interaction between the antibody and the extracellular domain of the proteins G is multivalent, it cannot be cut easily. Thus, to decrease the antibody-binding property of the extracellular domain of the protein G in the weakly acidic region, substitution for an amino acid residue in a binding surface of the extracellular domain of the protein G directly related to the binding to the Fab region should be executed from the wild-type to the non wild-type. Hence, first, in the complex in which the B3 domain of the protein G and the Fab region of immunoglobulin G had been bound together, amino acid residues of each cell membrane B3 domain of the protein G within a fixed distance range from the Fab region were specified, and were selected as candidates of the target part for the mutation. Next, to minimize the structural destabilization of the extracellular domain of the protein G associated with the amino acid substitution, among the above-mentioned candidates, only amino acid residues of the B3 domain of the protein G which had been exposed on the molecular surface were determined as the target parts for the mutation.

Specifically, as shown in the following examples, by setting the above-mentioned distance range to 4.0 angstroms or less and exposed surface area ratio to 40 % and over, ten amino acid residues of Lys10, Thr11, Lys13, Glyl4, Glu15, Thr16, Thr17, Asn35, Asp36 and Gly38 were selected as the target part for the mutation in the wild-type amino acid sequence of the B3 domain of the protein G represented by [SEQ ID NO. 3]. Moreover, as described above, since there exists extremely high identity among each extracellular domain of the protein G, each of the B1, B2 and B3 domains has the target parts for the mutation in common. Therefore, not only in the B3 domain but also in the B1 domain and the B2 domain, the ten amino acid residues can be selected as the target parts for the mutation.

On the other hand, the amino acid residue which is substituted for the original amino acid residue as the target part for the mutation can be specified by the following method. (iv) Other kinds of amino acid residue other than the wild-type amino acid and the cysteine is substituted. Consequently, eliminating a risk of a crosslinking reaction by the transduction of the cysteine, the decrease in the binding property with the Fab region due to the mutation of the wild-type amino acid can be produced.

Specifically, as seen in the examples below, amino acid residues other than Lys and Cys for Lys10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38; were specified as the amino acid residues which would be substituted for each extracellular domain protein of the wild-type protein G. However, a case is excluded where an amino acid sequence according to the above-mentioned selection of the amino acid residues is that in which Lys is substituted for Asn35 and/or Glu is substituted for Asp36 and in which an amino acid sequence except the positions where the substitution is executed is the same as the amino acid sequence of each cell membrane domain of the wild-type protein G. Consequently, the first aspect of the mutant proteins is distinguished from the following mutant protein with the improved stability of the cell membrane domain of the protein G claimed by the inventors.

C. The third aspect of the mutant proteins of the present invention comprises both the above-mentioned substitution of the amino acid residue for improving the binding property to the Fc region of immunoglobulin and the above-mentioned substitution of the amino acid residue for improving the binding property to the Fab region.

### [Selection of the target part for the mutation and specification of the amino acid residue which is substituted, based on the analyses of the surfaces bound to the Fc and the Fab]

By combining the above-mentioned amino acid residue which is specified based on the analysis of the surface bound to the Fc and is substituted for the target part for the mutation and the above-mentioned amino acid residue which is specified based on the analysis of the surface bound to the Fab and is substituted for the target part for the mutation, the selection of the target part for the mutation and the specification of the amino acid residue which is substituted are performed. Specifically, twenty amino acid residues of Asp22, Ala24, Thr25, Lys28, Val29, Lys31, Gln32, Asp40, Glu42, Thr44, Lys10, Thr11, Lys13, Gly14, Glu15, Thr16, Thr17, Asn35, Asp36 and Gly38 in each wild-type amino acid sequence of the B1 and B2 domains of the protein G (SEQ ID NO. 1, 2) are selected as the target parts for the mutation. Among the target parts for the mutation, Asp22, Ala24, Thr25, Lys28, Val29, Lys31, Gln32, Asp40, Glu42 and Thr44 are target parts for improving the binding property to the Fc region, and Lys10, Thr11, Lys13, Gly14, Glu15, Thr16 and Thr17 are target parts for improving the binding property to the Fab region. Asn35, Asp36 and Gly38 are parts for improving the binding property to the Fc region as well as parts for improving the binding property to the Fab region. Therefore, the substitution to the amino acid residue for Asn35, Asp36 or Gly38 for improving the binding property to the Fc region described in A is also the substitution to another amino acid residue other than the cysteine residue for simultaneously improving the binding property to the Fab region.

Namely, as used herein, "comprising both" the substitutions of the amino acid residue is defined as not only the case that the substitutions of the amino acid residue are combined when the target part for the mutation for improving the binding property to the Fc region and the target part for the mutation for improving the binding property to the Fab region are differently selected, but also the case that the substitution of the amino acid residue described in A is executed after the same part is selected as the target part for the mutation for improving the binding property to both the regions.

Lys, Arg or His for Asp22; Asp, Glu, Lys, Arg or His for Ala24; Asp, Glu, Lys, Arg or His for Thr25; Asp, Glu, or His for Lys28; Asp, Glu, Lys, Arg or His for Val29; Asp, Glu or His for Lys31; Asp, Glu, Lys, Arg or His for Gln32; Lys, Arg or His for Asp40; Lys, Arg or His for Glu42; Asp, Glu, Lys, Arg or His for Thr44; amino acid residues other than Lys and Cys for Lys10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38 are selected as the amino acid residues which would be substituted.

On the other hand, in the B3 domain of the protein G, seventeen amino acid residues of Asp22, Thr25, Lys28, Lys31, Gln32, Asp40, Thr44, Lys10, Thr11, Lys13, Gly14, Glu15, Thr16, Thr17, Asn35, Asp36 and Gly38 in the wild-type amino acid sequence (SEQ ID NO. 3) are selected as the target parts for the mutation. Among the amino acid residues, Asp22, Thr25, Lys28, Lys31, Gln32, Asp40 and Thr44 are target parts for the mutation for improving the binding property to the Fc region, and Lys10, Thr11, Lys13, Gly4, Glu15, Thr16 and Thr17 are target parts for the mutation for improving the binding property to the Fab region. Also, the amino acid residues have a commonality in that Asn35, Asp36 and Gly38 are the parts for improving the binding property to the Fc region as well as the parts for improving the binding property to the Fab region, so that, similar to the above-mentioned B1 or B2 domain of the protein G, the substitution to the amino acid residue for Asn35, Asp36 or Gly38 for improving the binding property to the Fc region described in A is also the substitution to another amino acid residue other than the cysteine residue for simultaneously improving the binding property to the Fab region.

However, a case is excluded where an amino acid sequence selected based on the above-mentioned selection of the amino acid residues is that in which Lys is substituted for Asn35 and/or Glu is substituted for Asp36 and in which an amino acid sequence except the positions where the substitution is executed is the same as the amino acid sequence of each cell membrane domain of the wild-type protein G. Consequently, the first aspect of the mutant proteins is distinguished from the following mutant protein with the improved stability of the cell membrane domain of the protein G claimed by the inventors.

Specific examples of the mutant proteins according to the present invention include the following a) to c).
a) A mutant protein of B1 domain protein of the wild-type protein G consisting of an amino acid sequence represented by (a) or of the amino acid sequence obtained by deleting, substituting, inserting or adding one or several amino acid residues in the amino acid sequence represented by (a), wherein
   the mutant protein has the binding property to the Fc region of immunoglobulin G, and
   the mutant protein has at least the binding property to the Fab region of immunoglobulin G and/or the binding property to the Fc region in the weakly acidic region is decreased in comparison with a B1 domain protein of the wild-type protein G.
   (a) AspThrTyrLysLeuIleLeuAsnGlyLysX11LeuLysGlyGluThrX17ThrGluAlaValX22Ala AlaX25AlaGluLysValPheLysX32TyrAlaX35X36X37GlyValX40GlyX42TrpThrTyrAspX47X4 8ThrLysThrPheThrValThrGlu
      (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or Leu; X47 is Asp or Pro; X48 is Ala, Lys or Glu; X22 is Asp; X25 is Thr; X32 is Gln; X40 is Asp; X42 is Glu; and X11 is Thr, and X17 is Thr simultaneously.)
b) A mutant protein of B2 domain protein of the wild-type protein G consisting of an amino acid sequence represented by (b) or of the amino acid sequence obtained by deleting, substituting, inserting or adding one or several amino acid residues in the amino acid sequence represented by (b), wherein
   the mutant protein has the binding property to the Fc region of immunoglobulin G, and
   the mutant protein has at least the binding property to the Fab region of immunoglobulin G and/or the binding property to the Fc region in the weakly acidic region is decreased in comparison with B2 domain protein of the wild-type protein G.
   (b)ThrThrTyrLysLeuValIleAsnGlyLysX11LeuLysGlyGluThrX17ThrGluAlaValX22Ala AlaX25AlaGluLysValPheLysX32TyrAlaX35X36X37GlyValX40GlyX42TrpThrTyrAspX47X4 8ThrLysThrPheThrValThrGlu
      (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; X42 is Glu; and X11 is Thr and X17 is Thr simultaneously.)
   c) mutant protein of B3 domain protein of the wild-type protein G consisting of an amino acid sequence represented by (c) or of the amino acid sequence obtained by deleting, substituting, inserting or adding one or several amino acid residues in the amino acid sequence represented by (c), wherein
      the mutant protein has the binding property to the Fc region of immunoglobulin G, and
      the mutant protein has at least the binding property to the Fab region of immunoglobulin G and/or the binding property to the Fc region in the weakly acidic region is decreased in comparison with B3 domain protein of the wild-type protein G.
(c)ThrThrTyrLysLeuValIleAsnGlyLysX11LeuLysGlyGluThrX17ThrLysAlaValX22Ala GluX25AlaGluLysAlaPheLysX32TyrAlaX35X36X37GlyValX40GlyValTrpThrTyrAspX47X4 8ThrLysThrPheThrValThrGlu
   (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; and X11 is Thr and X17 is Thr simultaneously.)
   In the above-mentioned definitions of the amino acid residues (a) to (c), the provisos are for distinguishing the amino acid residue from each cell membrane domain protein of the wild-type protein G and the following mutant protein with the improved stability of the cell membrane domain of the protein G claimed by the inventors.

Further specific examples of the mutant proteins according to the present invention include the following d) to i).
d) A mutant protein of B1 domain protein of the wild-type protein G consisting of an amino acid sequence represented by (d) or of the amino acid sequence obtained by deleting, substituting, inserting or adding one or several amino acid residues in the amino acid sequence represented by (d), wherein
   the mutant protein has the binding property to the Fc region of immunoglobulin G, and
   the mutant protein has the binding property to the Fab region of immunoglobulin G and/or the binding property to the Fc region in the weakly acidic region is decreased in comparison with B1 domain protein of the wild-type protein G.
   (d)AspThrTyrLysLeuIleLeuAsnGlyLysX11LeuLysGlyGluThrX17ThrGluAlaValX22AlaAla X25AlaGluLysValPheLysX32TyrAlaAsnAspAsnGlyValX40GlyX42TrpThrTyrAspAspAlaThrLys ThrPheThrValThrGlu
      (In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)
e) A mutant protein of B2 domain protein of the wild-type protein G consisting of an amino acid sequence represented by (e) or of the amino acid sequence obtained by deleting, substituting, inserting or adding one or several amino acid residues in the amino acid sequence represented by (e), wherein
   the mutant protein has the binding property to the Fc region of immunoglobulin G, and
   the mutant protein has the binding property to the Fab region of immunoglobulin G and/or the binding property to the Fc region in the weakly acidic region is decreased in comparison with B2 domain protein of the wild-type protein G.
   (e)ThrThrTyrLysLeuValIleAsnGlyLysX11LeuLysGlyGluThrX17ThrGluAlaValX22AlaAla X25AlaGluLysValPheLysX32TyrAlaAsnAspAsnGlyValX40GlyX42TrpThrTyrAspAspAlaThrLys ThrPheThrValThrGlu
      (In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)
f) A mutant protein of B3 domain protein of the wild-type protein G consisting of an amino acid sequence represented by (f) or of the amino acid sequence obtained by deleting, substituting, inserting or adding one or several amino acid residues in the amino acid sequence represented by (f), wherein
   the mutant protein has the binding property to the Fc region of immunoglobulin G, and
   the mutant protein has the binding property to the Fab region of immunoglobulin G and/or the binding property to the Fc region in the weakly acidic region is decreased in comparison with B3 domain protein of the wild-type protein G.
   (f)ThrThrTyrLysLeuValIleAsnGlyLysX11LeuLysGlyGluThrX17ThrLysAlaValX22Ala GluX25AlaGluLysAlaPheLysX32TyrAlaAsnAspAsnGlyValX40GlyValTrpThrTyrAspAspAla ThrLysThrPheThrValThrGlu
      (In the amino acid sequence, X22 represents Asp or His; X25 represents Thr or His; X32 represents Gln or His; X40 represents Asp or His; X11 represents Thr or Arg; and X17 represents Thr or Ile, respectively, with the proviso that a case is excluded where X22 is Asp; X25 is Thr; X32 is Gln; X40 is Asp; and X11 is Thr and X17 is Thr simultaneously.)
g) A mutant protein of B1 domain protein of the wild-type protein G consisting of an amino acid sequence represented by (g) or of the amino acid sequence obtained by deleting, substituting, inserting or adding one or several amino acid residues in the amino acid sequence represented by (g), wherein
   the mutant protein has the binding property to the Fc region of immunoglobulin G, and
   the mutant protein has the binding property to the Fc region in the weakly acidic region is decreased in comparison with B1 domain protein of the wild-type protein G.
   (g)AspThrTyrLysLeuIleLeuAsnGlyLysThrLeuLysGlyGluThrThrThrGluAlaValX22Ala AlaX25AlaGluLysValPheLysX32TyrAlaAsnAspAsnGlyValX40GlyX42TrpThrTyrAspAspAla ThrLysThrPheThrValThrGlu
      (In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)
h) Each of mutant proteins of B2 domain protein of the wild-type protein G consisting of an amino acid sequence represented by (h) or of the amino acid sequence obtained by deleting, substituting, inserting or adding one or several amino acid residues in the amino acid sequence represented by (h), wherein
   the mutant protein has the binding property to the Fc region of immunoglobulin G, and
   the mutant protein has the binding property to the Fc region in the weakly acidic region is decreased in comparison with B2 domain protein of the wild-type protein G.
(h)ThrThrTyrLysLeuValIleAsnGlyLysThrLeuLysGlyGluThrThrThrGluAlaValX22AlaA laX25AlaGluLysValPheLysX32TyrAlaAsnAspAsnGlyValX40GlyX42TrpThrTyrAspAspAlaT hrLysThrPheThrValThrGlu
   (In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)
i) Each of mutant proteins of B3 domain protein of the wild-type protein G consisting of an amino acid sequence represented by (i) or of the amino acid sequence obtained by deleting, substituting, inserting or adding one or several amino acid residues in the amino acid sequence represented by (i), wherein
   the mutant protein has the binding property to the Fc region of immunoglobulin G, and
   the mutant protein has the binding property to the Fc region in the weakly acidic region is decreased in comparison with B3 domain protein of the wild-type protein G.
   (i)ThrThrTyrLysLeuValIleAsnGlyLysThrLeuLysGlyGluThrThrThrLysAlaValX22AlaGl uX25AlaGluLysAlaPheLysX32TyrAlaAsnAspAsnGlyValX40GlyValTrpThrTyrAspAspAlaThr LysThrPheThrValThrGlu
      (In the amino acid sequence, X22 represents Asp or His; X25 represents Thr or His; X32 represents Gln or His; and X40 represents Asp or His, respectively, with the proviso that a case is excluded where X22 is Asp; and X25 is Thr; X32 is Gln and X40 is Asp simultaneously.)

In the above-mentioned definitions of the amino acid residues (d) to (i), the provisos are for distinguishing the amino acid residue from each cell membrane domain protein of the wild-type protein G.

As is clear from above, in the design of the mutant proteins of the present invention, the target part for the mutation which is selected and the amino acid residue which is substituted for the part are not limited to only one of each, so that the amino acid sequence of the mutant protein can be designed by appropriately selecting from the target parts for the mutation and the amino acid residues which are substituted for the parts. For example, to improve the binding property to the Fc region of immunoglobulin G, a plurality of amino acid sequences of the mutant proteins can be designed by the steps of; selecting Asp22, Thr25, Gln32, Asp40 and Glu42 in the amino acid sequence of the B1 or B2 domain of the wild-type protein G as the target parts for the mutation, selecting Asp22His, Thr25His, Gln32His, Asp40His and Glu42His as the corresponding amino acid residues which are substituted, and executing point mutation or multiplex mutation of up to five mutation positions / five substitutions based on any one of amino acid substitutions or a combination of the amino acid substitutions to the wild-type amino acid sequence of the B1 or B2 domain of the protein G (SEQ ID NO. 1, 2). The above-mentioned amino acid sequences in (g) and (h) represent such point mutation and such multiplex mutation of up to five mutation positions / five substitutions, and are an example of the mutant proteins of the present invention.

Also, for example, the mutant protein in which the improvement of the binding property to the Fab region of immunoglobulin G is further applied in addition to the above-mentioned improvement of the binding property to the Fc region of immunoglobulin G includes mutant proteins designed by the steps of; selecting Thr11 and Thr17 in the B 1 or B2 domain of the wild-type protein G, selecting Thr11Arg and Thr17Ile as the corresponding amino acid residues, and executing mutation of up to seven mutation positions / seven substitutions, in which these two options are added and transduced to the above-mentioned mutation of up to five mutation positions / five substitutions, to the wild-type amino acid sequence of the B1 or B2 domain of the protein G. The above-mentioned amino acid sequences in (d) and (e) represent examples of such point mutation or such multiplex mutation of up to seven mutation positions / seven substitutions, and, in the amino acid sequences, amino acid sequence with mutation of Thr11Arg and/or Thr17Ile and with any one or more of the above-mentioned mutation of Asp22His, Thr25His, Gln32His, Asp40His and Glu42His is that in which the improvement of the binding property to the Fab region of immunoglobulin G is further applied in addition to the improvement of the binding property to the Fc region of immunoglobulin G.

On the other hand, although the above-mentioned amino acid sequence in (i) is an example of an amino acid sequence of a B3 domain mutant protein of the wild-type protein G, it is designed similarly as the amino acid sequences in (g) and (h), except for mutation of up to four mutation positions / four substitutions of any one or more of Asp22His, Thr25His, Gln32His and Asp40His as the above-mentioned mutation for improving the binding property to the Fc region. Also, although the above-mentioned amino acid sequence in (f) is an example of the amino acid sequence of the B3 domain mutant protein of the wild-type protein G, it is designed similarly as the amino acid sequences in (d) and (e), except for mutation of up to six mutation positions / six substitutions of any one or more of Asp22His, Thr25His, Gln32His and Asp40His, as the above-mentioned mutation for improving the binding property to the Fc region, and Thr11Arg and Thr17Ile, as the above-mentioned mutation for improving the binding property to the Fab region.

In the present invention, the mutation which has been already known to make the property of the extracellular domain of the protein G more preferable may be further applied in addition to such mutation. For example, a mutation method for improving the thermal stability, the chemical resistance to a denaturing agent and the resistance to a decomposing enzyme of the extracellular domain of the protein G has been found through previous research by the inventors (Patent Document 6). Namely, transduction of mutation of any one or more of Asn35Lys, Asp36Glu, Asn37His, Asn37Leu, Asp47Pro, Ala48Lys and Ala48Glu improves the above-mentioned stability of the B1, B2 or B3 domain of the protein G. By combining this mutation with the above-mentioned mutation for improving a binding characteristic to the Fc region of immunoglobulin G and/or a binding characteristic to the Fab region thereof according to the present invention, the mutant proteins of the present invention become more useful.

For example, more stabilized amino acid sequence of a plurality of mutant proteins can be designed by the step of executing multiplex mutation of up to twelve mutation positions / fourteen substitutions, in which this mutation for the stabilization is added and transduced to the above-mentioned mutation of up to seven mutation positions / seven substitutions, to the wild-type amino acid sequence of the B1 or B2 domain of the protein G (SEQ ID NO. 1, 2).

Although the above-mentioned amino acid sequences in (a) and (b) represent such point mutation and such multiplex mutation of up to twelve mutation positions / fourteen substitutions, the wild-type sequence as well as the mutation only for the stabilization which is applied to the wild-type sequence are excluded.

Transduction of mutation of any one or more of Asn35Lys, Asp36Glu, Asn37His, Asn37Leu, Asp47Pro, Ala48Lys and Ala48Glu in the amino acid sequences in (a) and (b) improves the stability of the B1 or B2 domain mutant protein of the protein G in addition to the above-mentioned improvement of the binding characteristic to the Fab region of immunoglobulin G and/or the binding characteristic to the Fc region thereof in the weakly acidic region as the effect of the mutation of up to seven mutation positions / seven substitutions.

On the other hand, although the above (c) describes an example of the amino acid sequence of the B3 domain mutant protein of the wild-type protein G and point mutation and multiplex mutation of up to eleven mutation positions / thirteen substitutions, it is designed similarly as the amino acid sequences in (a) and (b), except for mutation of up to four mutation positions / four substitutions of any one or more of Asp22His, Thr25His, Gln32His and Asp40His as the above-mentioned mutation for improving the binding property to the Fc region. Therefore, transduction of mutation of any one or more of Asn35Lys, Asp36Glu, Asn37His, Asn37Leu, Asp47Pro, Ala48Lys and Ala48Glu in the amino acid sequence in (c) also improves the stability of the B3 domain mutant protein of the protein G in addition to the improvement of the binding characteristic to the Fab region of immunoglobulin G and/or the binding characteristic to the Fc region thereof in the weakly acidic region.

As described above, the target parts for the mutation in the present invention are selected by using each three-dimensional atomic coordinate data of the B2 domain of the protein G - Fc complex and the B3 domain thereof - Fab complex, but since the B1 domain hardly differs from the B2 domain in not only the amino acid sequences (FIG. 2) but also the stereoscopic structure, the above-mentioned selected mutation is effective equally for each domain. Also, since the B3 domain hardly differs from the B2 domain in not only the amino acid sequences (FIG. 2) but also the stereoscopic structure, the above-mentioned mutation selected in the B2 domain is effective equally for each domain.

For example, all the above-mentioned wild-type amino acid residues in the selected twelve mutation positions are common between the above-mentioned B2 domain and the above-mentioned B1 domain. Therefore, the point mutation and the multiplex mutation based on the combination of the above-mentioned selected five mutation positions / five substitutions, seven mutation positions / seven substitutions or twelve mutation positions / fourteen substitutions can be transduced to the B1 amino acid sequence which is highly equal to the B2 domain to transduce the amino acid sequence of the mutant protein of the B1 domain. Also, all the above-mentioned wild-type amino acids in the selected twelve mutation positions except the 42nd position are common between the above-mentioned B2 domain and the above-mentioned B3 domain (the wild-type amino acid residue in the 42nd position is Glu42 in the B2 domain and is Val42 in the B3 domain). Therefore, the point mutation and the multiplex mutation based on the combination of the four mutation positions / four substitutions, six mutation positions / six substitutions or eleven mutation positions / thirteen substitutions, in which only the mutation position in the 42nd position is excluded from the above-mentioned selected five mutation positions / five substitutions, seven mutation positions / seven substitutions or twelve mutation positions / fourteen substitutions, can be transduced to the amino acid sequence of the B3 domain which is highly equal to the B2 domain to produce the amino acid sequence of the mutant protein of the B3 domain. This is also clear from the fact that, as shown in the following examples, the mutant proteins of the B1 domain based on the selection by using each three-dimensional atomic coordinate data of the B2 domain of the protein G - Fc complex and the B3 domain thereof - Fab complex have performance as intended.

As described above, the amino acid sequence of the mutant proteins of the present invention is not limited to one, and there exist a plurality of amino acid sequences among which preferable sequences specifically include an amino acid sequence represented by [SEQ ID NO. 13], [SEQ ID NO. 14], [SEQ ID NO. 15], [SEQ ID NO. 16], [SEQ ID NO. 17], [SEQ ID NO. 18], [SEQ ID NO. 19] or [SEQ ID NO. 20].

As for the mutant protein represented by [SEQ ID NO. 13], mutation is transduced to the part, with respect to which, through the previous research, the inventors have found that the thermal stability, the chemical resistance to a denaturing agent and the resistance to a decomposing enzyme of the extracellular domain of the protein G can be improved, in the wild-type amino acid sequence of the B1 domain of the protein G represented by [SEQ ID NO.1], and, as for the mutant proteins represented by [SEQ ID NO. 14], [SEQ ID NO. 15], [SEQ ID NO. 19] and [SEQ ID NO. 20], mutation is further transduced to the part which is selected based on the analysis of the surface bound to the Fc.

On the other hand, as for the mutant proteins represented by [SEQ ID NO. 16], [SEQ ID NO. 17] and [SEQ ID NO. 18], mutation is transduced to the part, with respect to which, through the previous research, the inventors have found that the thermal stability, the chemical resistance to a denaturing agent and the resistance to a decomposing enzyme of the extracellular domain of the protein G can be improved, and to the part which is selected based on the analysis of the surface bound to the Fab, in the wild-type amino acid sequence of the B1 domain of the protein G represented by [SEQ ID NO. 1].

Although the mutant proteins of the present invention have the binding property to the protein having the antibody, the immunoglobulin G or the Fc region of immunoglobulin G, mutation such as deletion, substitution, insertion, or addition may be generated in relation to one or several (for example, two to five) amino acid residues of the amino acid sequence described above as any one of the mutant proteins of the present invention as long as at least the binding property to the Fab region of immunoglobulin G and/or the binding property to the Fc region thereof in the weakly acidic region is decreased in comparison with each extracellular domain protein of the wild-type protein G, so that the sequence identity of the mutant proteins to each amino acid sequence as a reference is more than 90 %, preferably more than 95 %, and more preferably more than 98 %.

An example of the proteins of the present invention includes a protein in which the three extracellular domain mutants constituting the tandem-type multimer consist of the amino acid sequences represented by SEQ ID NO. 19, as described in the following examples.

### Production of the protein

### (1) Production of the protein by a genetic engineering technique

### a. Gene encoding the mutant protein

In the present invention, a genetic engineering method can be used to produce the above-mentioned designed protein.

A gene used in such a method consists of a nucleic acid encoding the protein described in the above A to C, more specifically, encoding the amino acid sequence described in any one of the above (a) to (i), or consists of a nucleic acid encoding a protein which has an amino acid sequence obtained by deleting, substituting or adding one or several amino acid residues in the amino acid sequence described in any one of (a) to (i) and which has the binding property to the protein having the antibody, the immunoglobulin G or the Fc region of immunoglobulin G, wherein the binding property is decreased in the weakly acidic region in comparison with in the neutral region; and, more specifically, the nucleic acid consists of a base sequence represented by any one of [SEQ ID NO. 22] to [SEQ ID NO. 29], for example.

Moreover, a gene used in the present invention also includes a nucleic acid hybridizing with a nucleic acid which consists of a sequence complementary to the above-mentioned base sequence of the nucleic acid under a stringent condition, and encoding the above-mentioned mutant protein which has the binding property to the protein having the antibody, the immunoglobulin G or the Fc region of immunoglobulin G, wherein the binding property to the Fab region of immunoglobulin G and/or the binding property to the Fc region thereof in the weakly acidic region is decreased in comparison with each corresponding extracellular domain protein of the wild-type protein G. The stringent condition herein refers to a condition that a specific hybrid is formed and that a non-specific hybrid is not formed. Specifically, it refers to a condition that a nucleic acid with high identity (the identity is more than 60 %, preferably more than 80 %, more preferably more than 90 %, and most preferably more than 90 %) hybridizes. More specifically, it refers to a condition that sodium concentration is 150 mM to 900 mM, and preferably 600 mM to 900 mM and that temperature is 60 °C to 68 °C, and preferably 65 °C. If hybridization by a conventional means, such as Southern blot, dot blot hybridization, is confirmed, for example, under a hybridization condition of 65 °C and a washing condition of 65 °C, for ten minutes, in 0.1*SSC containing 0.1 % SDS, it can be called "hybridizing under the stringent condition".

The gene encoding the protein of the present invention includes a nucleic acid encoding the above-mentioned nucleic acid and the above-mentioned optional linker sequences, depending on the desired structure of the protein of the present invention. A plurality of nucleic acids which encode each mutant protein constituting the tandem-type multimer and a plurality of nucleic acids encoding the linker sequences may be alternately connected, or the nucleic acid may be designed to encode a fusion-type amino acid sequence by connecting the above-mentioned nucleic acid and a nucleic acid encoding an amino acid sequences of any protein.

### b. Gene, recombinant vector and transformant

The above-mentioned gene of the present invention can be synthesized by a chemical synthesis, a PCR, a cassette mutagenesis, a site-specific mutagenesis or the like. For example, a plurality of oligonucleotides up to about 100 bases with a complementary region of about 20 base pair at the terminal are chemically synthesized, and then by combining the oligonucleotides to perform the overlap extension method (Reference Document 8), the desired gene can be totally synthesized.

The recombinant vector of the present invention can be obtained by connecting (inserting) the gene comprising the above-mentioned base sequence to an appropriate vector. The vector used herein is not particularly limited as long as it is replicable in a host or it can incorporate the desired gene into a host genome. For example, the vector includes a bacteriophage, a plasmid, a cosmid, a phagemid and the like.

A plasmid DNA includes a plasmid derived from actinomycetes (such as pK4, pRK401 and pRF31), a palasmid derived from the Escherichia coli (such as pBR322, pBR325, pUC118, pUC119 and pUC18), a plasmid derived from hay bacillus (such as pUB110 and pTP5), a plasmid derived from yeast (such as YEp13, YEp24 and YCp50), and the like; and a phage DNA includes a λ phage (such as λgt10, λgt11 and λZAP). Moreover, an animal virus vector such as a retrovirus or a vaccinia virus and an insect virus vector such as a baculovirus may be used.

For inserting the gene into the vector, a method in which first a purified DNA is cut with an appropriate restriction enzyme and next the gene is inserted into a restriction enzyme site or a multicloning site of an appropriate vector DNA and connected with the vector, or the like is adopted. The gene must be incorporated into the vector so that the mutant protein of the present invention is expressed. Therefore, in addition to a promoter and the base sequence of the gene, a cis element such as an enhancer, a splicing signal, a poly A addition signal, a selection marker, a ribosome-binding sequence (an SD sequence), an initiation codon, a termination codon, and the like may be optionally connected to the vector of the present invention. Also, a tag sequence for facilitating purification of the protein which is produced may be connected, As the tag sequence, a base sequence encoding the known tag such as His tag, GST tag, MBP tag and BioEase tag may be used.

A confirmation as to whether the gene is inserted into the vector can be performed by using the known genetic engineering technology. For example, in the case of the plasmid vector and the like, the confirmation is performed by subcloning the vector with a competent cell to extract DNA and then specifying a base sequence of the DNA with a DNA sequencer. A similar means is available to other vectors as long as they can be subcloned with a bacteria or another host. Also, screening of the vector with the selection marker such as a drug resistant gene is effective.

The transformant can be obtained by transducing the recombinant vector of the present invention to a host cell so that the mutant protein of the present invention can be expressed. The host used for transformation is not particularly limited as long as it can express a protein or a polypeptide. For example, the host includes a bacteria (such as the Escherichia coli and the hay bacillus), a yeast, a plant cell, an animal cell (such as a COS cell and a CHO cell), and an insect cell.

When the bacteria is the host, it is preferable that the recombinant vector is autonomously replicable in the bacteria and, in addition, that the bacteria is constituted by the promoter, the ribosome-binding sequence, the initiation codon, the nucleic acid encoding the mutant protein of the present invention and a transcription termination sequence. For example, the Escherichia coli includes an Escherichia coli BL21 and the like, and the hay bacillus includes a Bacillus subtilis and the like. A method for transducing the recombinant vector to the bacteria is not particularly limited as long as it is a method for transducing DNA to bacteria.

For example, the method includes a heat shock method, a method using a calcium ion, an electroporation method and the like. When the yeast is the host, for example, a Saccharomyces cerevisiae, a Schizosaccharomyces pombe or the like is used. A method for transducing the recombinant vector to the yeast is not particularly limited as long as it is a method for transducing DNA to a yeast, and, for example, the method includes the electroporation method, a spheroplast method, a lithium acetate method and the like.

When the animal cell is the host, a monkey cell COS-7, a Vero cell, a chinese hamster ovarian cell (a CHO cell), a mouse L cell, a rat GH3, a human FL cell or the like is used. For example, a method for transducing the recombinant vector to the animal cell includes the electroporation method, a calcium phosphate method, a lipofection method and the like. When the insect cell is the host, a Sf9 cell or the like is used. For example, a method for transducing the recombinant vector to the insect cell includes the calcium phosphate method, the lipofection method, the electroporation method and the like.

A confirmation as to whether the gene is transduced to the host can be performed by using a PCR method, a southern hybridization method, a northern hybridization method or the like. For example, DNA is prepared from the transformant, and then a DNA-specific primer is designed to perform the PCR. Next, an amplification product of the PCR is subjected to an agarose gel electrophoresis, a polyacrylamide gel electrophoresis, a capillary electrophoresis or the like and is stained with an ethidium bromide, a SyberGreen solution or the like; and then, through detecting the amplification product as one band, it can be confirmed that the transformation has been performed. Alternatively, by using a primer previously labeled with a fluorescent pigment or the like, the PCR may also be performed to detect an amplification product.

### c. Acquisition of the protein by culturing the transformant

When the protein of the present invention is produced as a recombinant protein, it can be obtained by culturing the above-mentioned transformant and then collecting the protein from the cultured product. The cultured product refers to any one of a culture supernatant, a cultured cell, or a cultured cell body; and a disrupted product of a cell or a cell body. A method for culturing the transformant of the present invention is performed according to a conventional method used for culture of a host.

A medium for culturing a transformant obtained by using a microorganism, such as the Escherichia coli or a yeast fungus, as the host may be any one of a natural medium and a synthesis medium as long as it contains a carbon source, a nitrogen source, inorganic salts or the like which can be assimilated by the microorganism, and is a medium which can effectively culture a transformant. The carbon source includes a carbohydrate such as glucose, fructose, sucrose and starch; an organic acid such as acetic acid and propionic acid; and alcohols such as ethanol and propanol. The nitrogen source includes not only ammonia; an ammonium salt of an inorganic acid or an organic acid such as ammonium chloride, ammonium sulfate, ammonium acetate and ammonium phosphate; or other nitrogen-containing compounds; but also peptone, meat extract, corn steep liquor and the like. An inorganic substance includes monopotassium phosphate, dipotassium phosphate, magnesium phosphate, magnesium sulfate, sodium chloride, ferrous sulfate, manganese sulfate, copper sulfate, calcium carbonate and the like. The culture is normally performed under an aerobic condition of shake culture, aeration-agitation culture or the like, at 20 °C to 37 °C, for 12 hours to for 3 days.

After the culture, when the protein of the present invention is produced in the cell body or in the cell, the cell body or the cell is crushed by performing ultrasonic treatment, repetition of freezing and thawing, homogenizer treatment or the like to collect the protein. Alternatively, when the protein is produced out of the cell body or out of the cell, a culture solution is used as it is, or the cell body or the cell is removed by centrifugal separation or the like. Then, by using a general biochemical method for isolation and purification of a protein, such as ammonium sulfate precipitation, gel chromatography, ion exchange chromatography, affinity chromatography, alone or in proper combination, the protein of the present invention can be isolated and purified from the above-mentioned cultured product.

Moreover, by utilizing a so-called cell-free synthesis system in which only factors concerning biosynthesis reaction of a protein (such as an enzyme, the nucleic acid, an ATP, the amino acid) are mixed, the mutant protein of the present invention can be synthesized from the vector without using a living cell, in vitro (Reference Document 9). Then, by using a purification method similar to the above, the mutant protein of the present invention can be isolated and purified from a mixed solution after the reaction.

To confirm whether the protein of the present invention obtained by the isolation and purification is a protein consisting of the desired amino acid sequence, a sample containing the protein is analyzed. As an analysis method, the SDS-PAGE, a western blotting, a mass spectrometry, amino acid analysis, an amino acid sequencer and the like can be used (Reference Document 10).

### (2) Production of the protein by other means

The protein of the present invention may be produced by an organic chemical means such as a solid phase peptide synthesis method. The production method of the protein using such a means is well known in this technical field, and thus is concisely described below.

When the protein is chemically produced by the solid phase peptide synthesis method, protecting polypeptide with the amino acid sequence of the protein of the present invention is synthesized on a resin by repeating polycondensation reaction of an activated amino acid derivative, preferably with an automatic synthesizer. Next, at the same time that the protecting polypeptide is cleaved from the resin, the protecting groups of side-chains are also cleaved. It is known that, for the cleavage reaction, there exists a suitable cocktail depending on kinds of the resin and the protecting groups, and composition of the amino acids (Reference Document 11). Then, a roughly purified protein is transferred from an organic solvent layer to an aqueous layer, and the target protein is purified. As the purification method, reversed-phase chromatography or the like can be used (Reference Document 11).

### Performance confirmation test for the protein and the antibody capturing agent

Although the following performance confirmation tests may be performed for the mutant proteins and the proteins produced as described above (hereinafter, also referred to as "the protein" in brief) and the antibody capturing agents to select excellent products, the proteins and the antibody capturing agents of the present invention had all excellent performance.

### (1) Antibody-binding property test

The antibody-binding property of the proteins of the present invention may be confirmed and evaluated by using the western blotting, an immunoprecipitation, a pull-down assay, an ELISA (Enzyme-Linked ImmunoSorbent Assay), the surface plasmon resonance (SPR) method, and the like. Above all, in the SPR method, since interaction between living bodies can be observed over time in real time without label, a binding reaction of the mutant proteins can be evaluated quantitatively from a kinetic viewpoint. Moreover, the antibody-binding property of the mutant protein immobilized to the water-insoluble solid support can be confirmed and evaluated by the above-mentioned SPR method and a liquid chromatography method. Especially, by the liquid chromatography method, the pH dependence relative to the antibody-binding property can be precisely evaluated.

### (2) Thermal stability test for the protein

The thermal stability of the mutant proteins of the present invention may be evaluated by using a circular dichroism (CD) spectrum, a fluorescence spectrum, an infrared spectroscopy, a differential scanning calorimetry, residual activity after heating, and the like. Above all, since the CD spectrum is a spectroscopic analysis method sensitively reflecting change of secondary structure of a protein, a change of the stereoscopic structure of the mutant protein due to temperature can be observed and structural stability can be evaluated quantitatively from a thermodynamic viewpoint.

### [References]

Reference 1: Bjorck L, Kronvall G. (1984) Purification and some properties of streptococcal protein G, a novel IgG-binding reagent. J Immunol. 133, 69-74.
Reference 2: Boyle M. D.P., Ed. (1990) Bacterial Immunoglobulin Binding Proteins. Academic Press, Inc., San Diego, CA.
Reference 3: Gallagher T, Alexander P, Bryan P, Gilliland GL. (1994) Two crystal structures of the B1 immunoglobulin-binding domain of streptococcal protein G and comparison with NMR. Biochemistry 19, 4721-4729.
Reference 4: Sauer-Eriksson AE, Kleywegt GJ, Uhlen M, Jones TA. (1995) Crystal structure of the C2 fragment of streptococcal protein G in complex with the Fc domain of human IgG. Structure 3, 265-278.
Reference 5: Derrick JP, Wigley DB. (1994) The third IgG-binding domain from streptococcal protein G. An analysis by X-ray crystallography of the structure alone and in a complex with Fab. J Mol Biol. 243, 906-918.
Reference 6: Alexander P, Fahnestock S, Lee T, Orban J, Bryan P. (1992) Thermodynamic analysis of the folding of the streptococcal protein G IgG-binding domains B1 and B2: why small proteins tend to have high denaturation temperatures. Biochemistry 14, 3597-3603. Reference 7: D'souza VM, Holz RC. (1999) The methionyl aminopeptidase from Escherichia coli can function as an iron (II) enzyme. Biochemistry 38, 11079-11085.
Reference 8: Horton R. M., Hunt H. D., Ho S. N., Pullen J. M. and Pease L. R. (1989). Engineering hybrid genes without the use of restriction enzymes: gene splicing by overlap extension. Gene 77, 61-68.
Reference 9: Masato OKADA, Kaoru MIYAZAKI (2004) Notes for Protein Experiment (first volume). Yodosha Co., Ltd.
Reference 10: Shigeo OHNO, Yoshifumi NISHIMURA (ed.) (1997) Protocol for Protein Experiment 1 - Functional Analysis Part. Shujunsha Co., Ltd.
Reference 11: Shigeo OHNO, Yoshifumi NISHIMURA (ed.) (1997) Protocol for Protein Experiment 2 - Structural Analysis Part. Shunjusha Co., Ltd.

Hereinafter, the present invention will be specifically described with the following examples. However, the technical scope of the present invention is not limited to the following examples. In this specification, various amino acid residues are denoted by the following abbreviations. Ala; an L-alanine residue, Arg; an L-arginine residue, Asp; an L-aspartic acid residue, Asn; an L-asparagine residue, Cys; an L-cysteine residue, Gln; an L-glutamine residue, Glu; an L-glutamic acid residue, Gly; an L-glycine residue, His; an L-histidine residue, Ile; an L-isoleucine residue, Leu; an L-leucine residue, Lys; an L-lysine residue, Met; an L-methionine residue, Phe; an L-phenylalanine residue, Pro; an L-proline residue, Ser; an L-serine residue, Thr; an L-threonine residue, Trp; an L-tryptophan residue, Tyr; an L-tyrosine residue, and Val; an L-valine residue. Moreover, in this specification, an amino acid sequence of a peptide is described according to a conventional method, in which an amino terminal (hereinafter, referred to as an n-terminal) of the sequence is positioned at the left side while a carboxyl terminal (hereinafter, referred to as a c-terminal) thereof is positioned at the right side.

### Examples

### Example 1

In this example, the selection of the part to which the mutation for designing the amino acid sequence is transduced and the specification of the amino acid residue which is substituted are performed in association with the mutant protein (hereinafter, referred to as "an improved protein G") which is obtained by transducing the mutation to the B1, B2 or B3 domain of the protein G and which is the core of the present invention.

### 1. Selection of the target part for the mutation and specification of the amino acid residue which is substituted, based on an analysis of a surface bound to the Fc

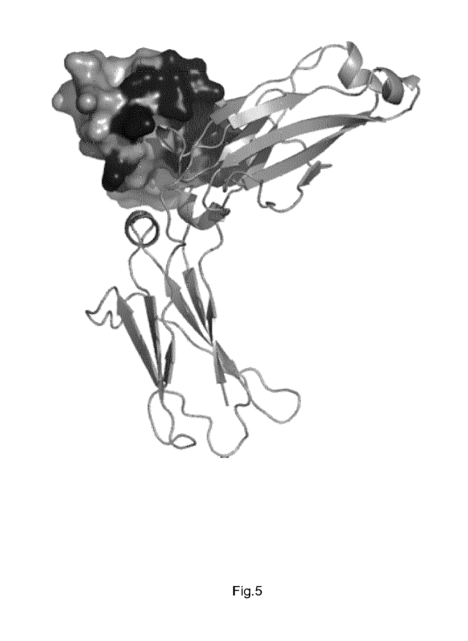

First, a three-dimensional coordinate data of the complex of the B2 domain of the protein G and the Fc region of human immunoglobulin G₁ was downloaded from Protein Data Bank (PDB; http://www.rcsb.org/pdb/home/home.do) which is an international protein stereoscopic structure data base (PDB code: 1FCC). Next, amino acid residues of the B2 domain of the protein G within a distance range of 6.5 angstroms from the Fc region and with an exposed surface area ratio of 40 % and over in the case of the B2 domain of the protein G alone were selected as the target parts for the mutation by calculating with the three-dimensional coordinate data. The number of the selected amino acid residues as the parts is thirteen: Asp22, Ala24, Thr25, Lys28, Val29, Lys31, Gln32, Asn35, Asp36, Gly38, Asp40, Glu42 and Thr44 in the wild-type amino acid sequence of the B2 domain of the protein G represented by [SEQ ID NO. 2]. FIG. 5 shows a position of the target parts for the mutation. The target parts for the mutation exist in the B1 domain commonly. Therefore, not only in the B2 domain but also in the B1 domain, some of the thirteen amino acid residues can be selected as the target parts for the mutation. Namely, the thirteen amino acid residues of Asp22, Ala24, Thr25, Lys28, Val29, Lys31, Gln32, Asn35, Asp36, Gly38, Asp40, Glu42 and Thr44 in the wild-type amino acid sequence of the B1 domain of the protein G represented by [SEQ ID NO. 1] were selected as the target parts for the mutation. Also, some of the target parts for the mutation exist in the B3 domain commonly. Therefore, not only in the B2 domain but also in the B3 domain, some of the thirteen amino acid residues can be selected as the target parts for the mutation. Namely, the ten amino acid residues of Asp22, Thr25, Lys28, Lys31, G1n32, Asn35, Asp36, Gly38, Asp40 and Thr44 in the wild-type amino acid sequence of the B3 domain of the protein G represented by [SEQ ID NO. 3] were selected as the target parts for the mutation.

On the other hand, as for the amino acid residue which would be substituted for the original amino acid residue as the selected target part for the mutation, (i) the amino acid with a charged side-chain (Asp, Glu, Lys, Arg, His) in the case that the original amino acid residue was the amino acid with an uncharged side-chain (Gly, Ala, Val, Leu, Ile, Ser, Thr, Asn, Gln, Phe, Tyr, Trp, Met, Cys, Pro); and (ii) the charged amino acid with opposite electric charge in the case that the original amino acid residue was a charged amino acid; were specified. Alternatively, (iii) when the original amino acid residue was other than the histidine, the histidine was specified. Namely, Lys, Arg or His for Asp22; Asp, Glu, Lys, Arg or His for Ala24; Asp, Glu, Lys, Arg or His for Thr25; Asp, Glu or His for Lys28; Asp, Glu, Lys, Arg or His for Val29; Asp, Glu or His for Lys31; Asp, Glu, Lys, Arg or His for Gln32; Asp, Glu, Lys, Arg or His for Asn35; Lys, Arg or His for Asp36; Asp, Glu, Lys, Arg or His for Gly38; Lys, Arg or His for Asp40; Lys, Arg or His for Glu42; and Asp, Glu, Lys, Arg or His for Thr44 were specified as the amino acid residue which is substituted.

The calculation in this example was performed with ccp4i 4.0 (Daresbury Laboratory, UK Science and Technology Facilities Council), Surface Racer 3.0 for Linux (Dr. Oleg Tsodikov, The University of Michigan), and Red Hat Enterprise Linux WS release 3 (Red Hat) (as software); and Dell Precision Workstation370 (Dell) (as hardware).

### 2. Selection of the target part for the mutation and specification of the amino acid residue which is substituted, based on an analysis of a surface bound to the Fab

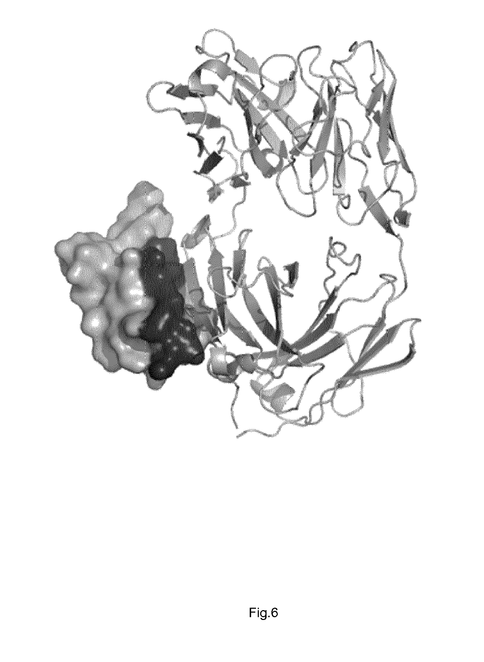

First, a three-dimensional coordinate data of the complex of the B3 domain of the protein G and the Fab region of mouse immunoglobulin G₁ was downloaded from Protein Data Bank (PDB; http://www.rcsb.org/pdb/home/home.do) which is an international protein stereoscopic structure data base (PDB code: 1IGC). Next, amino acid residues of the B3 domain of the protein G within a distance range of 4.0 angstroms from the Fab region and with an exposed surface area ratio of 40 % and over in the case of the B3 domain of the protein G alone were selected as the target parts for the mutation by calculating with the three-dimensional coordinate data. The number of the selected amino acid residues as the parts is ten: Lys10, Thr11, Lys13, Gly14, Glu15, Thr16, Thr17, Asn35, Asp36 and Gly38 in the wild-type amino acid sequence of the B3 domain of the protein G represented by [SEQ ID NO. 3]. FIG. 6 shows a position of the target parts for the mutation. The target parts for the mutation exist in each of the B1, B2 and B3 domains commonly. Therefore, not only in the B3 domain but also in the B1 domain and the B2 domain, the ten amino acid residues can be selected as the target parts for the mutation. Namely, the ten amino acid residues of Lys10, Thr11, Lys13, Gly14, Glu15, Thr16, Thr17, Asn35, Asp36 and Gly38 in the wild-type amino acid sequences of the B1 domain and the B2 domain of the protein G represented by [SEQ ID NO. 1] and [SEQ ID NO. 2] were selected as the target parts for the mutation.

On the other hand, as for the amino acid residue which would be substituted for the original amino acid residue as the selected target part for the mutation, (iv) other kinds of amino acid residue other than the original amino acid and the cysteine was specified. Namely, an amino acid residue other than Lys and Cys for Lys10; an amino acid residue other than Thr and Cys for Thr11; an amino acid residue other than Lys and Cys for Lys13; an amino acid residue other than Gly and Cys for Gly14; an amino acid residue other than Glu and Cys for Glu15; an amino acid residue other than Thr and Cys for Thr16; an amino acid residue other than Thr and Cys for Thr17; an amino acid residue other than Asn and Cys for Asn35; an amino acid residue other than Asp and Cys for Asp36; and an amino acid residue other than Gly and Cys for Gly38; were specified as the amino acid residues which would be substituted.

The calculation in this example was performed with ccp4i 4.0 (Daresbury Laboratory, UK Science and Technology Facilities Council), Surface Racer 3.0 for Linux (Dr. Oleg Tsodikov, The University of Michigan), and Red Hat Enterprise Linux WS release 3 (Red Hat) (as software); and Dell Precision Workstation370 (Dell) (as hardware).

### 3. Selection of the target part for the mutation and specification of the amino acid residue which is substituted, based on the analyses of the surfaces bound to the Fc and the Fab

The selected target parts for the mutation and the specified amino acid residues which would be substituted based on the above-mentioned analysis of the surface bound to the Fc and the above-mentioned analysis of the surface bound to the Fab were combined.

Namely, twenty amino acid residues of Asp22, Ala24, Thr25, Lys28, Val29, Lys31, Gln32, Asp40, Glu42, Thr44, Lys10, Thr11, Lys13, Gly14, Glu15, Thr16, Thr17, Asn35, Asp36 and Gly38 in the wild-type amino acid sequence of the B1 domain of the protein G (SEQ ID NO. 1) are selected as the target parts for the mutation. Lys, Arg or His for Asp22; Asp, Glu, Lys, Arg or His for Ala24; Asp, Glu, Lys, Arg or His for Thr25; Asp, Glu, or His for Lys28; Asp, Glu, Lys, Arg or His for Val29; Asp, Glu or His for Lys31; Asp, Glu, Lys, Arg or His for Gln32; Lys, Arg or His for Asp40; Lys, Arg or His for Glu42; Asp, Glu, Lys, Arg or His for Thr44; amino acid residues other than Lys and Cys for Lys10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38 are specified as the amino acid residues which would be substituted for the original amino acid residue.

In addition, twenty amino acid residues of Asp22, Ala24, Thr25, Lys28, Val29, Lys31, Gln32, Asp40, Glu42, Thr44, Lys10, Thr11, Lys13, Gly14, Glu15, Thr16, Thr17, Asn35, Asp36 and Gly38 in the wild-type amino acid sequence of the B2 domain of the protein G (SEQ ID NO. 2) are selected as the target parts for the mutation. Lys, Arg or His for Asp22; Asp, Glu, Lys, Arg or His for Ala24; Asp, Glu, Lys, Arg or His for Thr25; Asp, Glu, or His for Lys28; Asp, Glu, Lys, Arg or His for Val29; Asp, Glu or His for Lys31; Asp, Glu, Lys, Arg or His for Gln32; Lys, Arg or His for Asp40; Lys, Arg or His for Glu42; Asp, Glu, Lys, Arg or His for Thr44; amino acid residues other than Lys and Cys for Lys10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38 are specified as the amino acid residues which would be substituted for the original amino acid residue.

Furthermore, seventeen amino acid residues of Asp22, Thr25, Lys28, Lys31, Gln32, Asp40, Thr44, Lys10, Thr11, Lys13, Gly14, Glu15, Thr16, Thr17, Asn35, Asp36 and Gly38 in the wild-type amino acid sequence of the B3 domain of the protein G (SEQ ID NO. 3) are selected as the target parts for the mutation.

Lys, Arg or His for Asp22Asp, Glu, Lys, Arg or His for Thr25; Asp, Glu, or His for Lys28; Asp, Glu or His for Lys31; Asp, Glu, Lys, Arg or His for Gln32; Lys, Arg or His for Asp40; Asp, Glu, Lys, Arg or His for Thr44; amino acid residues other than Lys and Cys for Lys10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38 are specified as the amino acid residues which would be substituted for the original amino acid residue.

### Example 2

In this example, amino acid sequences of the improved protein G were designed by utilizing information on the above-mentioned selected target parts for the mutation and the above-mentioned specified amino acid residues which would be substituted.

As is clear from above, the target part for the mutation and the amino acid residue which is substituted for the part are not limited to only one of each, so that the amino acid sequence of the mutant protein can be designed by appropriately selecting from the target parts for the mutation and the amino acid residues which are substituted for the parts. The selection may be performed at random, or may be performed by considering other known information such as structure activity relationship. Moreover, the mutation which has been already known to make the property of the extracellular domain of the protein G more preferable may be combined. In this example, a plurality of amino acid sequences of the improved protein G represented by [SEQ ID NO. 10] were designed by the steps of; selecting Asp22, Thr25, Gln32, Asp40 and Glu42 from the parts selected in "1. Selection of the target part for the mutation and specification of the amino acid residue which is substituted, based on an analysis of a surface bound to the Fc" of Example 1, selecting Asp22His, Thr25His, Gln32His, Asp40His and Glu42His as the corresponding amino acid residues which would be substituted, and executing the point mutation or the multiplex mutation based on the combination of the five mutation positions / five substitutions to the wild-type amino acid sequence of the B1 domain of the protein G represented by [SEQ ID NO. 1].

In addition, a plurality of amino acid sequences of the improved protein G represented by [SEQ ID NO. 7] were designed by the steps of; selecting Thr11 and Thr17 from the parts selected in "2. Selection of the target part for the mutation and specification of the amino acid residue which is substituted, based on an analysis of a surface bound to the Fab" of Example 1, selecting Thr11Arg and Thr17Ile as the corresponding amino acid residues which would be substituted, and executing the point mutation or the multiplex mutation based on the combination of the seven mutation positions / seven substitutions, obtained by adding these two options to the above-mentioned five mutation positions / five substitutions, to the wild-type amino acid sequence of the B1 domain of the protein G represented by [SEQ ID NO. 1].

Moreover, a plurality of amino acid sequences of the improved protein G represented by [SEQ ID NO. 4] were designed by the steps of; selecting Asn35Lys, Asp36Glu, Asn37His, Asn37Leu, Asp47Pro, Ala48Lys and Ala48Glu, with respect to which, through the previous research, the inventors had found that the thermal stability, the chemical resistance to a denaturing agent and the resistance to a decomposing enzyme of the extracellular domain of the protein G could be improved, and executing the point mutation or the multiplex mutation based on the combination of the twelve mutation positions / fourteen substitutions, obtained by adding this mutation to the above-mentioned seven mutation positions / seven substitutions, to the wild-type amino acid sequence of the B1 domain of the protein G represented by [SEQ ID NO. 1].

In this example, amino acid sequences represented by [SEQ ID NO. 13] to [SEQ ID NO. 20] were finally selected as concrete amino acid sequences corresponding to the above-mentioned twelve mutation positions / fourteen substitutions in this example, and then improved proteins G with this sequences were actually synthesized to evaluate the molecular properties.

### Example 3

In this example, the base sequences of the nucleic acids encoding the amino acid sequences of the improved proteins G were designed.

The base sequences of the genes encoding the improved proteins G were designed with Gene Designer (DNA2.0 Inc.) based on the designed amino acid sequences of the improved proteins G to optimize expression efficiency in the Escherichia coli. Because the mutant proteins would be produced in the following two kinds from a practical viewpoint of protein synthesis, the base sequences of the genes were finely adjusted for each kind in consideration for a base sequence of the vector. The OXADac-PG protein is produced as a fusion protein having the sequence of the Oxaloacetate decarboxylase alpha-subunit c-terminal domain (OXADac) in an n-terminal side, and the sequence of the improved protein G in a c-terminal side. Namely, it is synthesized to have an amino acid sequence in which [SEQ ID NO. 31] and any one of [SEQ ID NO. 13] to [SEQ ID NO. 20] are connected. An M-PG protein is produced as a simple protein with no tag and no fusion by using the Escherichia coli. Therefore, an initiation codon sequence is added to the designed amino acid sequence. Namely, the M-PG protein is synthesized to have an amino acid sequence in which Met is added to the n-terminal of any one of [SEQ ID NO. 13] to [SEQ ID NO. 20].

### Example 4

In this example, the plasmid vectors which contain the genes encoding the improved proteins G were synthesized, and then fusion proteins of the Oxaloacetate decarboxylase alpha-subunit c-terminal domains (OXADac) and the mutant proteins shown in Table 1 (OXADac-PG01, OXADac-PG07, OXADac-PG13, OXADac-PG14, OXADac-PG15, OXADac-PG16, OXADac-PG17, OXADac-PG19 and OXADac-PG20) were produced with the Escherichia colis.

### (1) Synthesis of the plasmid for OXADac-PG expression

Homologous recombination between entry plasmids pDONR221-PG (DNA2.0) incorporated with PG genes consisting of base sequences represented by [SEQ ID NO. 21 to [SEQ ID NO. 29] (pg01, pg07, pg13, pg14, pg15, pg16, pg17, pg19 or pg20) and plasmids for expression Champion pET104.1-DEST (Invitrogen) was performed with Gateway LR Clonase Enzyme Mix (Invitrogen). Escherichia coli strains for preservation DH5a (Toyobo, Competent high) were transformed with a reaction liquid. The obtained transformants were selected by a colony PCR and a DNA sequencing (GE Healthcare Bioscience, BigDye Terminator v1.1), and the plasmids for OXADac-PG expression were extracted with a QIAprep Spin Miniprep Kit (Qiagen).

### (2) Expression and immobilization of the OXADac-PG fusion protein

Escherichia colis for expression BL21(DE3) (Novagen) were transformed with the plasmids for OXADac-PG expression. The precultured transformants were subcultured into the LB mediums in 2.5 ml / 500 ml, and were shake cultured to O.D.₆₀₀ = 0.8 to 1.0. After IPTG (0.5 mM) was added in order to express the OXADac-PG fusion proteins, the transformants were further shake cultured at 37 °C for two hours. The collected cell bodies were suspended in 10 ml of PBS and then were ultrasonically crushed before the filter sterilization, and the obtained solutions were treated as wholly protein solutions. Parts of the wholly protein solutions were purified with an IgG Sepharose 6 Fast Flow (GE Healthcare Bioscience) microspin to confirm the expression and the purification by the SDS-PAGE. The rests were injected in a liquid chromatography apparatus AKTApurifier (GE Healthcare Bioscience) to which HiTrap streptavidin HP columns (GE Healthcare Bioscience) had been set, and by operating the apparatus under a condition of 0.3 ml / min (running buffer: 20 mM Na phosphate (pH 6.7), 150 mM NaCl), the OXADac-PG fusion proteins were immobilized on the columns. Since the OXADac has one biotinylated lysine in the molecule, it couples to streptavidin in the column selectively and irreversibly. To maximize the immobilization amounts, a large excess of the OXADac-PG fusion proteins (more than 10 times) to a binding permissible amount of the HiTrap streptavidin HP columns was injected.

**Table 1. Modified Protein G produced**

| Name | Amino Acid Sequence | Location of mutation | Note |
|---|---|---|---|
| OXADac -PG01 | Linked amino acid sequence of [SEQ ID NO:31] and [SEQ ID NO:1] | Wild-type sequence | a |
| OXADac -PG07 | Linked amino acid sequence of[SEQ ID NO:31] and [SEQ ID NO:13] | Four mutations of Asp36Glu/Asn37His/Asp47Pro/Ala48Glu | a |
| OXADac -PG13 | Linked amino acid sequence of [SEQ ID NO:31] and [SEQ ID NO:14] | Five mutations of Asp36Glu/Asn37His/Asp47Pro/Ala48Glu/Asp40His | a |
| OXADac -PG14 | Linked amino acid sequence of [SEQ ID NO:31] and [SEQ ID NO:15] | Five mutations of Asp36Glu/Asn37His/Asp47Pro/Ala48Glu/Glu42His | a |
| OXADac -PG15 | Linked amino acid sequence of [SEQ ID NO:31] and [SEQ ID NO:16] | Five mutations of Asp36Glu/Asn37His/Asp47Pro/Ala48Glu/Thr11Arg | a |
| OXADac -PG16 | Linked amino acid sequence of [SEQ ID NO:31] and [SEQ ID NO:17] | Five mutations of Asp36Glu/Asn37His/Asp47Pro/Ala48Glu/The17Ile | a |
| OXADac -PG17 | Linked amino acid sequence of [SEQ ID NO:31 ]and [SEQ ID NO:18] | Six mutations of Asp36Glu/Asn37His/Asp47Pro/Ala48Glu/Thr11Arg/The 17 Ile | a |
| OXADac -PG19 | Linked amino acid sequence of [SEQ ID NO:31] and [SEQ ID NO:19] | Seven mutations of Asp36Glu/Asn37His/Asp47Pro/Ala48Glu/Asp40His/ Glu42His/Gln32His | a |
| OXADac -PG20 | Linked amino acid sequence of [SEQ ID NO:31] and [SEQ ID NO:20] | Eight mutations of Asp36Glu/Asn37His/Asp47Pro/Ala48Glu/Asp40His/ Glu42His/Gln32His/Asp22His | a |
| M-PG01 | Amino acid sequence of [SEQ ID NO:1] having Met added to its N end | Wild-type sequence | b |
| M-PG07 | Amino acid sequence of [SEQ ID NO:13] having Met added to its N end | Four mutations of Asp36Glu/Asn37His/Asp47Pro/Ala48Glu | b |
| M-PG19 | Amino acid sequence of [SEQ ID NO:19] having Met added to its N end | Seven mutations of Asp36Glu/Asn37His/Asp47Pro/Ala48Glu/Asp40His/ Glu42His/Gln32His | b |
| M-PG20 | Amino acid sequence of [SEQ ID NO:20] having Met added to its N end | Eight mutations of Asp36Glu/Asn37His/Asp47Pro/Ala48Glu/Asp40His/ Glu42His/Gln32His/Asp22His | b |

| | | | |
|---|---|---|---|
| a: Isolated and purified after having been synthesized as Oxaloacetate decarboxylase alpha-subunit c-terminal domain (OXADac) fused protein b: Isolated and purified after having been synthesized as a simple protein without a tag | | | |

### Example 5

In this example, the plasmid vectors which contain the genes encoding the improved proteins G were synthesized, and then the Met addition improved proteins G shown in Table 1 (M-PG01, M-PG07, M-PG19 and M-PG20) were produced with the Escherichia colis.

### (1) Synthesis of the plasmid for M-PG expression

Using the plasmids for OXADac-PG expression prepared in Example 4 as templates, primers comprising restriction enzyme-recognizing sequences were added and the PCR was performed (anneal 45 °C, for 15 seconds to 55 °C, for 5 seconds) to amplify PG gene regions. As the primers, for the M-PG01 and the M-PG07, a sense primer (ATAGCTCCATG GACACTTACAAATTAATCC (SEQ ID NO. 32)) and an antisense primer (ATTGGATCC TTATTCAGTAACTGTAAAGGT (SEQ ID NO. 33)) were used, and for the M-PG19 and the M-PG20, a sense primer (ATAGCTCCATG GATACCTACAAACTGATCC (SEQ ID NO. 34)) and an antisense primer (ATTGGATCC TTATTCGGTAACGGTGAAGGT (SEQ ID NO. 35)) were used. The amplified products obtained by the PCR were confirmed by the agarose electrophoresis (3 %, 100 V), and then were purified with a QIAquick PCR Purification kit (Qiagen). Subsequently, plasmids pET16b (Novagen) digested with restriction enzymes: Nco I and BamH I (NIPPON GENE, 37 °C, for one day) and dephosphorylated (Takara Shuzo, CIAP, 50 °C, for 30 minutes), and the PG genes (pg01, pg07, pg19 or pg20) digested with the same restriction enzymes were ligated (Toyobo, Ligation High, 16 °C, for one hour), and then the Escherichia coli strains for preservation DH5a (Toyobo, Competent high) were transformed with the obtained plasmid vectors, and were selected with LB plating mediums containing 100 µg/mL of ampicillin. The transformants having correct inserted sequences were selected by the colony PCR and the DNA sequencing (AB, BigDye Terminator v1.1), and the plasmids for M-PG expression were extracted with the Qiaprep Spin Miniprep kit (Qiagen). Furthermore, Escherichia coli strains for expression BL21(DE3) (Novagen) were transformed with these plasmids.

### (2) Expression and purification of the recombinant protein

The transformants of the Escherichia colis BL21(DE3) precultured with the LB mediums were subcultured into the LB mediums in 2.5 ml / 500 ml, and were shake cultured to O.D.₆₀₀ = 0.8 to 1.0. After the IPTG was added at a final concentration of 0.5 mM, the transformants were further shake cultured at 37 °C for two hours. The collected cell bodies were suspended in 10 ml of the PBS and then were ultrasonically crushed. After the crushed liquid was filter sterilized, the filtrate was injected in the liquid chromatography apparatus AKTApurifier (GE Healthcare Bioscience) to which IgG Sepharose 6 Fast Flow columns (GE Healthcare Bioscience) had been set to perform an affinity chromatography method (running buffer: 50 mM Tris-HCl(pH 7.6), 150 mM NaCl, 0.05 % Tween20; elution buffer: 0.5 M acetic acid) and/or was injected in the liquid chromatography apparatus AKTApurifier (GE Healthcare Bioscience) to which RESOURCE S columns (GE Healthcare Bioscience) had been set to perform an ion exchange chromatography method (running buffer: 20 mM citric acid, pH 3.5, elution buffer: 20 mM citric acid, 1M NaCl, pH 3.5), so that the M-PG recombinant proteins were purified. After the divided fractions were neutralized with NaOH, they were concentrated with a centrifugal concentrator (RABCONCO, CentriVap concentrator) and were dialyzed with 50 mM of phosphate buffer solutions (pH 6.8). Each solution was freeze-dried to preserve the powdered recombinant proteins (M-PG01, M-PG07, M-PG19 and M-PG20) at -20 °C.

### Example 6

In this example, purity of the improved proteins G was confirmed by the polyacrylamide gel electrophoresis method.

The improved proteins G before and after the purification were prepared to aqueous solutions in concentration of about 75 µM, respectively, and then, by performing Tricine-SDS-PAGE (16 % T-head, 2.6% C, 100 V, 100 min) to detect bands by CBB (G-250) staining, the purity was confirmed. As a result, the improved protein G was detected as a major band in all measured samples, so that it was confirmed that the synthesis yields of the improved proteins G (OXADac-PG19, OXADac-PG20, M-PG01 and M-PG07) were high (>10 mg / L-medium) and that the degrees of purification were also sufficient.

### Example 7

In this example, by measuring molecular weight of the improved proteins G with a MALDI-TOF type mass spectrometer, the produced proteins were identified.

First, the mutant proteins obtained by the isolation and purification were prepared to aqueous solutions in concentration of 15 µM to 25 µM. Next, on sample plates for mass spectrometry, 1µl of matrix solution (aqueous solution containing 50 % (v/v) of acetonitril and 0.1 % of TFA, saturated with α-cyano-4-hydroxycinnamic acid) was dropped and 1µl of each sample solution was further dropped, and then the solutions were mixed and dried on the sample plates. Subsequently, a laser of intensity 2500 to 3000 was irradiated with a mass spectrometry apparatus Voyager (Applied Biosystems) to obtain mass spectrums. As a result of comparing molecular weight of a peak detected from the mass spectrum and theoretical molecular weight calculated from the amino acid sequence of the produced mutant protein, both match within a measurement error, so that it was confirmed that the target protein (OXADac-PG19) had been produced.

### Example 8

In this example, by using the columns on which the OXADac-PG fusion proteins were immobilized, a pH gradient affinity chromatography was performed to determine pH for eluting a monoclonal antibody, so that antibody dissociation of the improved proteins G in the weakly acidic region was evaluated.

After the OXADac-PG fusion protein immobilized columns were set to the liquid chromatography apparatus AKTApurifier (GE Healthcare Bioscience) and were equilibrated by supplying TST buffer (50 mM Tris-HCl(pH 7.6), 150 mM NaCl, 0.05 % Tween20) under a condition of 1 ml /min, IgG1 type humanized monoclonal antibodies prepared to 100µg/200µl were injected. Then, the TST buffer was replaced with 50 mM of Na3 citrate (pH 7.0), and further continuously replaced with 0.5 M of acetate(pH 2.5) for 10 min at a flow rate of 0.5 ml/min to realize the pH gradient (pH 7.0 to 2.5 / 10min). The pH at peaks where the monoclonal antibodies were eluted were recorded from outputs of a UV meter (280 nm) and a pH meter attached to the liquid chromatography apparatus.

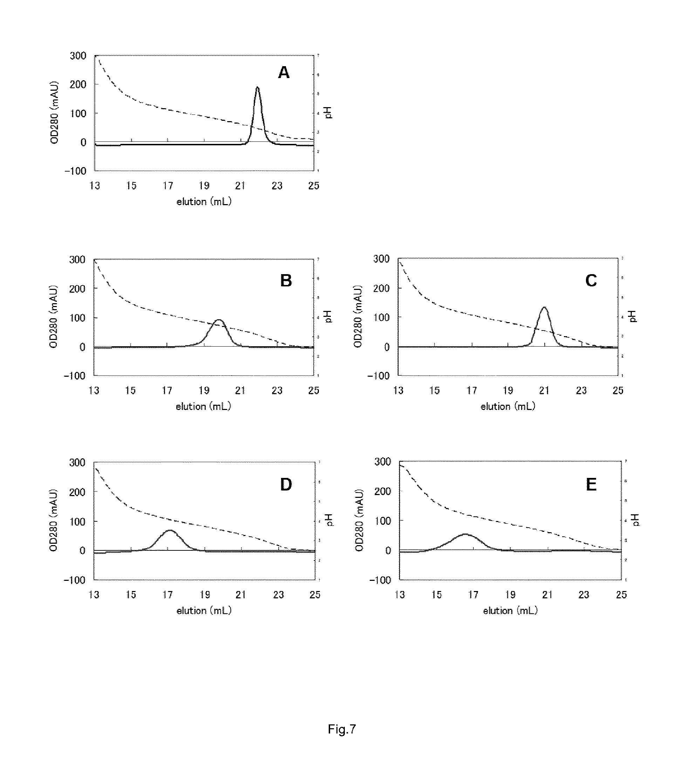

As a result, it was clarified that, in all the measured columns on which the improved proteins G (OXADac-PG13, OXADac-PG17, OXADac-PG19 and OXADac-PG20) were immobilized, the humanized monoclonal antibodies were eluted at higher pH levels in comparison with the column on which a control protein (OXADac-PG01) with the wild-type amino acid sequence was immobilized (FIG. 7). For example, of these, the best improved protein G (OXADac-PG20) elutes the antibody by 1.1 pH points higher than that of the wild-type. (Table 2)

Table 2

**Table 2. Characteristics of Produced Modified Protein G, M-PG07, M-PG19, M-PG20**

| Sample No. | Value of the elution peak of antibody in pH-gradient chromatography | Recovery ratio of antibody in stepwise pH chromatography | Ratio of the remaining amount of antibody by SPR sensor gram | Dissociation rate constant of antibody by SPR sensor gram |
|---|---|---|---|---|
| | pH | at pH4.0 (%) | at pH4.0 (%) | *k*_{off} at pH4.0 (1/s) |
| OXADac-PG01 | 3.18 | 10 | 99 | 4.2 × 10⁻⁵ |
| OXADac-PG07 | n.d. | n.d. | 93 | 1,1 × 10⁻⁴ |
| OXADac-PG13 | 3.56 | n.d. | 64 | 6.0 × 10⁻² |
| OXADac-PG14 | n.d. | n.d. | 71 | 4.0 × 10⁻² |
| OXADac-PG15 | n.d. | n.d. | 90 | 4.4 × 10⁻⁴ |
| OXADac-PG16 | n.d. | n.d. | 94 | 1.3 × 10⁻⁴ |
| OXADac-PG17 | 3.27 | n.d. | 86 | 3.2 × 10⁻² |
| OXADac-PG19 | 4.04 | 88 | 38 | 2.7 × 10⁻¹ |
| OXADac-PG20 | 4.29 | 71 | 58 | n.d. |

| | | | | |
|---|---|---|---|---|
| n.d.: not determined | | | | |

### Example 9

In this example, by using the columns on which the OXADac-PG fusion proteins were immobilized, a stepwise pH affinity chromatography was performed to examine the elution of the monoclonal antibodies at some pH, so that the antibody dissociation of the improved proteins G in the weakly acidic region was evaluated.

After the OXADac-PG fusion protein immobilized columns were set to a liquid chromatography apparatus AKTA prime plus (GE Healthcare Bioscience) and were equilibrated by supplying phosphate buffer (50 µm Na₂HPO₄/NaH₂PO₄ (pH 7.0)) under a condition of 0.4 ml/min, 100 µL of 1 mg/ml samples (ChromPure Human IgG, Fc Fragment) were added. Washing with 12 ml of the phosphate buffer and the elution with 10 ml of elution buffer (100 mM CH₃COOH/CH₃COONa, pH 4) were performed. Then, the columns were washed with pH 2.5, 500 mM of CH₃COOH and finally were equilibrated with 12 ml of the phosphate buffer again. Patterns of human polyclonal Fc region elution with stepwise pH were obtained from outputs of a UV meter (280 nm) attached to the liquid chromatography apparatus.

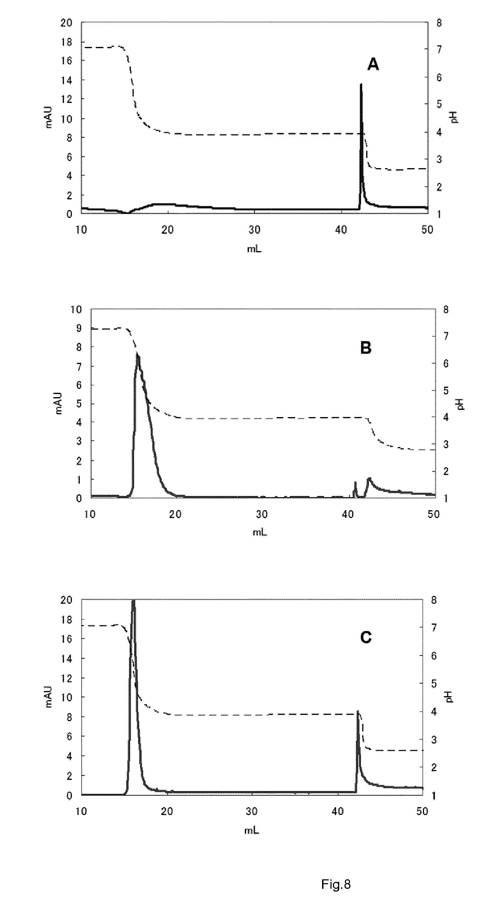

As a result, it was clarified that, in the measured columns on which the improved proteins G (OXADac-PG19 and OXADac-PG20) were immobilized, the human polyclonal antibody Fc regions were eluted at higher pH levels in comparison with the column on which the control protein (OXADac-PG1) with the wild-type amino acid sequence was immobilized (FIG. 8). Elution rates of PG1, PG19 and PG20 in pH 4 region were 10 % , 88 % and 71 %, respectively, so that it was confirmed that the elution rates of the improved proteins G (OXADac-PG19 and OXADac-PG20) were over 7 times higher than that of the wild-type (Table 2).

### Example 10

In this example, binding dissociation of the mutant proteins (protein G mutants) was evaluated by the surface plasmon resonance (SPR) method. It has been recognized that the SPR method is a superior method in which a specific interaction between biopolymers can be measured over time and in which the reaction can be interpreted quantitatively from the kinetic viewpoint.

First, on measuring cells of sensor chips SA (Biacore), the OXADac-PG fusion proteins were immobilized by aid of biotin. Next, by dissolving the human immunoglobulin IgG into HBS-P (10 mM HEPES pH 7.4, 150 mM NaCl, 0.05 % v/v Surfactant P20) as running buffer solution, 1 µM of sample solutions were prepared. The measurement of the SPR was performed at a reaction temperature of 25 °C with Biacore T100 (Biacore). After addition of the sample solutions, dissociation behavior of the IgG due to dissociation solutions (10 mM sodium acetate pH 4.0) was measured. BIAevaluation version 4.1 was used for analyses of observation results. By dividing RU changes before and after the dissociations by binding RU values of the IgG, remaining amount ratios of the IgG were calculated, and by fitting dissociation curves of the IgG to a 1:1 Langmuir model, dissociation rate constants k_{off} were determined.

Under the dissociation condition used for the experiment, significant dissociation was not shown in OXADac-PG01 with the wild-type amino acid sequence, whereas remarkable dissociation behavior was shown in the mutant proteins (OXADac-PG13, OXADac-PG14, OXADac-PG19 and OXADac-PG20) (Table 2). For example, in the OXADac-PG19, more than 60 % of adsorption IgG was dissociated under the condition used for the experiment, and the dissociation rate constant indicates 3 order or more of increase with respect to that of the wild-type.

### Example 11

In this example, the antibody-binding property of the mutant proteins, in the neutral region and a weakly acidic region in which more than 95 % of histidine residues were protonated, was evaluated by the surface plasmon resonance (SPR) method.

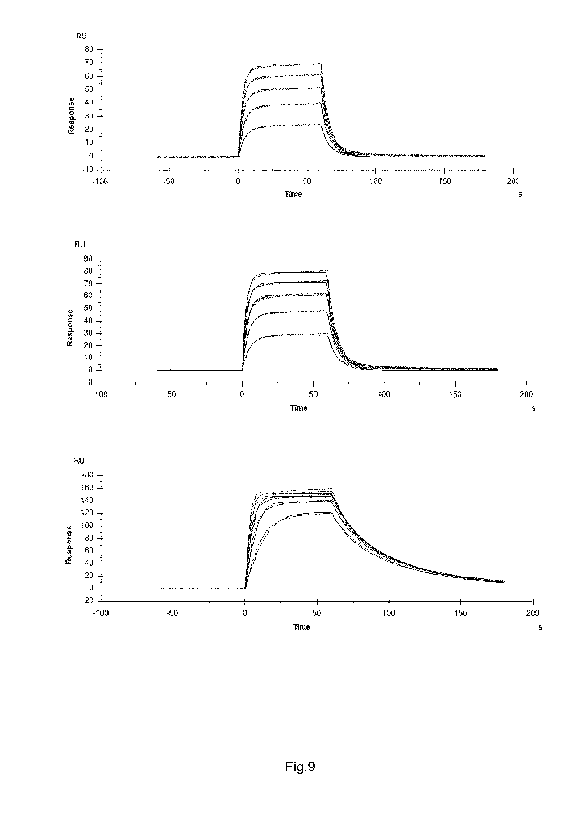

First, on measuring cells of the sensor chips, the Fc region of human immunoglobulin was immobilized by an amine coupling method. As a control of the measurement, reference cells in which carboxymetyl groups were blocked with ethanolamine were used. As the sensor chips, CM5 (Biacore) was used for the measurement in the neutral region and CM4 (Biacore) was used for the measurement in the weakly acidic region. Next, by dissolving the mutant proteins obtained by the isolation and purification into HBS-P (10 mM HEPES pH 7.4, 150 mM NaCl, 0.05 % v/v Surfactant P20) as running buffer solution in the neutral region or into running buffer solutions in the weakly acidic region (10 mM sodium acetate pH 4.5, 150 mM NaCl, 0.05 % v/v Surfactant P20)), sample solutions of five concentrations were prepared, respectively: 500 nM, 400 nM, 300 nM, 200 nM, 100 nM and 1000 nM, 800 nM, 600 nM, 400 nM, 200 nM. The measurement of the SPR was performed at a reaction temperature of 25 °C with Biacore T100 (Biacore). The collected data were analyzed with Biacore T100 Evaluation Software, and, by fitting to the 1:1 Langmuir model, dissociation equilibrium constants K_{D} were calculated (FIG. 9).

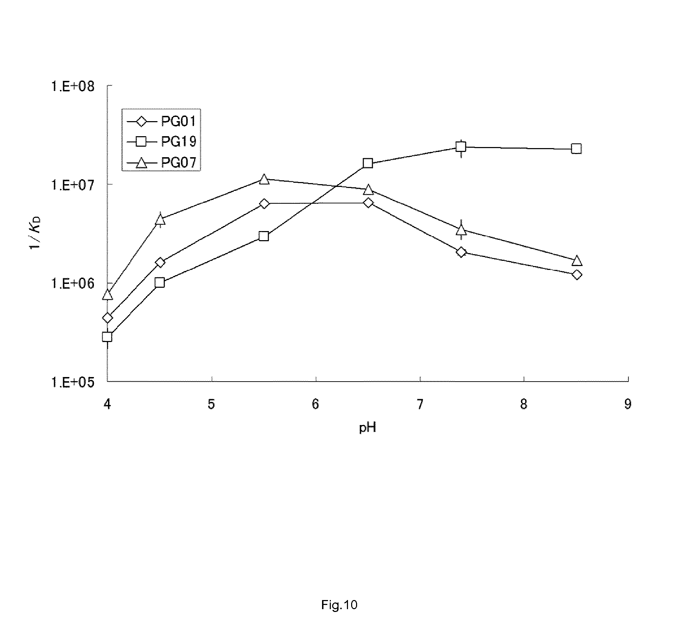

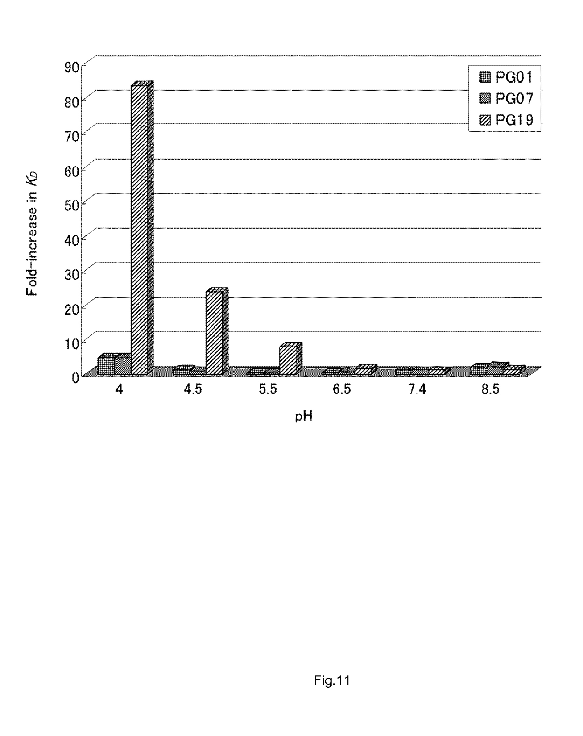

As a result, it was clarified that, although the mutant protein M-PG19 exhibits 11 times or more of avidity in the neutral region, an affinity in the acidic region decreases to around 0.6 times, in comparison with a control protein M-PG01 with the wild-type amino acid sequence (Table 3, FIG. 10). Moreover, according to calculation results of a ratio of the K_{D} at pH 4.0 and the K_{D} at pH 7.0 in each protein, the mutant protein M-PG19 has an extremely large ratio (FIG. 11). This means that an antibody elution amount due to a shift of the pH is large, and thus shows that, by using the mutant protein M-PG19, a recover rate of the antibody in the affinity chromatography can be greatly improved.

Table 3

**Table 3. Characteristics of Produced Modified Protein G, M-PG07, M-PG19, M-PG20**

| Sample No. | Binding activity of antibody | | Thermal stability | |
|---|---|---|---|---|
| | *K*_{D} at pH4.5 (M) | *K*_{D} at pH7.4 (M) | *T*ₘ (K) | Δ*H*ₘ (kJ/mol) |
| M-PG01 | 6.2 × 10⁻⁷ | 4.9 × 10⁻⁷ | 351.3 | 274 |
| M-PG07 | 2.3 × 10⁻⁷ | 2.9 × 10⁻⁷ | 360.5 | 275 |
| M-PG19 | 1.0 × 10⁻⁶ | 4.3 × 10⁻⁸ | 359.0 | 278 |
| M-PG20 | n.d. | n.d. | 346.7 | 226 |

| | | | | |
|---|---|---|---|---|
| n.d.: not determined | | | | |

### Example 12

In this example, the thermal stability of the mutant protein was evaluated. It is known that the circular dichroism (CD) spectrum is a spectroscopic analysis method sensitively reflecting change of the secondary structure of a protein. By observing molar ellipticity corresponding to intensity of the CD spectrum while changing a temperature of a sample, temperature around which each improved protein G is denatured can be clarified. Aqueous solutions containing the mutant proteins obtained by the isolation and purification with several concentrations of 15 µM to 25µM (50 mM sodium phosphate buffer solution, pH 6.8) were prepared. The sample solutions were injected in cylindrical cells (cell length 0.1 cm) and the CD spectra were obtained by moving a measurement wavelength from 260 nm to 195 nm at a temperature of 20 °C with J-805 circular dichroism spectrophotometer (JASCO). After the same samples were heated to 98 °C and further were cooled from 98 °C to 20 °C, circular dichroism spectra at a wavelength from 260 nm to 195 nm were obtained. Molar ellipticities from the spectra in the case of the re-cooling after heating recovered at more than 60%, so that it was confirmed that the stereoscopic structure of the improved protein G was reversible to thermal denaturation to some extent.

Next, the measurement wavelength was fixed at 222 nm, and temporal changes of molar ellipticities were measured while raising the temperature from 20 °C to 100 °C at a rate of 1 °C /min. Obtained thermal melting curves were analyzed by using a theoretical formula for two-state phase transition model (Non-patent Document: Arisaka, An Introduction to Protein Science), so that denaturing temperature Tₘ and change in enthalpy of denaturation at Tₘ ΔHₘ were determined. As a result, it was clarified that, among the measured improved proteins G, the thermal stability of the M-PG07 and the M-PG19 was improved in comparison with the control protein (M-PG01) with the wild-type amino acid sequence (Table 3).

### Example 13

In this example, single crystals of the mutant protein were produced and the stereoscopic structure was determined by an X-ray diffraction analysis.

First, an isolated and purified mutant protein M-PG19 was crystallized by the following hanging drop method. To obtain single crystals belonging to a space group P43212, crystallizing solutions were prepared by dropping and mixing 1µl of protein sample solution obtained by dissolving the protein sample into a tris-hydrochloric acid buffer solution of 10 mM and pH 7.4 to become a concentration of 5-10 mg/ml and an equal amount of crystallizing agent solution (70 % MPD, 20 mM HEPES buffer solution pH 7.4) on cover glasses (manufactured by Hampton corp.) with Pipetman. The above-mentioned crystallizing agent solution was injected in a 24-well plate manufactured by Hampton corp., and then, by covering with the cover glasses on which the crystallization solutions were dropped, the solutions were sealed with a high vacuum grease. The plate was stored in an incubator which was kept at 20 °C. After approximately 1 to 2 weeks, high quality single crystals were obtained in the crystallization solutions.

Next, the obtained single crystals were scooped in a loop for crystal analysis with a very small amount of mother liquor, and were rapidly frozen with liquid nitrogen gas to use in an X-ray diffraction experiment. The diffraction measurement was performed with Beam line BL-6A at Photon Factory in High Energy Accelerator Research Organization according to a conventional method, and diffraction datas to resolution 1.6 Å were collected. To obtained diffraction images, indexing of diffraction spots and, measurement and digitalizing of integrated intensity were performed with a program HKL-2000 (HKL Research Inc.), so that 68,935 intensity data were obtained. In this stage, crystal parameters of the used single crystals were determined. Namely, the space group of the crystals was tetragonal P43212, and a lattice constant was a = b = 23.26 Å and c = 178.7 Å. Moreover, merging and scaling were performed with HKL-2000, so that 8,862 unique intensity data were obtained. An R_{sym} value of the data was 6.8 %.

The structure determination was performed by a molecular substitution method with the three-dimensional coordinate data of the B1 domain of the wild-type protein G and a program Molrep (Vagin, A., and Teplyakov, A. (1997) Journal of Applied Crystallography 30, 1022-1025), and then a structure refinement was performed with programs CNS (Brunger, A. et al.(1998) Acta. Crystallogr. D Biol. Crystallogr. 54, 905-921), REFMAC5 (Murshudov, G. N., et al. (1997) Acta. Crystallogr. D Biol. Crystallogr. 53, 240-255) and Coot (Emsley, P., and Cowtan, K. (2004) Acta. Crystallogr. D Biol. Crystallogr. 60, 2126-2132). As a result, an R-value which is considered as an index of parameter accuracy by those skilled in the art was 23 % to all intensity data.

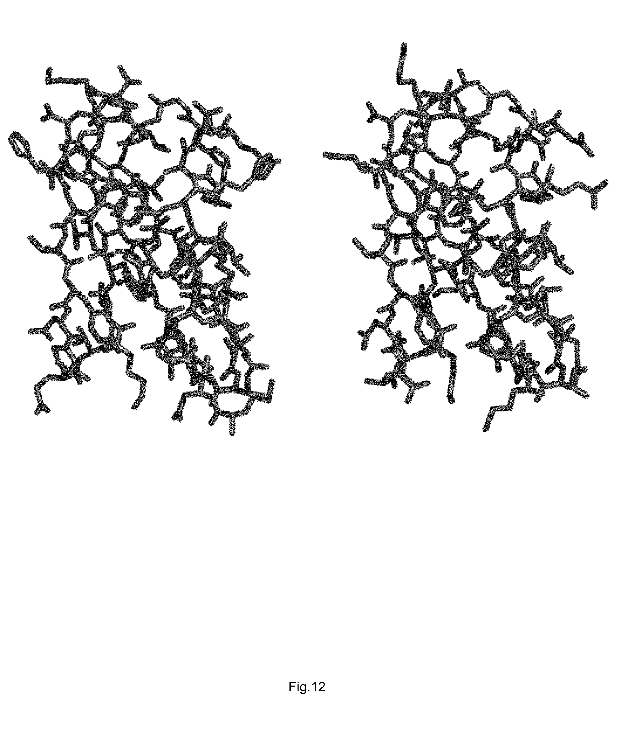

The three-dimensional structure of the mutant protein M-PG19 thus obtained was extremely similar to the B1 domain of the wild-type protein G. Namely, when determined coordinates of a main chain of the M-PG19 and coordinates of a main chain of the wild-type registered in the Protein Data Bank (PDB code: 1PGA) are compared, the root mean square deviation (RMSD) is 0.71 Å. From the above results, it was proved that the amino acid substitution performed to the mutant protein in the present invention does not almost change the stereoscopic structure of the B1 domain of the wild-type protein G (FIG. 12).

### Example 14

Production of the tandem-type multimer of the extracellular domain mutants of the protein G of the present invention and preparation of the column using the protein

### (1) Preparation of recombinant PG expression plasmid

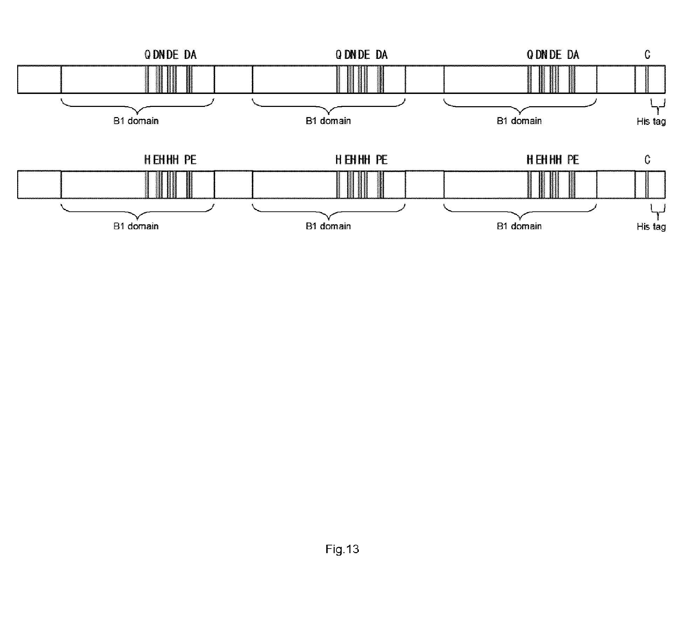

By using restriction enzymes NcoI and BamHI, gene fragments were respectively extracted from two kinds of artificial synthesis plasmids (SYN2608-2-18 and SYN2608-1-4, respectively, Takara Bio) incorporated with genes encoding the trimer wild-type PG (CGB01H-3D, FIG. 13 upper, SEQ ID NO. 36) or the tandem-type trimer of the mutant-type PG, which is the protein of the present invention, (CGB19H-3D, FIG. 13 under, SEQ ID NO. 37), in both of which the cysteine residue and the His tag had been added to the carboxyl terminal side. The target fragments were separated by the agarose electrophoresis and were purified with a QIAquick Gel Extraction Kit (Qiagen), and then were ligated with plasmids for expression pET16b (Invitrogen) to which similar restriction enzyme treatment and dephosphorylation with an alkali dephosphorylation enzyme derived from bovine small intestine (CIP, Takara Shuzo) had been performed. Escherichia coli strains for preservation DH5 (Competent high, Toyobo) were transformed with the reaction liquid. The obtained transformants were selected by a colony PCR method and a DNA sequencing method (BigDye Terminator v1.1, GE Healthcare Bioscience), and the recombinant PG expression plasmids were extracted with the QIAprep Spin Miniprep Kit (Qiagen).

### (2) Expression and purification of the recombinant PG

The escherichia colis strains for expression BL21(DE3) (Novagen) were transformed with the recombinant PG expression plasmids. The precultured transformants were subcultured into the LB mediums in 2.5 ml / 500 ml, and were shake cultured to O.D.₆₀₀ = 0.8 to 1.0. After 0.5 mM of IPTG was added in order to express the target proteins, the transformants were further shake cultured at 37 °C for two hours. The collected cell bodies were suspended in 10 ml of PBS and then were ultrasonically crushed before the filter sterilization, and the obtained solutions were treated as wholly protein solutions. After the recombinant PG were adsorbed on Ni Sepharose (GE Healthcare Bioscience) 2 ml columns and were washed with 20 mM of imidazole, purified proteins were eluted with 500 mM of imidazole.

### (3) Immobilization of the recombinant PG with Epoxy-activated Sepharose 6B, and preparation of the column

After 2.5 mg of the purified recombinant PG were dissolved into 50 mM of phosphate buffer (pH 8.0), the solutions were mixed with 0.3 g of Epoxy-activated Sepharose 6B (GE Healthcare) which had been equilibrated in the same manner, and were reacted at 37 °C for one day to bind the recombinant PG to the resins. The amount of a non-immobilized supernatant sample after the reaction was 1.28 mg in CGB01H-3D and 0.97 mg in CGB19H-3D, from which it was estimated that an immobilization rate was 49 % and 61 %, respectively. The resultants were washed with 50 mM of phosphate buffer, and then, by adding 1M of ethanolamine (pH 7.5), were reacted at 37 °C for six hours to mask unreacted functional groups. The resultants were washed with a washing liquid 1 (0.1 M acetic acid, 0.1 M sodium chloride), and then with a washing liquid 2 (0.1 M tris-hydrochloric acid, 0.5 M sodium chloride, pH 8.0). 1 ml of recombinant PG-immobilized resins were packed in Tricon 5/20 Columns.

### (4) Immobilization of the recombinant PG with SulfoLink Immobilization Kit (Pierce), and preparation of the column

After 2.5 mg of the purified CGB19H-3D was dissolved into a sample preparation buffer solution (0.1 M sodium phosphate, 5 mM EDTA, pH 6.0), the solution was added in an attached mercaptoethanol vial to react at 37 °C for one and a half hours. After the reaction liquid was added to an attached desalting column to remove the mercaptoethanol, the resultant was prepared with a coupling buffer solution (50 mM tris-hydrochloric acid, 5 mM EDTA, pH 8.5) and was added to SulfoLink Resin. The resin was reacted at room temperature for 15 minutes to bind the recombinant PG to the resin. The amount of a non-immobilized sample after the reaction was 0.18 mg, from which it was estimated that the immobilization rate was 75 %. The resin was washed with 1M of sodium chloride, and then, by adding 50 mM of L-cystein hydrochloric acid, was reacted at room temperature for one hour to mask unreacted functional groups. The resin was washed with PBS, and then 1 ml of the immobilized resin was packed in Tricon 5/20 Column.

### 2. pH gradient affinity chromatography

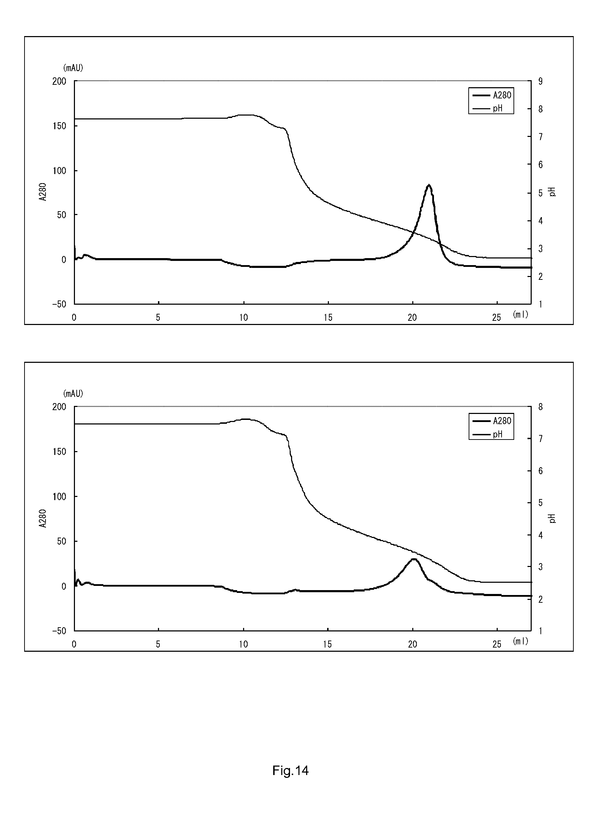

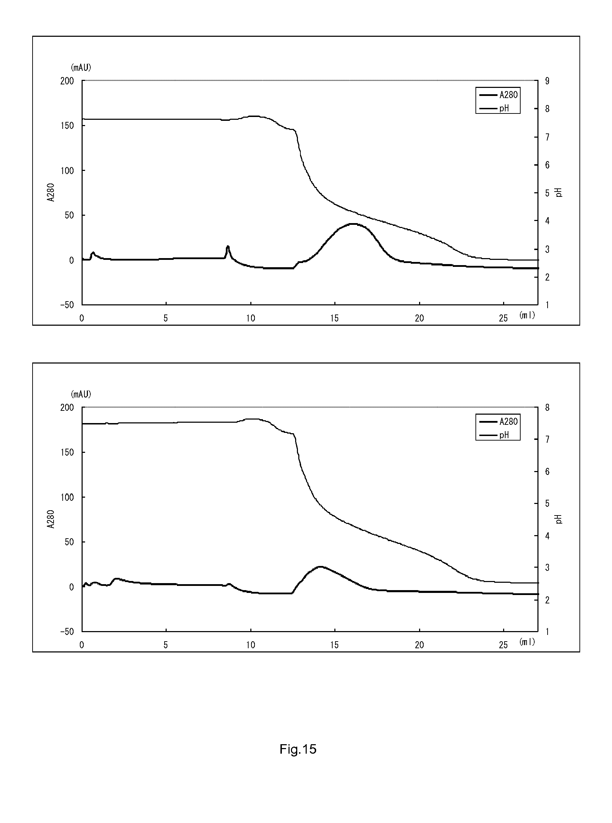

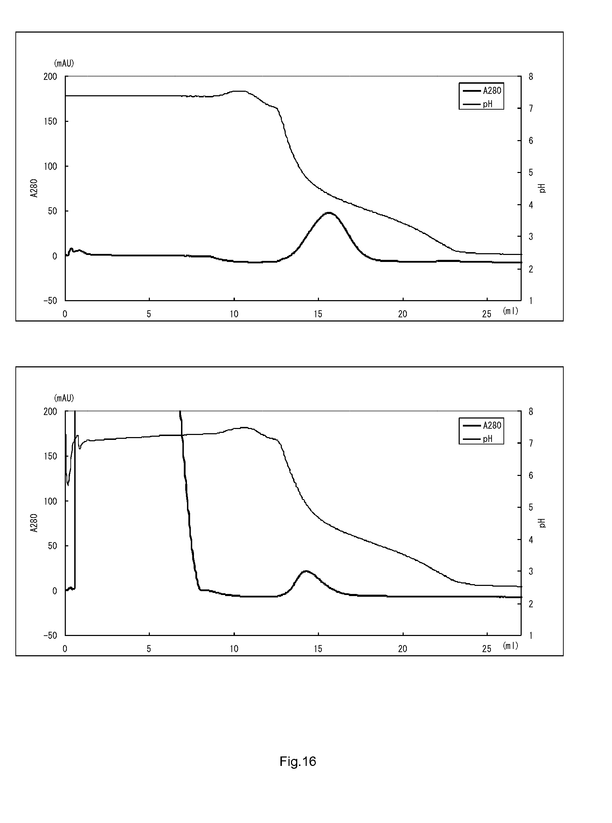

After the recombinant PG immobilized columns were set to the liquid chromatography apparatus AKTApurifier (GE Healthcare Bioscience) and were equilibrated by supplying TST buffer solutions (50 mM tris-hydrochloric acid, 150 mM sodium chloride, 0.05% Tween20, pH 7.6) under a condition of 0.3 ml /min (0.5 ml/min for 1.- (4)), the IgG1 type humanized monoclonal antibodies prepared to 100µg/200µl or human IgG3 prepared to 50 µg/µl were injected. The TST buffer solution was replaced with 50 mM of sodium citrate (pH 7.0), and further continuously replaced with 0.5 M of acetic acid (pH 2.5) for 10 min at a flow rate of 0.3 ml/min (0.5 ml/min for 1.- (4)) to determine pH conditions for eluting the IgG1 or the IgG3. In the CGB01H-3D immobilized column, the IgG1 was eluted between pH 3.9 and pH 2.9: a peak is formed around pH 3.3 (FIG. 14 upper), and the IgG3 was eluted between pH 5.1 and pH 3.8: a peak is formed around pH 3.4 (FIG. 14 under). On the other hand, in the Epoxy-activated column of the CGB19H-3D immobilized columns, the IgG1 was eluted between pH 5.4 and pH 3.8: a peak is formed around pH 4.3 (FIG. 15 upper), and the IgG3 was eluted between pH 6.2 and pH 4.1: a peak is formed around pH 4.9 (FIG. 15 under). In the SulfoLink column (CGB19H-3D only), the IgG1 was eluted between pH 5.9 and pH 3.7: a peak is formed around pH 4.3 (FIG. 16 upper), and the IgG3 was eluted between pH 6.2 and pH 4.2: a peak is formed around pH 5.2 (FIG. 16 under). From the above results, it was clarified that, in each case of the CGB19H-3D immobilized columns, the antibodies can be eluted under the milder acidic conditions in comparison with the CGB01H-3D immobilized column, and that both of the IgG1 antibody and the IgG3 antibody can be purified with the CGB19H-3D immobilized column.

### 3. pH stepwise change affinity chromatography

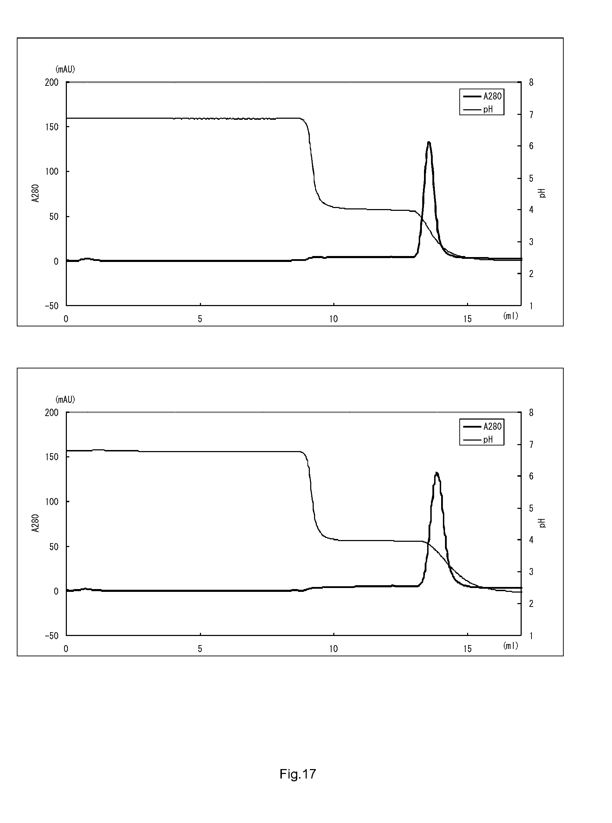

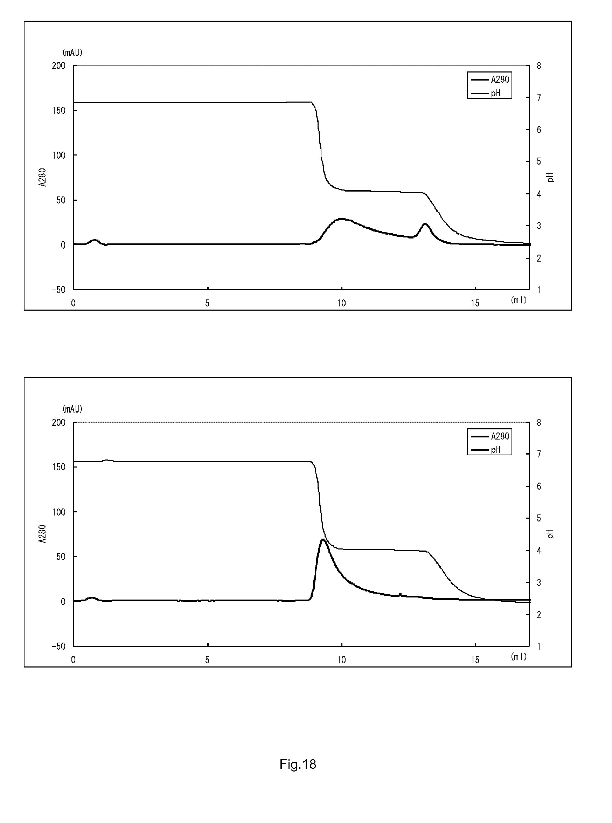

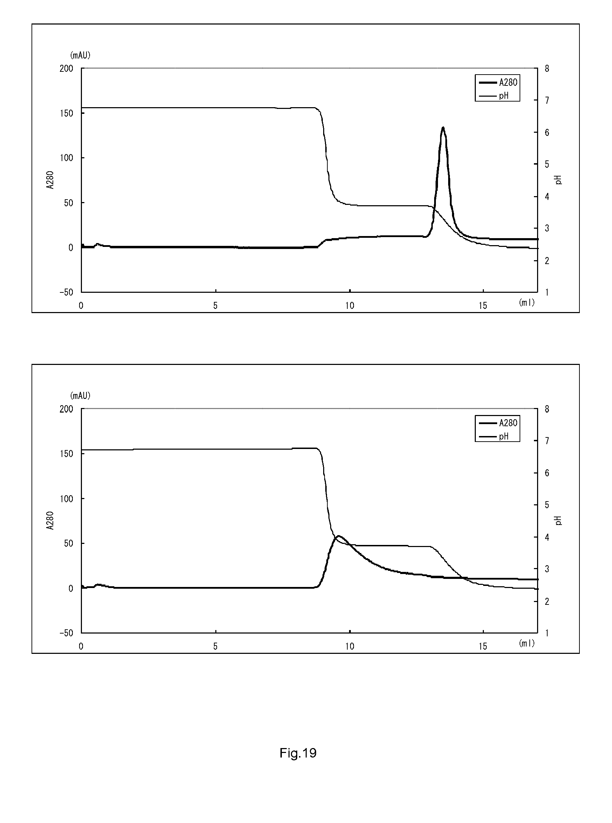

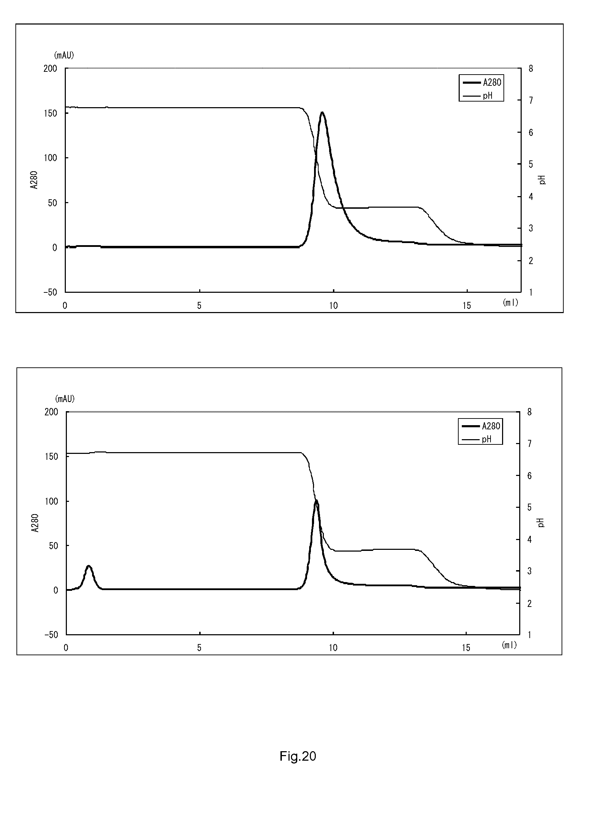

After the recombinant PG immobilized columns were set to the liquid chromatography apparatus AKTApurifier (GE Healthcare Bioscience) and were equilibrated by supplying phosphate buffer solutions (20 mM sodium phosphate, 150 mM sodium chloride, pH 7.0) under a condition of 0.3 ml /min (0.5 ml/min for 1.- (4)), the IgG1 type humanized monoclonal antibodies prepared to 100µg/200µl or human IgG3 prepared to 50 µg/µl were injected. The phosphate buffer solution was replaced with 20 mM of sodium citrate (pH 4.0 or pH 3.75), and further replaced with 20 mM of citric acid (pH 2.4), at a flow rate of 0.3 ml/min (0.5 ml/min for 1.- (4)), to determine pH conditions for eluting the IgG1 or the IgG3. In the CGB01H-3D immobilized columns, the IgG1 (FIG. 17 upper) and the IgG3 (FIG. 17 under) were not eluted by a change from pH 7.0 to pH 4.0 and were eluted by a change from pH 4.0 to pH 2.4, respectively. On the other hand, in the CGB19H-3D immobilized columns, the IgG1 (FIG. 18 upper) and the IgG3 (FIG. 18 under) were eluted by the change from pH 7.0 to pH 4.0, and were not eluted by the change from pH 4.0 to pH 2.4, respectively. Also, at pH 3.75 which was the stronger acid condition, the same results were obtained: in the CGB01H-3D immobilized column, the IgG1 was not eluted by a change from pH 7.0 to pH 3.75 and was eluted by a change from pH 3.75 to pH 2.4 (FIG. 19 upper), and in the CGB19H-3D immobilized column, the IgG1 was eluted by the change from pH 7.0 to pH 3.75 and was not eluted by the change from pH 3.75 to pH 2.4 (FIG. 19 under) (in both columns, IgG1 only). In the SulfoLink columns (CGB19H-3D only), the same results were also obtained for the IgG1 (FIG. 20 upper) and the IgG3 (FIG. 20 under), respectively. From the above results, it was clarified that, in each case of the CGB19H-3D immobilized columns, the antibodies can be eluted under the milder acidic conditions in comparison with the CGB01H-3D immobilized column, and that both of the IgG1 antibody and the IgG3 antibody can be purified with the CGB19H-3D immobilized column.

### Example 15

In this example, the tandem-type multimer of the extracellular domain mutants of the protein G of the present invention and the monomer thereof were compared.

The antibody binding dissociation in the neutral region of single domain-type and 3 domains-type molecules of the extracellular domain mutants of the protein G (referred to as M-PG19 and CGB19H-3D, respectively) was evaluated by the surface plasmon resonance (SPR) method. It has been recognized that the SPR method is a superior method in which a specific interaction between biopolymers can be measured over time and in which the reaction can be interpreted quantitatively from the kinetic viewpoint.

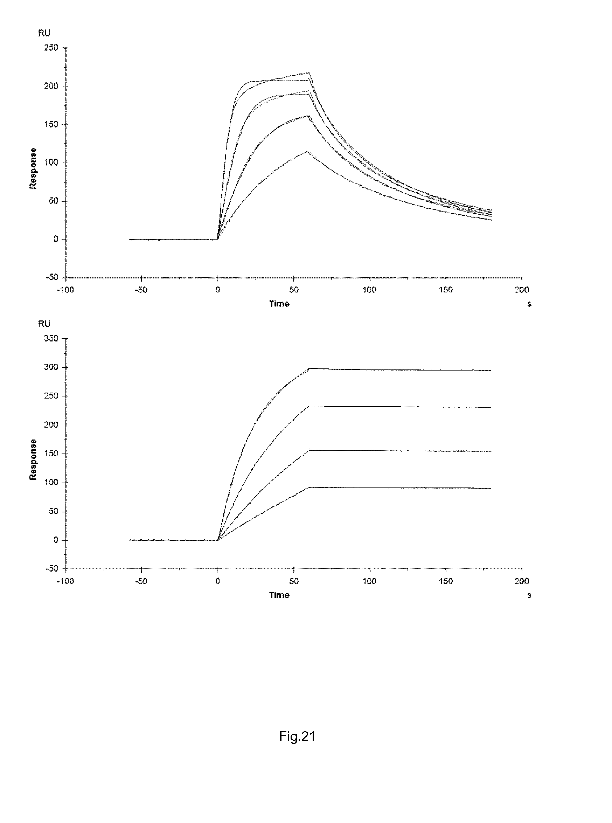

First, on measuring cells of the sensor chips CM-5 (GE Healthcare), the IgG1 type humanized monoclonal antibodies were immobilized by the amine coupling method. The immobilization amounts were determined to 5000 RU. Next, by dissolving M-PG19 and CGB19H-3D into running buffer solutions (10 mM HEPES pH 7.4, 150 mM NaCl, 1 mM Cystein, 0.05 % v/v SurfactantP20), sample solutions were prepared, respectively: 25 nM, 50 nM, 100 nM, 200 nM (M-PG19) and 6.25 nM, 12.5 nM, 25 nM, 50 nM (CGB19H-3D). The measurement of the SPR was performed at a reaction temperature of 25 °C with Biacore T100 (GE Healthcare). BIAevaluation version 4.1 was used for kinetic analyses of observation results. By fitting dissociation curves to the 1:1 Langmuir model, equilibrium dissociation constants K_{D} were determined. The single domain-type M-PG19 was bound to the IgG1 with the K_{D} of 19 nM (FIG. 21 upper). On the other hand, the 3 domains-type CGB19H-3D was bound to the IgG1 with the K_{D} of 0.10 nM (FIG. 21 under). The above-mentioned results show that 190-fold avidity improvement can be realized by producing the protein G mutants as the multi domain-type. Moreover, it was also clarified that the avidity improvement was caused by decrease in the dissociation rate mainly rather than a binding rate. When only the dissociation rate constants are compared, the difference between the two constants is approximately 370 times. It is conceivable that this results from an avidity effect (a multivalent effect) due to the production as the multi domain-type.

### Example 16

A recombinant PG expression plasmid was prepared, and the recombinant PG was expressed and purified in a similar way as Example 14. The capturing agent and column according to the present invention were then prepared by means of the following various immobilization methods.

### (1) Immobilization of the recombinant PG with CNBr-activated Sepharose 4F FastFlow, and preparation of the column

After 0.375g of CNBr-activated Sepharose 4F FastFlow (GE Healthcare) was weighed, it was swollen in I mM of HCl and washed on a glass filter, and then washed with a coupling buffer (0.1M sodium bicarbonate, 0.5 M sodium chloride, pH 8.0). The resulting carrier was then transferred into a flask, mixed with 1.5 ml of the coupling buffer and 432µl of a solution comprising 17.4 mg/ml of the recombinant PG, and shaken at 4 °C and 130 rpm for 21 hours so as to immobilize the recombinant PG to the carrier. The carrier was then filtered through the glass filter and washed with the coupling buffer. The resulting filtrate was determined by BradFord method to show that 4.9 mg of the recombinant PG had been immobilized per 1 ml of the carrier. The carrier was then transferred into a flask, mixed with 3ml of 1M ethanol amine aqueous solution (pH 8.0) and shaken at 25 °C and 130 rpm for two hours so as to mask unreacted active groups. It was filtered through the glass filter, washed in three cycles alternatively with 15 ml of a washing solution 1 (0.1 M tris-HCl, 0.5M sodium chloride, pH8.0) and 15 ml of washing solution 2 (0.1 M tris-HCl, 0.5M sodium chloride, pH8.0). The immobilized carrier (1 ml) was washed with ultrapure water and packed into Tricon 5/20 Column.

### (2) Immobilization of the recombinant PG with Epoxy-activated Sepharose 4F FastFlow, and preparation of the column

Sepharose 4F FastFlow (GE Healthcare) was filtered through a glass filter washed with ultrapure water to give 10 ml of carrier. The resulting carrier was then transferred into a flask, mixed with 3 ml of 4.6 M sodium hydroxide aqueous solution and 4 g of butanediol diglycidyl ether to react at 36 °C for two hours under shaking. Filtration through the glass filter and washing with ultrapure water gave an activated carrier. The activated carrier (1 ml) was taken on the glass filter and washed with a coupling buffer (0.1M sodium carbonate, 1 mM EDTA, pH 9.0). The activated carrier was then transferred into a flask, mixed with 1 ml of the coupling buffer and 500 µl of a solution comprising 20 mg/ml of the recombinant PG, and shaken at 37°C and 130 rpm for 14 hours so as to immobilize the recombinant PG to the carrier. The carrier was then filtered through the glass filter and washed with the coupling buffer. The resulting filtrate was determined by BradFord method to show that 4.0 mg of the recombinant PG had been immobilized per 1 ml of the carrier. The carrier was then transferred into a flask, mixed with 1M ethanol amine, 0.1 M sodium carbonate, ImM EDTA solution (3 ml), and shaken at 25 °C and 130 rpm for three hours so as to mask unreacted active groups. It was filtered through the glass filter, washed in three cycles alternatively with 15 ml of a washing solution 1 (0.1 M tris-HCl, 0.5M sodium chloride, pH8.0) and 15 ml of washing solution 2 (0.1 M acetic acid, 0.5M sodium chloride, pH4.0). The immobilized carrier (1 ml) was washed with ultrapure water and packed into Tricon 5/20 Column.

### (3) Immobilization of the recombinant PG with N,N'-disuccinimidyl carbonate, and preparation of the column

Sepharose 4F FastFlow (GE Healthcare) was filtered through a glass filter washed with acetonitrile to give 6.5 ml of carrier. The resulting carrier was then transferred into a flask, mixed with 5 ml of acetonitrile, and 20 ml of a acetonitrile solution containing 0.11 g of N,N'-disuccinimidyl carbonate, and shaken at 4 °C and 100 rpm. It was then mixed with 2 ml of an acetonitrile solution containing 0.08 g of N,N'-dimethylamino pyridine to react for 19 hours under shaking. Filtration through the glass filter and washing successively with 50 ml of acetonitrile, 50 ml of dioxane containing 5% acetic acid, 50 ml of methanol and 2-propanol gave an activated carrier. The activated carrier (1 ml) was taken on the glass filter and washed with a coupling buffer (0.1M calcium phosphate, pH 7.6). The activated carrier was then transferred into a flask, mixed with 1 ml of the coupling buffer and 575 µl of a solution comprising 17.4 mg/ml of the recombinant PG, and shaken at 4°C and 130 rpm for 23 hours so as to immobilize the recombinant PG to the carrier. The carrier was then filtered through the glass filter and washed with the coupling buffer. The resulting filtrate was determined by BradFord method to show that 8.2 mg of the recombinant PG had been immobilized per 1 ml of the carrier. The carrier was then transferred into a flask, mixed with 3 ml of 1M ethanol amine and shaken at 4 °C and 130 rpm for five hours so as to mask unreacted active groups. It was filtered through the glass filter, washed with the coupling buffer. The immobilized carrier (1 ml) was washed with ultrapure water and packed into Tricon 5/20 Column.

### Example 17

### 1. Static Binding Capacity (SBC) of the recombinant PG-immobilized filler

The Static Binding Capacity of the recombinant PG-immobilized filler prepared in Examples 14 and 16 were then determined as follows.

A solution (400 µl) of 25% of the recombinant PG-immobilized carrier in PBS suspension was collected in a conical plastic container. PBS solution (14 ml) of γ-globulin derived from human serum (Wako Pharmaceuticals) at 1mg/ml was added to the container, followed by stirring at 25 °C for three hours with a rotary mixer. The concentration of γ-globulin in a supernatant was determined based on absorbance at 280 nm, and SBC was then obtained on a basis of material balance.

SBC of each immobilized filler is summarized in Table 4 below. It was confirmed that the recombinant PG-immobilized filler (column) according to the present invention had a higher (larger) static binding capacity (SBC) than a capturing agent that was immobilized to a water-insoluble solid support via mainly the -SH group.

Table 4

| **Activated Carrier** | **Carrier** | **Functional group of ligand** | **SBC** |
|---|---|---|---|
| **CNBr-activated Sepharose 4FastFlow** | **Cross-linked 4% agarose** | **- NH2** | **44** |
| **Epoxy-activated Sepharose 4FastFlow** | **Cross-linked 4% agarose** | **-NH2, -OH, >-SH** | **41** |
| **N,N'-disuccinimidyl carbonate activated Sepharose4FastFlow** | **Cross-linked 4% agarose** | **-NH2** | **48** |
| **SulfoLink Immobilization** | **Cross-linked 6% agarose** | **-SH** | **13** |

### 2. pH gradient affinity chromatography with the recombinant PG-immobilized column

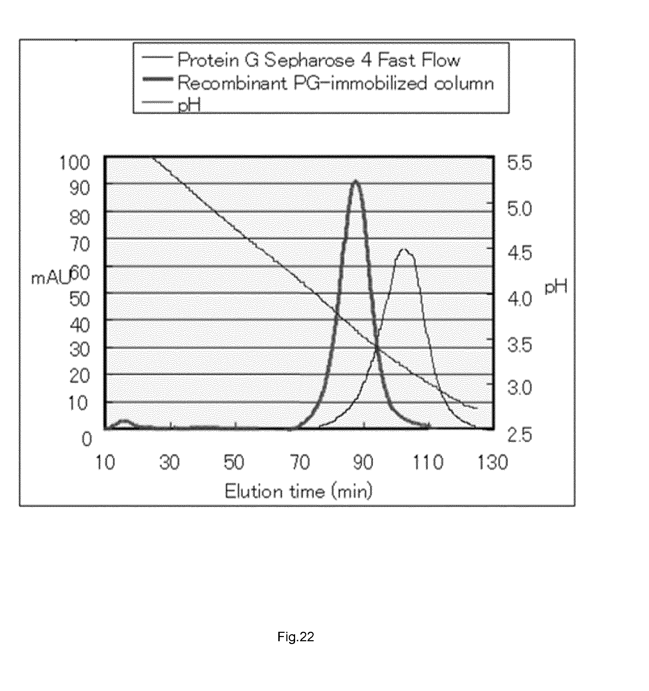

After Sepharose 4 Fast Flow column (GE Healthcare) or the recombinant PG-immobilized column according to the present invention (DSC-activated CGB19H-3D-immobilized column prepared with N,N'-disuccinimidyl carbonate in Example 16) was equilibrated by supplying an absorbing buffer solution (20 mM sodium phosphate, pH 7.2, 150 mM NaCl) under a condition of 0.2 ml /min, human γ-globulin derived from human serum (Wako Pharmaceuticals) adjusted at 1mg/ml was injected. After being washed with the absorbing buffer solution, it was then continuously replaced with 20 mM of citric acid (pH 2.4) at a flow rate of 1 ml/min for 130 min to determine pH conditions for eluting the IgG. In the Sepharose 4 Fast Flow, the IgG was eluted between pH 3.6 - pH 2.9: a peak was formed around pH 3.2. In the recombinant PG-immobilized column, the IgG was eluted between pH 4.0 - pH 3.3: a peak was formed around pH 3.6 (FIG. 22). From the above results, it was clarified that, in the case of the recombinant PG-immobilized column according to the present invention, the antibodies can be eluted under the milder acidic conditions in comparison with the Sepharose 4 Fast Flow column.

### Industrial Applicability

Currently, the extracellular domain of the wild-type protein G is marketed as an affinity chromatography carrier for purifying an antibody and an inspection reagent for detecting an antibody, and is widely used in each field of life science. Moreover, with recent development of the antibody-related industries, including antibody medicine, demand for these products expands dramatically.

Accordingly, the capturing agent wherein a protein consisting of a tandem-type multimer of extracellular domain mutants that have binding property to the protein comprising the Fc region of the immunoglobulin G is immobilized to a water-insoluble solid support via mainly an amino group, and the column for a chromatography for separating and purifying the protein, which is filled with the capturing agent, which are provided by the present invention, will significantly contribute to the technical development in the wide technical field where the antibody is treated.

## Claims

1. A capturing agent for an antibody, immunoglobulin G or a protein comprising an Fc region of the immunoglobulin G, wherein a protein consisting of a tandem-type multimer of extracellular domain mutants that have binding property to the protein comprising the Fc region of the immunoglobulin G is immobilized to a water-insoluble solid support via mainly an amino group.

2. The capturing agent according to the claim 1, which is a capturing agent for an antibody, immunoglobulin G or a protein comprising an Fc region of the immunoglobulin G, wherein a protein consisting of a tandem-type multimer of extracellular domain mutants that have binding property to the protein comprising the Fc region of the immunoglobulin G is immobilized to a water-insoluble solid support, and a static binding capacity (SBC) is 40 (mg-human IgG/ml-gel) or more.

3. The capturing agent according to the claim 2, wherein the static binding capacity (SBC) is 44 (mg-human IgG/ml-gel) or more

4. The capturing agent according to any one of the claims 1 to 3, wherein the tandem-type multimer is a tandem-type trimer.

5. The capturing agent according to any one of the claims 1 to 4, wherein the extracellular domain mutants constituting the multimer are the same as one another.

6. The capturing agent according to any one of the claims 1 to 5, wherein each of the extracellular domain mutants is connected by a linker sequence.

7. The capturing agent according to any one of the claims 1 to 6, wherein the extracellular domain having the binding property to the protein comprising the Fc region of immunoglobulin G is any one of B1, B2 and B3 of a protein G from streptococcus of genus Streptococcus.

8. The capturing agent according to any one of the claims 1 to 7, wherein
the protein has the binding property to the Fc region of immunoglobulin G, and
at least binding property of the protein to an Fab region of immunoglobulin G and/or binding property of the protein to the Fc region in a weakly acidic region is decreased in comparison with a protein consisting of a tandem-type multimer of B domain of a wild-type protein G.

9. The capturing agent according to any one of the claims 1 to 8, wherein
at least one of the extracellular domain mutants constituting the multimer is a mutant protein of B1 domain protein of the wild-type protein G,
the mutant protein consists of an amino acid sequence represented by (a) or of the amino acid sequence obtained by deleting, substituting, inserting or adding one or several amino acid residues in the amino acid sequence represented by (a),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with a B1 domain protein of the wild-type protein G.
(a)AspThrTyrLysLeuIleLeuAsnGlyLysX11LeuLysGlyGluThrX17ThrGluAlaValX22Ala AlaX25AlaGluLysValPheLysX32TyrAlaX35X36X37GlyValX40GlyX42TrpThrTyrAspX47X4 8ThrLysThrPheThrValThrGlu
(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or Leu; X47 is Asp or Pro; X48 is Ala, Lys or Glu; X22 is Asp; X25 is Thr; X32 is Gln; X40 is Asp; X42 is Glu; and X11 is Thr, and X17 is Thr simultaneously.)

10. The capturing agent according to any one of the claims 1 to 8, wherein
the at least one extracellular domain mutant constituting the multimer is a mutant protein of B2 domain protein of the wild-type protein G,
the mutant protein consists of an amino acid sequence represented by (b) or of the amino acid sequence obtained by deleting, substituting, inserting or adding one or several amino acid residues in the amino acid sequence represented by (b),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B2 domain protein of the wild-type protein G.
(b)ThrThrTyrLysLeuValIleAsnGlyLysX11LeuLysGlyGluThrX17ThrGluAlaValX22Ala AlaX25AlaGluLysValPheLysX32TyrAlaX35X36X37GlyValX40GlyX42TrpThrTyrAspX47X4 8ThrLysThrPheThrValThrGlu
(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; X42 is Glu; and X11 is Thr and X17 is Thr simultaneously.)

11. The capturing agent according to any one of the claims 1 to 8, wherein
the at least one extracellular domain mutant constituting the multimer is a mutant protein of B3 domain protein of the wild-type protein G,
the mutant protein consists of an amino acid sequence represented by (c) or of the amino acid sequence obtained by deleting, substituting, inserting or adding one or several amino acid residues in the amino acid sequence represented by (c),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B3 domain protein of the wild-type protein G.
(c)ThrThrTyrLysLeuV alIleAsnGlyLysX11LeuLysGlyGluThrX17ThrLysAlaValX22Ala GluX25AlaGluLysAlaPheLysX32TyrAlaX35X36X37GlyValX40GlyValTrpThrTyrAspX47X4 8ThrLysThrPheThrValThrGlu
(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; and X11 is Thr and X17 is Thr simultaneously.)

12. The capturing agent according to any one of the claims 1 to 8, wherein
the at least one extracellular domain mutant constituting the multimer is a mutant protein of B 1 domain protein of the wild-type protein G,
the mutant protein consists of an amino acid sequence represented by (d) or of the amino acid sequence obtained by deleting, substituting, inserting or adding one or several amino acid residues in the amino acid sequence represented by (d),
the mutant protein has the binding property to the Fc region of immunoglobulin G, and
the mutant protein has the binding property to the Fab region of immunoglobulin G and/or the binding property to the Fc region in the weakly acidic region is decreased in comparison with B1 domain protein of the wild-type protein G.
(d)AspThrTyrLysLeuIleLeuAsnGlyLysX11LeuLysGlyGluThrX17ThrGluAlaValX22AlaAla X25AlaGluLysValPheLysX32TyrAlaAsnAspAsnGlyValX40GlyX42TrpThrTyrAspAspAlaThrLys ThrPheThrValThrGlu
(In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

13. The capturing agent according to any one of the claims 1 to 8, wherein
the at least one extracellular domain mutant constituting the multimer is a mutant protein of B2 domain protein of the wild-type protein G,
the mutant protein consists of an amino acid sequence represented by (e) or of the amino acid sequence obtained by deleting, substituting, inserting or adding one or several amino acid residues in the amino acid sequence represented by (e),
the mutant protein has the binding property to the Fc region of immunoglobulin G, and
the mutant protein has the binding property to the Fab region of immunoglobulin G and/or the binding property to the Fc region in the weakly acidic region is decreased in comparison with B2 domain protein of the wild-type protein G.
(e)ThrThrTyrLysLeuValIleAsnGlyLysX11LeuLysGlyGluThrX17ThrGluAlaValX22AlaAla X25AlaGluLysValPheLysX32TyrAlaAsnAspAsnGlyValX40GlyX42TrpThrTyrAspAspAlaThrLys ThrPheThrValThrGlu
(In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

14. The capturing agent according to any one of the claims 1 to 8, wherein
the at least one extracellular domain mutant constituting the multimer is a mutant protein of B3 domain protein of the wild-type protein G,
the mutant protein consists of an amino acid sequence represented by (f) or of the amino acid sequence obtained by deleting, substituting, inserting or adding one or several amino acid residues in the amino acid sequence represented by (f),
the mutant protein has the binding property to the Fc region of immunoglobulin G, and
the mutant protein has the binding property to the Fab region of immunoglobulin G and/or the binding property to the Fc region in the weakly acidic region is decreased in comparison with B3 domain protein of the wild-type protein G.
(f)ThrThrTyrLysLeuValIleAsnGlyLysX11LeuLysGlyGluThrX17ThrLysAlaValX22Ala GluX25AlaGluLysAlaPheLysX32TyrAlaAsnAspAsnGlyValX40GlyValTrpThrTyrAspAspAla ThrLysThrPheThrValThrGlu
(In the amino acid sequence, X22 represents Asp or His; X25 represents Thr or His; X32 represents Gln or His; X40 represents Asp or His; X11 represents Thr or Arg; and X17 represents Thr or Ile, respectively, with the proviso that a case is excluded where X22 is Asp; X25 is Thr; X32 is Gln; X40 is Asp; and X11 is Thr and X17 is Thr simultaneously.)

15. The capturing agent according to any one of the claims 1 to 8, wherein
the at least one extracellular domain mutant constituting the multimer is a mutant protein of B 1 domain protein of the wild-type protein G,
the mutant protein consists of an amino acid sequence represented by (g) or of the amino acid sequence obtained by deleting, substituting, inserting or adding one or several amino acid residues in the amino acid sequence represented by (g),
the mutant protein has the binding property to the Fc region of immunoglobulin G, and
the mutant protein has the binding property to the Fc region in the weakly acidic region is decreased in comparison with B 1 domain protein of the wild-type protein G.
(g)AspThrTyrLysLeuIleLeuAsnGlyLysThrLeuLysGlyGluThrThrThrGluAlaValX22Ala AlaX25AlaGluLysValPheLysX32TyrAlaAsnAspAsnGlyValX40GlyX42TrpThrTyrAspAspAla ThrLysThrPheThrValThrGlu
(In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

16. The capturing agent according to any one of the claims 1 to 8, wherein
the at least one extracellular domain mutant constituting the multimer is each of mutant proteins of B2 domain protein of the wild-type protein G,
the mutant protein consists of an amino acid sequence represented by (h) or of the amino acid sequence obtained by deleting, substituting, inserting or adding one or several amino acid residues in the amino acid sequence represented by (h),
the mutant protein has the binding property to the Fc region of immunoglobulin G, and
the mutant protein has the binding property to the Fc region in the weakly acidic region is decreased in comparison with B2 domain protein of the wild-type protein G.
(h)ThrThrTyrLysLeuValIleAsnGlyLysThrLeuLysGlyGluThrThrThrGluAlaValX22AlaA laX25AlaGluLysValPheLysX32TyrAlaAsnAspAsnGlyValX40GlyX42TrpThrTyrAspAspAlaT hrLysThrPheThrValThrGlu
(In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

17. The capturing agent according to any one of the claims 1 to 8, wherein
the at least one extracellular domain mutant constituting the multimer is each of mutant proteins of B3 domain protein of the wild-type protein G,
the mutant protein consists of an amino acid sequence represented by (i) or of the amino acid sequence obtained by deleting, substituting, inserting or adding one or several amino acid residues in the amino acid sequence represented by (i),
the mutant protein has the binding property to the Fc region of immunoglobulin G, and
the mutant protein has the binding property to the Fc region in the weakly acidic region is decreased in comparison with B3 domain protein of the wild-type protein G.
(i)ThrThrTyrLysLeuValIleAsnGlyLysThrLeuLysGlyGluThrThrThrLysAlaValX22AlaGl uX25AlaGluLysAlaPheLysX32TyrAlaAsnAspAsnGlyValX40GlyValTrpThrTyrAspAspAlaThr LysThrPheThrValThrGlu
(In the amino acid sequence, X22 represents Asp or His; X25 represents Thr or His; X32 represents Gln or His; and X40 represents Asp or His, respectively, with the proviso that a case is excluded where X22 is Asp; and X25 is Thr; X32 is Gln and X40 is Asp simultaneously.)

18. The capturing agent according to any one of the claims 1 to 8, wherein at least one of the extracellular domain mutants constituting the multimer consists of an amino acid sequence represented by any one of SEQ ID NO. 13 to 20 or an amino acid sequence obtained by deleting, substituting, inserting or adding one or several amino acid residues in the amino acid sequences represented by any one of SEQ ID NO. 13 to 20.

19. The capturing agent according to any one of the claims 1 to 8, wherein the three extracellular domain mutants constituting the trimer consist of the amino acid sequence represented by SEQ ID NO. 19 or the amino acid sequence obtained by deleting, substituting, inserting or adding one or several amino acid residues in the amino acid sequence represented by SEQ ID NO. 19.

20. A column for a chromatography for separating and purifying the protein, which is filled with the capturing agent according to any one of the claims 1 to 19.
